(19) Europäisches Patentamt / European Patent Office / Office européen des brevets

(11) **EP 4 458 982 B1**

(12) **EUROPEAN PATENT SPECIFICATION**

(45) Date of publication and mention
of the grant of the patent:
**08.07.2026 Bulletin 2026/28**

(21) Application number: **21969225.8**

(22) Date of filing: **29.12.2021**

(51) International Patent Classification (IPC):
*C12Q 1/68* $^{(2018.01)}$     *C12Q 1/686* $^{(2018.01)}$

(52) Cooperative Patent Classification (CPC):
(C-Sets available)
**G01N 33/54388; A61K 38/4893; B01L 7/52;
C12Q 1/68; C12Q 1/6806; C12Q 1/6825;
C12Q 1/6844; C12Q 1/686**            (Cont.)

(86) International application number:
**PCT/CN2021/142714**

(87) International publication number:
**WO 2023/123134 (06.07.2023 Gazette 2023/27)**

(54) **NUCLEIC ACID AMPLIFICATION METHOD AND DEVICE, AND NUCLEIC ACID DETECTION METHOD AND DEVICE**

VERFAHREN UND VORRICHTUNG ZUR AMPLIFIKATION VON NUKLEINSÄUREN SOWIE VERFAHREN UND VORRICHTUNG ZUM NACHWEIS VON NUKLEINSÄUREN

PROCÉDÉ ET DISPOSITIF D'AMPLIFICATION D'ACIDE NUCLÉIQUE, ET PROCÉDÉ ET DISPOSITIF DE DÉTECTION D'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.11.2024 Bulletin 2024/45**

(73) Proprietor: **Chang Gung University
Taoyuan County 333 (TW)**

(72) Inventors:
• **WU, Minxian
Taoyuan County (TW)**
• **CHEN, Zhiyou
Taoyuan County (TW)**

(74) Representative: **Zaboliene, Reda
Metida
Business center Vertas
Gyneju str. 16
01109 Vilnius (LT)**

(56) References cited:
| | |
|---|---|
| WO-A1-2013/113910 | WO-A1-2020/118444 |
| CN-A- 101 205 560 | CN-A- 105 505 766 |
| US-A1- 2019 143 334 | US-B1- 6 310 354 |

• **YOU MINLI ET AL: "Ultrafast Photonic PCR Based on Photothermal Nanomaterials", TRENDS IN BIOTECHNOLOGY., vol. 38, no. 6, 1 June 2020 (2020-06-01), GB, pages 637 - 649, XP093281727, ISSN: 0167-7799, DOI: 10.1016/ j.tibtech.2019.12.006**
• **LI, SONG ET AL.: "A Novel In situ Magnetic Particle PCR Based High-throughput Genotyping Method Using Universal Tags", PROGRESS IN BIOCHEMISTRY AND BIOPHYSICS, vol. 34, no. 10, 15 October 2007 (2007-10-15), XP009547413**

**EP 4 458 982 B1**

**(Cont. next page)**

(52) Cooperative Patent Classification (CPC): (Cont.)

C-Sets
**C12Q 1/686, C12Q 2565/537, C12Q 2563/149,
C12Q 2563/155, C12Q 2563/143, C12Q 2563/137,
C12Q 2523/313, C12Q 2523/313**

**Description**

**FIELD OF THE INVENTION**

The present invention relates to nucleic acid amplification method and its device, and nucleic acid detection method and its device, particularly to a method and a device for forming an *in-situ* environment around a solid-phase carrier and performing nucleic acid amplification within the *in-situ* environment, and a method and a device for performing nucleic acid detection after completion of *in-situ* nucleic acid amplification.

**BACKGROUND OF THE INVENTION**

Nucleic acid amplification tests (NAAT) have been widely applied in molecular diagnosis, including the diagnosis of infectious pathogens, genetic tests, forensic science, agriculture, and clinical medicine. Even if the analyte only has trace amounts of the target substance, nucleic acid amplification tests exhibit high sensitivity and specificity due to the amplification of the target nucleic acid fragments. Polymerase chain reaction (PCR) is one of the methods used for target nucleic acid amplification testing. It includes three steps for generating new deoxyribonucleic acid (DNA) strands: (a) denaturation of double-strand DNA to single-strand DNA (ssDNA) acting as a template by raising the temperature; (b) annealing of specific primers to the ssDNA template through DNA complementary sequence by lowering the temperature to the range of melting temperature; (c) new DNA strands are synthesized through extension of the thermostable DNA polymerases. The abo - mentioned is the thermal cycling of PCR with 2 to 3 temperature ranges. As the number of thermal cycling increases, newly synthesized amplicons are generated exponentially. However, conventional PCR instruments are bulky, expensive, have high energy consumption (using electric heating modules), and have long turnaround times (usually>1 hour).

Additionally, isothermal nucleic acid amplification technologies (iNAATs) have been developed to replace conventional nucleic acid amplification technologies (NAATs), allowing nucleic acid amplification at a constant and relatively moderate temperature. As compared to conventional NAATs, iNAATs significantly reduce the complexity of temperature management controls (e.g. the setup of thermal cycling, and rapid heating and cooling of the thermal block), its related complex devices, and the overall operating time required for nucleic acid amplification. Due to the absence of complex temperature management controls, iNAATs can be performed by using simple instruments, (i.e. heating plate, oven, or water bath). Among various iNAAT methods/technologies, loop-mediated isothermal amplification (LAMP) is the most widely adopted method, this is mainly because LAMP has good tolerance to DNA polymerase inhibitors (e.g., hemoglobin, IgG, or IgM), moreover, LAMP has high specificity, high sensitivity, and high amplification efficiency. This means that LAMP has the potential to directly amplify target DNA or RNA from partially processed or unprocessed biological samples.

In addition, the literature titled "Loop-mediated isothermal amplification (LAMP)-review and classification of methods for sequence-specific detection" (published in Analytical Methods on February 14, 2020, by Lisa Becherer, Nadine Borst, Mohammed Bakheit, Sieghard Frischmann, Roland Zengerle, and Felix von Stetten) has proposed that LAMP demonstrates increased sensitivity of 10 to 100 times as compared to conventional quantitative PCR (qPCR) or PCR.

Furthermore, the literature titled "Reduced False Positives and Improved Reporting of Loop-Mediated Isothermal Amplification using Quenched Fluorescent Primers" (published in Scientific Reports on May 14, 2019, by Patrick Hardinge and James A. H. Murray) has mentioned that since the use of 4 to 6 target-specific primers are used in LAMP reaction, the specificity of LAMP is expected to be superior to conventional PCR that uses only two primers. Additionally, LAMP can generate more than $10^9$ DNA amplicons within an hour. The presence of target analytes can be determined by directly or indirectly detecting the abundantly amplified DNA amplicons or by-products of the LAMP. However, owing to its high false positive rate and low discrimination, the applications of LAMP-based point-of-care testing are further limited.

Conventional PCR platform mainly uses thermoelectric elements (e.g., Peltier elements) to perform the programmed heating and cooling processes to achieve the required thermal cycling for PCR. This method relies merely on heat conduction to transfer thermal energy between the thermoelectric element and the reaction solution, leading to its relatively slow heating and cooling rates (1.5 to 3°C/second), resulting in a prolonged turnaround time in overall PCR procedures. The literature titled "Emerging ultrafast nucleic acid amplification technologies for next-generation molecular diagnostics" (published in Biosensors and Bioelectronics on June 18, 2019, by Sang Hun Lee, Seung-Min Park, Brian N. Kim, Oh Seok Kwon, Won-Yep Rho, Bong-Hyun Jun) proposes that the key factors for achieving ultra-fast PCR, including rapid thermal cycling, low heat capacity materials, high thermal conductivity, and high-speed DNA polymerases. With the development of protein engineering technology on direct molecular evolution, the second-generation DNA polymerase with a high extension rate has been screened out (e.g., KAPA2G, KAPA Biosystems). Its extension rate can reach up to 1 kb/second, significantly reducing the time required for the PCR reaction. In the past, in order to achieve rapid thermal cycling, the PCR reaction volumes were limited to nanoliters to microliters (nL-μL) in capillaries or microfluidic chips, the increased surface area to volume ratios led to improve heat conducting effects between the thermal block and the PCR reactions [e.g., the LightCycler® 2.0 Carousel-Based System (Roche Diagnostics) or microfluidic polymerase chain reactions were employed]. Additionally, materials with low heat capacity are utilized for performing heating and cooling in thermal cycling, [e.g., air was utilized in the LightCycler® 2.0 Carousel-Based System (Roche Diagnostics)]. Although the overall nucleic acid amplification can be completed within several minutes, they are still accompanied by several technical issues, including low amplicon yield, evaporation and bubble generation in the small reaction volumes, platform scalability, and heat control management. Therefore, rapid nucleic acid amplification/detection is not only about the achievement of rapid thermal cycling, but a strategy for nucleic acid amplification that ensures it is not adversely affected while achieving rapid temperature cycling.

In response to the aforementioned issues, many research teams have conducted studies and made improvements. For example, Royal Institution for the Advancement of Learning McGill University has a U.S. patent with the publication number US20190048397A1 (titled "HEATING MECHANISM FOR DNA AMPLIFICATION, EXTRACTION, OR STERILIZATION USING PHOTO-THERMAL NANOPARTICLES"). The abstract discloses that the photothermal nanoparticles are used to heat the PCR solution in both contact and non-contact manner under the illuminations of an external light source. Different photothermal converted temperature ranges are achieved by exciting the nanoparticles with external energy of varying intensities, enabling sterilization, and photothermal lysis for the nucleic acid extraction and performing amplification of specific nucleic acid fragments. For example, the Regents of the University of California has a U.S. patent with the publication number US20180080064A1 (titled "LED DRIVEN PLASMONIC HEATING APPARATUS FOR NUCLEIC ACIDS AMPLIFICATION"). Its abstract discloses plasmonic heating generated in a 3D miniature reaction chamber with a specific thickness of the gold film by a light-emitting diode (LED) at a specific wavelength, thereby rapid thermal cycling is achieved for PCR under manipulations of LED illuminations. For example, the University of Chicago has a U.S. patent with the publication number US 2018/0297120 A1 (titled "BIPYRAMID-TEMPLATED SYNTHESIS OF MONODISPERSE NOBLE METAL NANOCRYSTALS"). By using synthesized gold nanoparticles with a bipyramid structure, plasmonic heating is generated under the LED illuminations with a specific wavelength, enabling the rapid thermal cycling required for PCR in the miniature reaction chamber. The above-mentioned methods use nanoparticles with noble metal materials and specific configurations to convert light energy to thermal energy (reaction time > 100 picoseconds) through localized surface plasmon resonance (LSPR) effect under the excitation of a specific light source. The photothermal conversion property can be regarded as a fast and immediate response. This concept of plasmonic nanoscale heaters exhibits rapid heating rates and generates a uniform thermal field during PCR thermal cycling. By adjusting the concentration of these nanoparticles, and the intensity of the incident light, the manipulation of photothermal converted temperature can be easily controlled. However, there are still some challenges and issues to be overcome in the above-mentioned methods, which as listed as follows: (1) the liquid evaporation in the miniature reaction chamber, (2) the cooling effect of plasmonic nanoparticles is not obvious during thermal cycling, requiring the use of external devices (e.g., cooling fans) to achieve cooling, (3) the possibility of overlapping wavelength ranges between the incident light source for localized surface plasmon resonance of plasmonic nanoparticles and the spectra of certain organic fluorescent dyes is existed, which may restrict the application in fluorescence-based quantitative PCR.

In addition, the review article by You et al., "Ultrafast Photonic PCR Based on Photothermal Nanomaterials" (Trends in Biotechnology, 2020) discloses photonic PCR based on photothermal nanomaterials for rapid thermal cycling. The article also reviews different photothermal nanomaterial-based photonic PCR approaches and discusses their advantages, drawbacks, and existing challenges.

In addition to the aforementioned photothermal heating method using plasmonic nanoparticles, another method that involves applying an alternating magnetic field to induce heat in magnetic nanoparticles has also been employed in PCR nucleic acid amplification techniques. For example, the team led by Dr. Shieh, Dar-Bin at National Cheng Kung University has a U.S. patent with the publication number US20190143334A1 (titled "METHOD AND DEVICE FOR POLYMERASE CHAIN REACTION"). Its claims refer to the method for nucleic acid amplification comprising a reaction mixture containing the target nucleic acid and particles containing transitional metal material in a reaction unit under electromagnetic radiations (EMR) with a frequency ranging from approximately 200 kHz to 500 THz. The inducing heating generated by the particles of the transitional metal materials is used for PCR amplification. A similar commercial device known as the Mic qPCR, produced by Bio Molecular Systems (BMS), is the world's first qPCR machine using electromagnetic induction heating as the heat source.

The heating methods described above, such as the use of photothermal nanoparticles or magnetic nanoparticles, or the conventional PCR with electric heating elements to heat the PCR solution, all belong to the category of "volumetric heating" (i.e., increasing the temperature of the entire reaction mixture through heating).

In contrast to this, patent application WO 2013/113910 A1 discloses a nucleic acid amplification using nanoparticles that are excited to transfer heat to their environment, thereby enabling local heating for amplification reactions such as PCR. WO 2013/113910 A1 also describes that the excitation may be controlled, for example, by moving an excitation beam or by scanning different regions of the reaction volume, so as to generate temperature conditions suitable for nucleic acid amplification. GNA Biosolutions (GmbH) has developed a unique "Pulse Controlled Amplification (PCA)" to achieve ultra-fast PCR. According to the literature titled "Ultra-fast PCR technologies for point-of-care testing" (published in Journal of Laboratory Medicine on October 12, 2017, by Lars Ullerich, Stephanie Campbell, Frank Krieg-Schneider, Federico Bürsgens and Joachim Stehr) and the U.S. patent with publication number US20140377764A1 (titled "Method for the amplification of nucleic acids using heat transfer for nanoparticles; " related patent applications: DE102012201475B4, CN107604052A, EP2809806B1, WO2013113910A1), it is disclosed that this pulse-controlled nucleic acid amplification technology is a technique for controlling "localized heating" in a specific region. This technology integrates primer-functionalized nanoparticles with photothermal conversion properties, a laser light source, and a large volume of reaction solution for cooling purposes. By using short-period laser pulses (with time intervals between laser pulses ranging from 10 nanoseconds to 500 milliseconds) to selectively irradiate certain gold nanoparticles, a specific thermal radiation field required for PCR is generated, and the thermal radiation field generated and dissipated rapidly by this method. Furthermore, this approach effectively only heated the microenvironment of the surface region of the gold nanoparticles upon laser irradiations. Once the laser irradiation on the nanoparticles is paused, due to their high surface area-to-volume ratio of plasmonic nanoparticles, the photothermal-induced thermal field around the nanoparticles will dissipate rapidly and cool down to the temperature of the surrounding nucleic acid amplification solution. During the PCA process (i.e., consecutive and repeated thermal cycling), the temperature of the nucleic acid amplification solution surrounding the nanoparticles remains nearly unchanged. As described above, the PCA technique enables PCR to achieve rapid thermal cycling around the microenvironment of nanoparticles. This technique can not only significantly improve the issue of reaction solution evaporation that may arise from the aforementioned "volumetric heating" (which can affect the accuracy of the subsequent detection work), but also reduces the requirement for specialized and precise cooling devices (e.g., using cooling fans and temperature feedback control system) in conventional nucleic acid amplification processes (e.g., PCR). Nevertheless, there are still drawbacks to consider, which will be further discussed in the following paragraphs.

Moreover, regarding the aforementioned phenomenon of achieving "localized heating" through the use of "pulse-controlled amplification," this technique can not only use "photothermal particles" to achieve the "localized heating," but also employ other principles or mechanisms to generate the phenomenon of "localized heating." The GNA Biosolutions has a European patent with publication number EP3733292A1 (titled "METHOD FOR CARRYING OUT A POLYMERASE CHAIN REACTION AND DEVICE FOR CARRYING OUT THE METHOD;" related applications: US2019/0249168A1) and the literature titled "Pulse-Controlled Amplification-A new powerful tool for on-site diagnostics under resource limited conditions" (published in PLOS NEGLECTED TROPICAL DISEASES on January 29, 2021, by Katharina Müller, Sarah Daßen, Scott Holowachuk, Katrin Zwirglmaier, Joachim Stehr, Federico Buersgens, Lars Ullerich, Kilian Stoecker). It discloses that PCA amplification was achieved by using a rapid energy pulse to heat microcyclers (micro metal heating element directly embedded in nucleic acid amplification reaction vessel). By applying a microsecond-period electric heating, an instantaneously generated thermal radiation field occurs only in the surface area of the micrometal heating element (designed nucleic acid amplification reaction region). Once the electric pulse is ceased, the heat of the metal heating element will dissipate rapidly and cool down by the surrounding large volume of the reaction solution. Through repeated control and generation of the aforementioned temperature field, this design enables the rapid generation of temperature cycles required for nucleic acid amplification reactions (e.g., PCR), thereby achieving the objective of fast nucleic acid amplification.

Based on the aforementioned prior patents and literature, GNA Biosolutions has developed a unique "Pulse-Controlled Amplification (PCA)" that achieves ultra-fast nucleic acid amplification (e.g., PCR), and subsequent detection of nucleic acids. This technique can significantly improve the issues of reaction solution evaporation caused by "volumetric heating" in conventional PCR and reduces the requirement for specialized cooling designs or devices in conventional nucleic acid amplification processes.

However, the PCA technique mainly controls the relative positions of the laser illumination beams within the reaction region of PCR dynamically, allowing high-power NIR laser beams to trigger plasmonic nanoparticles to generate a photothermal effect within the reaction region of PCR with laser illuminations in very short time intervals ranging from 10 nanoseconds to 500 milliseconds. By irradiating a portion of the plasmonic nanoparticles within the reaction region of PCR selectively and specifically, the required temperature fields for performing nucleic acid amplification are generated as mentioned above. Basically, the performing of the PCA technique has certain technical difficulties. In addition, the volume of PCR solution used for cooling purposes in the PCA technology is relatively large (e.g., 100-500 µL). This is because in the PCA technology, the nucleic acid products (e.g., amplicons) are generated in the form of single-strand DNA (ssDNA) within the PCR solution. The detection method of the amplicons, as described in the European patent with publication number EP2481817A1 (titled "Process for detecting nucleic acids"), the European patent with publication number EP3733292A1, and by Müller et al. (2020), is achieved by detecting the polymer (e.g., amplified free single-strand DNA) generated by (primer-functionalized) plasmonic nanoparticles and, based on the spectral difference at a wavelength of 650 nm or the fluorescent signal generated by TaqMan probes. In the aforementioned nucleic acid detection methods, due to the dilution effect [e.g., a large volume of PCR solution (100-500μL)], the PCA technology will lead to the need for performing more thermal cycles of nucleic acid amplification to accumulate the signal of amplicons. Those drawbacks further affect the total turnaround time required for nucleic acid amplification and detection.

Additionally, according to the prior patents of the GNA Biosolutions, there are still many aspects of PCA nucleic acid amplification methods and devices requiring improvement, summarized as follows:

1. Localized heating: By integrating the monodispersed photothermal nanoparticles within a large volume of PCR solution, PCA technology enables the achievement of "ultra-fast nucleic acid amplification" through "ultra-fast temperature (thermal) cycling," and during the overall process of ultra-fast nucleic acid amplification, there is no significant change in the temperature of the PCR solution. This technical characteristic can improve the issue of evaporation of the trace volume of PCR solution during the temperature (thermal) cycling of the conventional fast nucleic acid amplification techniques (e.g., the accuracy of detection). However, this technique mainly relies on dynamic control of the relative positions of the laser beam and the illumination regions within the PCR reaction. By allowing high-power NIR laser beams to trigger plasmonic nanoparticles to generate a photothermal effect within the reaction region of PCR with laser illuminations in very short time intervals ranging from 10 nanoseconds to 500 milliseconds. By irradiating a portion of the plasmonic nanoparticles within the reaction region of PCR selectively and specifically, the required temperature fields for performing nucleic acid amplification are generated as mentioned above. Basically, this operation has certain technical difficulties.

2. The prior patents of GNA Biosolutions mention that the PCR used in the PCA nucleic acid amplification reactions, which employs a two-step temperature (thermal) cycling, including a denaturation temperature of 95°C required for double-strand separations of DNA, and an annealing and extension temperature of 65°C required for oligo primer/probe annealing and DNA polymerase extension, respectively. Although the PCA technique utilizes a large volume of PCR solution as a cooling medium to cool the localized heating of plasmonic nanoparticles upon laser off (e.g., from 95°C to 65°C), because the temperature difference is merely 30°C, the cooling rate may not be as fast as claimed for the "instant cooling" effect by the PCA technology.

3. The PCA technique utilizes a large volume of PCR solution as a cooling medium to cool down the localized heating of plasmonic nanoparticles upon laser off. However, the amplicons of PCA generated in the form of free single-strand DNA (ssDNA) within the PCR solution, due to the dilution effect of a large volume of PCR solution (e.g., 100-500μL), which will lead to the requirement for more thermal cycles of nucleic acid amplification to accumulate the signal of amplicons. Those drawbacks further affect the total turnaround time required for nucleic acid amplification and detection

4. When encountering complex nucleic acid analytes in a biological sample: the conventional PCR employs a primer pair design to perform nucleic acid amplification. When encountering complex nucleic acid analytes, including partially similar or identical sequences complementary with oligo primer sets, these sequences can undergo partial hybridization during the annealing process, resulting in the generation of non-specific chimeric by-products. This will contribute to imprecise discrimination with point-of-care testing devices.

Therefore, how to implement simple and fast nucleic acid amplification and detection while avoiding the aforementioned technical issues remains a critical unresolved challenge.

## SUMMARY OF THE INVENTION

In view of the issues with prior art, one of the objectives of the present invention is to provide a nucleic acid amplification method and its device, enabling easy and rapid temperature (thermal) cycling required for nucleic acid amplification, and the nucleic acid amplification involves amplification of nucleic acid molecules partially immobilized on the surface of a solid-phase carrier. Another objective of the present invention is to achieve fast purification, separation, and enrichment of nucleic acids on the surface of the solid-phase carrier, thereby facilitating the subsequent detection and analysis of target nucleic acid molecules with high specificity and sensitivity.

According to one objective of the present invention, a nucleic acid amplification method is provided, comprising: a reaction unit including at least one analyte, a nucleic acid amplification solution, at least one solid-phase carrier within its interior, wherein the reaction unit, and the content within its interior, including the analyte, the nucleic acid amplification solution and the solid-phase carriers are maintained at a cooling temperature in a cooling environment; by modulating the output of an external energy and the timing sequence of on and off of the external energy. Meanwhile, by coordination with the cooling temperature of the cooling environment, thereby generating one or more temperature (thermal) cycles required for nucleic acid amplification happened around solid-phase carriers (i.e., *in-situ* environment ), wherein in each of the temperature (thermal) cycles, all the solid-phase carriers simultaneously form its corresponding *in-situ* environment triggered upon irradiations of the external energy, when the external energy to the solid-phase carriers is paused, the *in-situ* environment around solid-phase carriers will be dissipated. By the formation and dissipation of the *in-situ* environment on solid-phase carriers, which are allowed to perform the nucleic acid amplification under the presence of target analytes, thereby generating amplicons.

Among these, the nucleic acid amplification reactions comprise polymerase chain reaction (PCR), ligase chain reaction (LCR), or isothermal nucleic acid amplification technologies (iNAAT).

Among these, during each turn-on period of external energy irradiation of the thermal cycle, all the solid-phase carriers within the reaction unit are simultaneously triggered, resulting in the formation of the *in-situ* environment around each of the solid-phase carriers, and further generating the amplicons immobilized on each of the carriers. During each turn-on period of high-power external energy irradiation, the immobilized amplicons will be denatured, and a portion of the amplicons is retained on each of the solid-phase carriers, while another portion of the amplicons is released into the nucleic acid amplification solution as templates; and during each turn-off period of external energy irradiation of the thermal cycle, the excitation of each of the solid-phase carriers is paused, and each of the *in-situ* environments dissipates through the cooling temperature of the cooling environment.

Among these, the enzyme can be a polymerase, wherein the polymerase comprises DNA polymerase, RNA polymerase, or the enzyme can also be one or a combination of reverse transcriptase (RT), ribonuclease (RNase), helicases, DNA ligase, and can act coordinately with the polymerase.

Among these, the transfer methods of external energy irradiation to each of the solid-phase carriers include contact or non-contact excitation mode.

Among these, the non-contact excitation mode includes photothermal excitation or magnetic excitation. The light source utilized for photothermal excitation is one of either laser or LED arrays, with wavelengths ranging from the visible to near-infrared spectrum, and the wavelength range from 380 nanometers (nm) to 1.4 micrometers ($\mu$m).

Among these, the magnetic excitation system uses alternating magnetic fields (AMF) to generate heat (i.e., AMF-induced hyperthermia). The alternating magnetic field is generated by an alternating magnetic field generator, and the amplitude and frequency of the alternating magnetic field are set according to the temperature conditions required for nucleic acid amplification on the solid-phase carriers. Furthermore, the amplitude of the alternating magnetic field ranges from 0.5 to 550 kA/m, and the frequency of the alternating magnetic field ranges from 3 to 3,500 kHz.

Among these, the contact excitation mode is electric heating, in which electrical energy can be transmitted through electronic circuits or induced magnetic flux for electric energy transmission (wireless charging). The electric heating modes can be Joule heating, thermoelectric heating, or surface acoustic waves (SAWs).

Among these, the ratio of the total volume of the solid-phase carriers to the volume of the nucleic acid amplification solution is from 1:200 to $1:1\times10^9$.

Among these, the size of each of the solid-phase carriers ranges from 8 to 2,000,000 nm, among which the preferred size of the solid-phase carrier for photothermal excitation and magnetic field excitation is 8 to 1,000 nanometers; while the preferred size of the solid-phase carrier for electric heating is 1,000 to 2,000,000 nanometers.

Among these, the shape of each of the solid-phase carriers can be in the form of spheres, ellipsoids, disc-shaped, star-shaped, rod-shaped, squares, anisotropic structures, nanoshells, nanocages, bipyramidal structures, microfilament or a combination of two or more of these.

Among these, each of the solid-phase carriers can be either suspended within the reaction unit, or each of the solid-phase carriers can be tethered to the inner wall of the reaction unit, or a combination of both.

Among these, the cooling temperature ranges from -10 to 50°C.

Among these, the reaction unit or the nucleic acid amplification solution can be pre-cooled to the cooling temperature or placed in an external auxiliary cooling unit, wherein the pre-cooled reaction unit or the external auxiliary cooling unit maintains the cooling temperature during one or more thermal cycles.

Among these, the external auxiliary cooling unit comprises ice, a water-swelling macromolecular polymer, chemical endothermic reactants, thermoelectric cooling chips, or a combination of any two of these. The water-swelling macromolecular polymer is carboxymethyl cellulose, among which the chemical endothermic reactants are ammonium nitrate or urea dissolved in water, involving endothermic reactions.

Among these, the analytes to be tested can be a cell, an organelle, a bacterium, a virus, a protozoan, or a combination thereof. The solid-phase carrier comprises a multifunctional body, at least one enrichment ligand, and at least one amplification ligand, among which each of the enrichment ligands is immobilized on the surface of the multifunctional body used for specific capture of the analyte; wherein the amplification ligands are also immobilized to the surface of the multifunctional body and are used for binding a biological substance. Among these, the biological substances are deoxyribonucleic acid or ribonucleic acid released from the analyte, or the amplicons replicated on the amplification ligand and subsequently released into the nucleic acid amplification solution. Among these, the enrichment ligand can be an antibody, nucleic acid aptamer, oligonucleotide, protein, polysaccharide, or a combination thereof. Wherein the amplification ligand can be a nucleic acid aptamer or oligonucleotide, and has a functional group modification for immobilization to the solid-phase carrier, which can be modified with a primary amine, biotin, thiol groups, or a combination thereof. In order to enhance the specificity and stability of oligonucleotide for hybridization capture to biological substances, the modifications can be a locked nucleic acid (LNA), phosphorothioates, morpholino modifications, or a combination thereof.

Among these, the analyte to be tested can be a cell, an organelle, a bacterium, a virus, a protozoan, or a combination thereof. Part of the solid-phase carriers includes an enrichment body and at least one enrichment ligand, each of the enrichment ligands is immobilized to the surface of the enrichment body and is used for specific capture of the analyte; another part of solid-phase carriers includes an amplification body and at least one amplification ligand, each of the amplification ligands are immobilized to the surface of the amplification body and is used for hybridization binding a biological substance released by the analyte. The biological substance is deoxyribonucleic acid or ribonucleic acid released from the analyte, or the amplicons replicated on the amplification ligand and subsequently released into the nucleic acid amplification solution. Among these, the enrichment body corresponded to the multifunctional body functionalized with only the enrichment ligand. Among these, the amplification moiety corresponds to a multifunctional moiety with only the amplification ligand.

Among these, the analyte is a free deoxyribonucleic acid or a free ribonucleic acid, among which each of the solid-phase carriers includes an amplification body and at least one amplification ligand, each of the amplification ligands is immobilized to the surface of the amplification body and are used for hybridization binding of the analyte.

According to one objective of the present invention, a rapid nucleic acid amplification system is provided, comprising: a reaction unit, an auxiliary cooling unit, an external energy excitation unit, an integrated driver; among which, the reaction unit is provided to accommodate a reaction solution, an analyte, a nucleic acid amplification solution, and one or more solid-phase carriers, wherein the auxiliary cooling unit can be a pre-cooled nucleic acid amplification solution at a cooling temperature, or can be an external auxiliary cooling unit arranged around the reaction unit, continuously cooling the reaction unit and maintaining the nucleic acid amplification solution at a cooling temperature; the external energy excitation unit is configured to provide contact and non-contact energy transfer to the solid-phase carriers to generate heat; the integrated driver is configured to control the energy output and timing sequence of on and off of external energy excitation unit, among which the integrated driver is also configured to modulate energy output of the external excitation unit and feedback control of the external auxiliary cooling unit by an external energy calibrator and a temperature detection unit, thereby generating one or more temperature (thermal) cycles required for nucleic acid amplification.

According to another aim of the present invention, a nucleic acid detection method is provided, comprising the following steps for nucleic detection after completing the nucleic acid amplification: an operation unit is used to assist in the purification, separation, and concentration of the amplicons immobilized on the solid-phase carriers; a detection module to detect one or a combination of optical changes, thermal sensing changes, electrochemical changes, magnetic changes, or mass changes (e.g., piezoelectric) occurring on the amplicons bound to the solid-phase carriers. Furthermore, the detection module detects amplification signals by the amplicons bound to the solid-phase carriers.

Among these, the methods for detecting the optical changes on the amplicons immobilized on solid-phase carriers, comprised of detecting the change of light intensity generated directly by the primer labeled with the nucleic acid tags or fluorophore incorporated into amplicons with a spectrophotometer or a fluorometer, or detecting the optical changes or chemiluminescence changes generated by an enzyme-linked immunosorbent assay, or detecting spectral changes resulting from the repulsion on the amplicons immobilized on the solid-phase carriers.

Among these, the method for detecting optical changes on the amplicons immobilized on the solid phase carriers, wherein the detection module comprises nucleic acid lateral flow strips or lateral flow immunoassay strips, which are used to detect the optical changes.

Among these, the sensitivity of the nucleic acid lateral flow strips or the lateral flow immunoassay strips is enhanced by combining the nucleic acid lateral flow strips or the lateral flow immunoassay strips with a thermal sensing detection, surface plasmon resonance spectroscopy, or any combination thereof.

Among these, a method for electrochemical changes occurring on the amplicons immobilized on solid-phase carriers, comprising one or a combination of both electrochemical detections coupled with enzyme-linked immunosorbent assay and electrochemical impedance spectroscopy.

Among these, a method for magnetic changes occurring on the amplicons immobilized on solid-phase carriers, including the detections of frequency-dependent alternating current (AC) susceptibility with the AC susceptometry, and the giant magnetoresistive measurement (GMR), or a combination of thereof.

Among these, a method for mass changes occurring on the amplicons immobilized on solid-phase carriers that is performed using a quartz crystal microbalance (QCM).

According to another aim of the present invention, a rapid nucleic acid detection device is provided, comprising an operation unit and a detection module, wherein the operation unit is configured to assist in purification, separation, and concentration of the solid-phase carriers; the detection module is configured to detect one or a combination of optical changes, thermal sensing changes, electrochemical changes, magnetic changes, or mass changes occurring on the amplicons immobilized to the solid-phase carriers.

In summary, the present invention is different from conventional nucleic acid amplification methods and possesses the following advantages:

(1) By simply controlling the characteristic of the external energy excitation (i.e., the magnitude and frequency of external energy) and the cooling effect in the reaction unit, it is possible to instantly generate the temperature (thermal) cycling required for nucleic acid amplification within the *in-situ* environment around the surface of a solid-phase carrier, thereby achieving the purpose of fast nucleic acid amplification.

(2) The nucleic acid amplification solution is pre-cooled to the cooling temperature, or using an auxiliary cooling unit to keep the reaction unit in a cooling environment, it is possible to inhibit non-specific primer annealing/extension and non-target nucleic acid amplification, thereby improving the specificity of amplification for the target nucleic acid.

(3) Due to the limited capacity of the amplification ligands on the surface of the solid-phase carrier, the immobilized amplified nucleic acid molecules on the solid-phase carrier surface can reach a saturation state within a relatively short operation time (or a relatively small number of thermal cycles). This will facilitate the subsequent detection of amplified nucleic acid molecules.

(4) If the solid-phase carrier has strong magnetism, the amplicons generated on the solid-phase carrier can be further purified and concentrated through magnetic manipulation by the operation unit. This technological characteristic will facilitate the subsequent detection of the amplicons immobilized on the solid-phase carriers (e.g., enhancing detection performance).

(5) Because the overall nucleic acid amplification solution is maintained at a low temperature, it not only avoids the issues of evaporation and bubble generation in the reaction solution during thermal cycling but also has the advantage of inhibiting or reducing non-specific nucleic acid amplification.

## BRIEF DESCRIPTION OF THE DRAWINGS

FIG. 1(a) is a schematic diagram of rapid temperature increase in one implementation example of the present invention;

FIG. 1(b) is a schematic diagram of rapid temperature decrease in one implementation example of the present invention;

FIG. 2 is a schematic diagram of the structure and surface characteristics of the solid-phase carrier in one embodiment of the present invention;

FIG. 3 is a schematic diagram of the setup of a nucleic acid amplification system in one embodiment of the present invention;

FIG. 4(a) is a schematic diagram of the suspension state of solid-phase carriers mixed with the nucleic acid amplification solution within the reaction unit in one embodiment of the present invention;

FIG. 4(b) shows that the solid-phase carriers are tethered to the inner wall of the bottom of the reaction unit with the covalent bonding in one embodiment of the present invention;

FIG. 4(c) is a schematic diagram of the solid-phase carriers embedded in the inner wall of the reaction unit in one embodiment of the present invention;

FIG. 5 is a schematic diagram of a nucleic acid amplification system, using solid-phase carriers with photothermal conversion capability for performing water cooling on-bead polymerase chain reaction in one embodiment of the present invention;

FIG. 6 is a schematic diagram of the purification, separation, and concentration of amplicons immobilized on the solid-phase carriers by using the operation unit in one embodiment of the present invention.

FIG. 7(a)-(e) are schematic diagrams of the first reaction process of the water cooling on-bead polymerase chain reaction (the water cooling on-bead PCR);

FIG. 7(f)-(g) are schematic diagrams of the second reaction process after completing FIG. 7(a)-(e) in one embodiment of the present invention;

FIG. 7(h)-(k) are schematic diagrams of the third reaction process after completing FIG. 7(f)-(g) in one embodiment of the present invention;

FIG. 7(l) is a schematic diagram after completing multiple photonic cycles;

FIG. 8 is a flowchart of the nucleic acid amplification method and detection method of the present invention;

FIG. 9(a) is a schematic diagram of the primer pairs sets used for performing the water-cooling on-bead loop-mediated isothermal amplification (water-cooling on-bead LAMP) in one embodiment of the present invention;

FIG. 9(b)-(e) are schematic diagrams of the procedures for performing the pulse-controlled water cooling on-bead LAMP in one embodiment of the present invention;

FIG. 10(a) shows that the result of the micro-scale photothermal temperature measurement of solid-phase carriers (i.e. the magnetic gold nanoshells, $2.9 \times 10^{10}$ particles/ml, 20 μL) under NIR laser irradiations with consecutive 10 photonic cycles (i.e., the setup of the photonic cycle, NIR laser on at 400 mW/0.16cm$^2$ for 60 seconds, and laser off for 120 seconds).

FIG. 10(b) shows that the result of the micro-scale photothermal temperature measurement of the solid-phase carriers (i.e. the magnetic gold nanoshells, $2.9 \times 10^{10}$ particles /mL, 20 μL) under NIR laser irradiations with different excitation powers (i.e., 200 mW/0.16 cm$^2$ to 700 mW/0.16 cm$^2$ for 300 seconds).

FIG. 11(a) shows that the result of the relationship between the applied NIR laser intensity and the micro-scale photothermal converted temperature of the monodispersed magnetic gold nanoshells (MGNs) in one embodiment of the present invention;

FIG. 11(b) is an infrared thermal image of a micro-scale suspension of magnetic gold nanoshells after NIR laser irradiations with different powers.

FIG. 12(a) is the layout of the auxiliary cooling unit in one embodiment of the present invention.

FIG. 12(b) the overall temperature changes of the nucleic acid amplification solution containing the monodispersed magnetic gold nanoshells were measured under 100 photonic cycles with the assistance of the above-mentioned auxiliary cooling unit (each photonic cycle is modulated by the integrated driver at 400 mW/0.16 cm$^2$ for 1.25 seconds, followed by 0.5 seconds of laser off, and then at 150 mW/0.16 cm$^2$ for 7.5 seconds of irradiation);

FIG. 13(a) is a schematic diagram of the lateral flow immunoassay strip before detection in one embodiment of the present invention;

FIG. 13(b) is a schematic diagram of the detection results of the water cooling on-bead polymerase chain reaction using lateral flow immunoassay strip in one embodiment of the present invention;

FIG. 14(a) is a schematic diagram of a positive result of the water cooling on-bead PCR detected by integrating the plasmonic thermal sensing and the lateral flow immunoassay strip as illustrated in FIG. 13(b);

FIG. 14(b) is a schematic diagram of a negative result of the water cooling on-bead PCR detected by integrating the plasmonic thermal sensing and the lateral flow immunoassay strip as illustrated in FIG. 13(b);

FIG. 15(a) shows that the lateral flow immunoassay strips were used to detect the amplicons immobilized on MGNs at different photonic cycles (i.e., from 10 to 50 cycles) of the water cooling on-bead polymerase chain reaction. Each photonic cycle is modulated by the integrated driver at 400 mW/0.16 cm$^2$ for 1.25 seconds, followed by 0.5 seconds of laser off, and then at 150 mW/0.16 cm$^2$ for 7.5 seconds;

FIG. 15(b) shows that the denaturing urea agarose gel electrophoresis was used to measure the residual amplicons in the nucleic acid amplification solution at different photonic cycles (i.e., from 10 to 30 cycles) of the water cooling on-bead polymerase chain reaction;

FIG. 16(a) shows that the results of analytical sensitivity and specificity of the conventional polymerase chain reaction for detecting the *malB* gene with a simple DNA sample derived from *Escherichia coli(E.coli);*

FIG. 16(b) is the analytical sensitivity results subjected to water cooling on-bead PCR and lateral flow immunoassay strips with a simple DNA sample derived from *E.coli;*

FIG. 17(a) is the results of analytical sensitivity and specificity of the conventional polymerase chain reaction for detecting the *malB* gene with a complex DNA sample comprised of the genomic DNAs derived from multiple pathogenic bacteria and *E.coli;*

FIG. 17(b) is the analytical sensitivity results subjected to water cooling on-bead PCR and lateral flow immunoassay strips with a complex DNA sample comprised of the genomic DNAs derived from multiple pathogenic bacteria and *E.coli;*

FIG. 18(a) is the performance evaluation of the enrichment ligands functionalized solid-phase carriers on the capabilities of target analyte enrichment and photothermal lysis. The colony-forming-unit (CFU) testing is used to assess the capture efficiency rate of target analytes (e.g., *E.coli* ) and the photothermal lysis efficiency under the NIR irradiations with different power densities (i.e., 0 to 500 mW/0.16cm$^2$ for 300 seconds);

FIG. 18(b) is the performance evaluation combined with the photothermal lysis, the water cooling on-bead PCR, and the lateral flow immunoassay strip in one embodiment of the present invention. The signal readouts on lateral flow immunoassay strips are utilized to assess the photothermal lysis efficiency under the NIR irradiations with different powers;

FIG. 19(a) is a flowchart of the overall operation process for performing the NIR laser-driven water cooling on-bead PCR;

FIG. 19(b) is the analytical sensitivity evaluation of the NIR laser-driven water cooling on-bead PCR by using the lateral flow immunoassay strips. The solid-phase carrier mixture comprises two types of functional MGNs (i.e., enrichment ligand and amplification ligand-functionalized MGNs), and the samples spiked with various amounts of target analytes (i.e., *E.coli*) are used to assess the analytical sensitivity of NIR laser-driven water cooling on-bead PCR ;

FIG. 20(a) is the quantitative results of images on the test lines region of the lateral flow immunoassay strips as shown in FIG. 19(b). The image analysis software (i.e., Image J) is used to analyze pixel intensities of test lines on the lateral flow immunoassay strips;

FIG. 20(b) is the results integrated the plasmonic thermal sensing and conventional lateral immunoassay strips, the thermal imaging on the test line region of the lateral flow immunoassay strip was captured by infrared thermal camera under different 808 nm laser irradiations (i.e., 24 mW or 140 mW);

FIG. 20(c) is the quantitative result of the increment of plasmonic heating after subtracting the background temperature from the thermal image in FIG. 20(b);

FIG. 21(a) is the result of the overall temperature changes of nucleic acid amplification solution for performing the NIR laser-driven water cooling on-bead polymerase chain reaction under nucleic acid amplification solutions with a relatively low cooling temperature range (e.g., ice-cold, cold, and cool temperature).

FIG. 21(b) is the *in-situ* nucleic acid amplification evaluation for performing the NIR laser-driven water cooling on-bead PCR under nucleic acid amplification solutions with a relatively low cooling temperature range.

EXPLANATION OF CODES

    10: reaction unit
    11: integrated driver
    12: energy excitation unit
    121: laser collimator
    122: laser emitter
    13: external auxiliary cooling unit
    14: temperature detection unit
    15: operation unit
    16: external energy calibrator
    17: detection module
    171: lateral flow immunoassay strip
    1711: test line
    1712: control line
    172: NIR laser-coupled infrared thermal sensor
    20: analyte
    21: biological substance
    30: nucleic acid amplification solution
    301: primer
    3011: fluorophore
    302: polymerase
    40: solid-phase carrier
    401: magnetic core
    402: intermediate layer
    403: gold nanoshell
    41: multifunctional body
    42: amplification body
    50: *in-situ* environment
    60: amplicon
    70: enrichment ligand
    71: amplification ligand
    72: polyethylene glycol
    73: bovine serum albumin
    S10-S90: step
    S100-S400: step

DETAILED DESCRIPTION OF THE PREFERRED EMBODIMENTS

To make the objectives, technical solutions, and advantages of the present invention clearer and more understandable, the following detailed description of the present invention is provided in conjunction with the accompanying drawings and embodiments. The methods described herein are performed in vitro or ex vivo on samples removed from a human or animal body, and are not practiced on the human or animal body. It should be noted that the specific embodiments described herein are provided for the purpose of illustrating the present invention and should not be considered as limiting the scope of the present invention.

The present invention is a nucleic acid amplification method, comprised of providing an analyte 20, a nucleic acid amplification solution 30, and at least one solid-phase carrier 40 inside the reaction unit 10. The reaction unit 10 is placed in a cooling environment that achieves the required cooling temperature. The cooling temperature ranges from -10 to 50°C. The preferred cooling temperatures range from 15 to 30°C and 30 to 50°C, with the optimal cooling temperatures ranging from -10 to 0°C, and from 2 to 15°C. By controlling operating conditions of external energy excitation (e.g., to modulate energy output and frequency), and using the cooling effect achieved by the cooling environment of the reaction unit 10, an *in-situ* environment 50 is formed around the surface of the solid-phase carrier 40 within the reaction unit 10, and a temperature (thermal) cycling required for nucleic acid amplification is performed within the *in-situ* environment 50, enabling the generation of amplicons 60 on the solid-phase carrier 40 during each temperature (thermal) cycling within the reaction unit 10. It should be noted that the temperature (thermal) cycling required for nucleic acid amplification within the reaction unit 10 refers to different temperatures needed for nucleic acid denaturation, primer annealing, and DNA polymerase extension, but the actual implementation is not limited thereto. The size of each of the solid-phase carriers ranges from 8 to 2,000,000 nm, wherein the preferred size of the solid-phase carrier for photothermal excitation and magnetic field excitation is 8 to 1,000 nanometers; while the preferred size of the solid-phase carrier for electric heating is 1,000 to 2,000,000 nanometers.

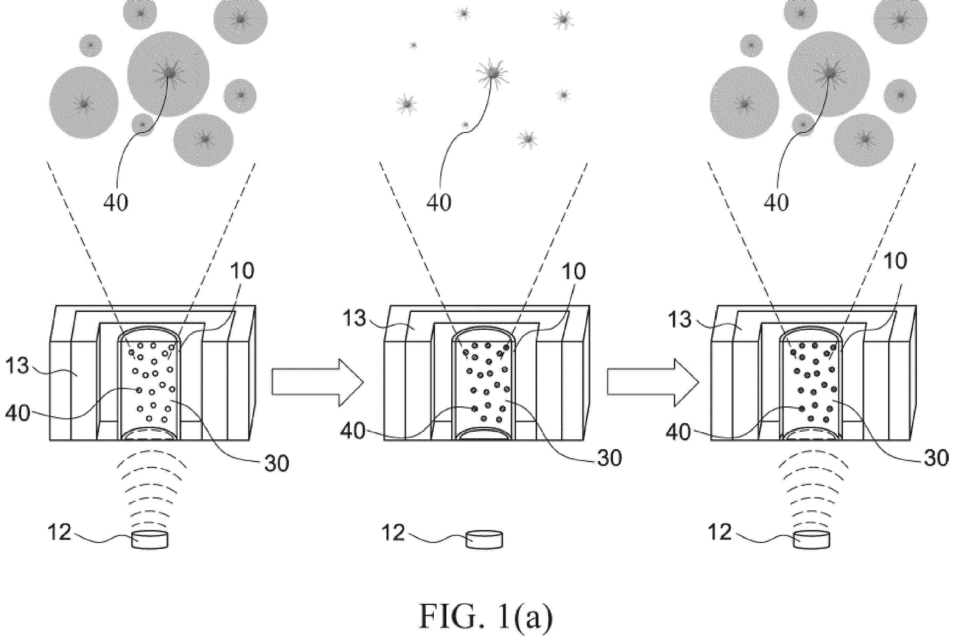

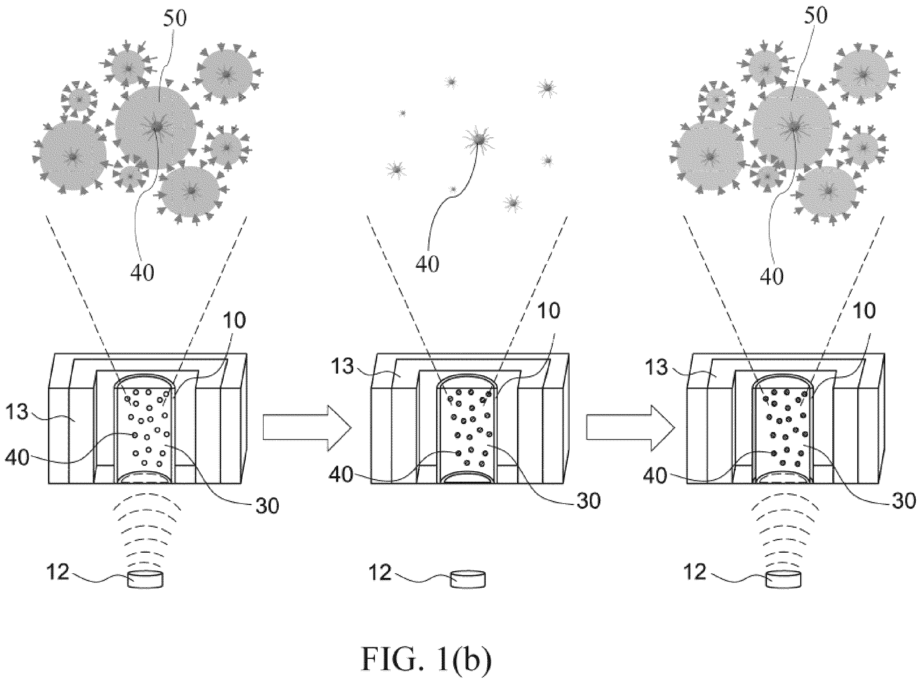

The term *"in-situ* environment 50"* as described in the present invention refers to the configurations shown in FIG. 1(b). When the solid-phase carriers 40 inside the reaction unit 10 are in an evenly distributed state (i.e., monodispersed suspension), by controlling the operation of external energy excitation conditions as shown in FIG. 1(a), and the cooling temperature of the cooling environment in the reaction unit 10 as shown in FIG. 1(b), the size of the thermal radiation field (i.e., heat transfer distance) emitted outwardly from the solid-phase carriers 40 upon excited by the external energy, which can be further adjusted coordinately by controlling the operating conditions of external energy excitation and the cooling temperature of the cooling environment within the reaction unit 10, ensuring the thermal radiation fields generated by the solid-phase carriers 40 do not overlap with each other, and form an independent space for nucleic acid amplification, or referred to as the localized heating region of the solid-phase carriers 40 (as shown in FIG. 1(b)), and named as *"in-situ* environment"* in the present invention. Furthermore, when the excitation of external energy is paused, the cooling temperature of the reaction unit 10 is used to dissipate heat rapidly and to achieve the purpose of instant cooling of the solid-phase carriers 40.

Moreover, the term *"in-situ* nucleic acid amplification"* as described in the present invention refers to that the nucleic acid amplification occurs in the *in-situ* environment 50 on the surface of the solid-phase carriers 40, and the amplicons 60 are immobilized on the surface of the solid-phase carriers 40. The amplicons 60 includes but is not limited to amplified nucleic acid molecules. A portion of the amplicons 60 generated through the *in-situ* nucleic acid amplification is retained on each of the solid-phase carriers 40, while another portion of the amplicons 60 is released into the nucleic acid amplification solution 30.

In the present invention, the nucleic acid amplification solution 30 includes primers 301, nucleotides, enzymes, and reaction additives. The enzyme can be a polymerase 302, wherein the polymerase 302 includes DNA polymerase, RNA polymerase, or the enzyme can also be one or a combination of reverse transcriptase (RT), ribonuclease (RNase), helicases, DNA ligase, and can act coordinately with the polymerase.

In the present invention, nucleic acid amplification can include polymerase chain reaction (PCR), ligase chain reaction (LCR), or isothermal nucleic acid amplification technologies (iNAATs). Among the various iNAATs methods, loop-mediated isothermal amplification (LAMP) is preferred. In the polymerase chain reaction, a two-step temperature (thermal) cycling program is used, with denaturation temperatures ranging from 85 to 95°C, and annealing and polymerase extension temperatures ranging from 60 to 65°C; the temperature for loop-mediated isothermal amplification ranges from 60 to 65°C.

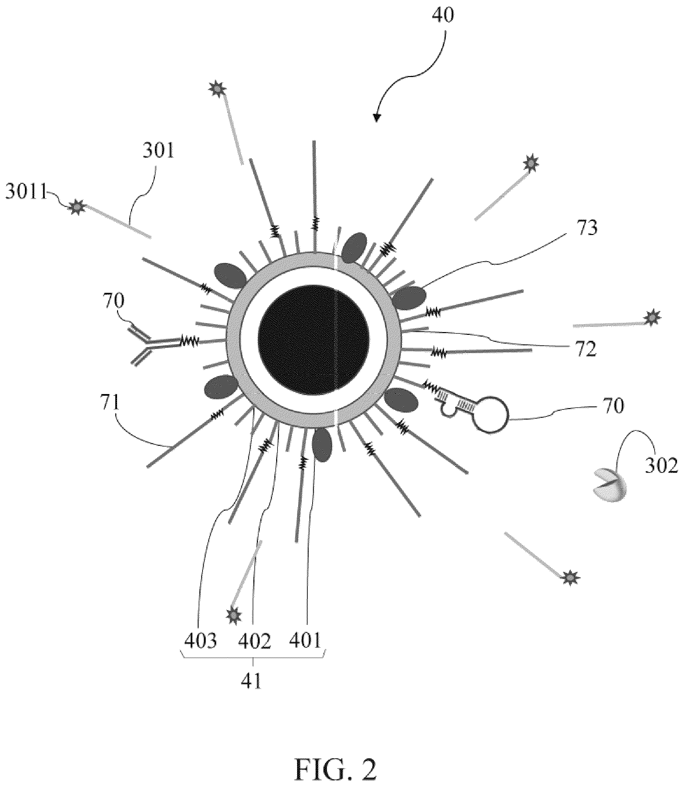

In one embodiment of the present invention, as shown in FIG. 2, the solid-phase carrier 40 includes a multifunctional body 41, at least one enrichment ligand 70, and at least one amplification ligand 71. Each of the enrichment ligands 70 is immobilized to the surface of the multifunctional body 41. The enrichment ligand 70 can be an antibody, nucleic acid aptamer, oligonucleotide, protein, polysaccharide, or a combination thereof. The amplification ligands 71 are immobilized to the surface of the multifunctional body 41. The amplification ligand 71 can be a nucleic acid aptamer or oligonucleotide, modified with functional groups for binding to the solid-phase carrier 40, which can be primary amines, biotin, thiol groups, or a combination thereof. It can also include modifications for enhancing the specificity and stability of oligonucleotide hybridization binding to biomolecules, the modifications can be a locked nucleic acid (LNA), phosphorothioates, morpholino modifications, or a combination thereof, but the actual implementation of the present invention is not limited thereto.

In this embodiment, the selected enrichment ligand 70 is capable of capturing specifically the aforementioned analyte 20. The analyte 20 to be tested can be a cell, an organelle, a bacterium, a virus, a protozoan, or a combination thereof. The enrichment ligand 70 specifically binds to the analyte 20, allowing the analyte 20 to be captured onto the enrichment ligand 70 of the solid-phase carrier 40.

In this embodiment, at least one amplification ligand 71 is functionalized on the multifunctional body 41, which is used to bind to the oligonucleotide primers or probes utilized for nucleic acid amplification. Furthermore, the amplification ligand 71 can be designed for hybridization capture to a biological substance 21, which can be the nucleic acid molecules released from the analyte 20 after thermal lysis or the amplicons 60 replicated on the amplification ligand 71 and subsequently released into the nucleic acid amplification solution 30. Additionally, the labeled primer 301 can incorporated into the amplicons in the nucleic acid amplification solution 30. The tag labeling of the labeled primers 301 can be a nucleic acid tag, including but not limited to radioactive isotopes (such as $^3$H, $^{14}$C, and $^{32}$P), digoxin (DIG), biotin, fluorophore [e.g., fluorescein isothiocyanate (FITC), Texas Red, Cy2, Cy3, Cy5, Cy7, rhodamine B], and luminescent substances (e.g., 2',6'-DiMethylcarbonylphenyl-10-sulfopropylacridinium-9-carboxylate 4'-NHS ester), but the actual implementation of the present invention is not limited thereto.

In this embodiment, the surface of the solid-phase carrier 40 is subjected to one or more surface modification procedures, resulting in the formation of dual-functionalization of enrichment ligands 70 and amplification ligands 71 simultaneously. These ligands are used for the specific analysis of trace amounts of the analyte 20.

In another embodiment of the present invention, the analyte 20 to be tested can be a cell, an organelle, a bacterium, a virus, a protozoan, or a combination thereof. A portion of the solid-phase carriers 40 are enrichment bodies with at least one enrichment ligand 70, while another portion of the solid-phase carriers 40 are amplification bodies 42 with at least one amplification ligand 71. The enrichment ligand 70 is immobilized to the enrichment body, and the enrichment ligand 70 is used to specifically capture the analyte 20. The amplification ligand 71 is immobilized to the amplification body 42, and the amplification ligand 71 is used to bind to the biological substance 21 released by the analyte 20, or the amplicons 60 replicated on the amplification ligand 71 and subsequently released into the nucleic acid amplification solution 30.

In another embodiment of the present invention, the analyte 20 can be free deoxyribonucleic acid or a free ribonucleic acid. Moreover, free nucleic acid includes cell-free nucleic acid, free tumor nucleic acid, or a combination thereof. Each solid-phase carrier 40 includes the amplification body 42 and at least one amplification ligand 71, amplification ligand 71 is immobilized to the amplification body 42, and the amplification ligand 71 is used to bind to analyte 20, or the amplicons 60 replicated on the amplification ligand 71 and subsequently released into the nucleic acid amplification solution 30.

In each embodiment of the present invention, to allow the solid-phase carriers 40 to collaborate with different external energy excitation modes, various external excitation methods for the corresponding solid-phase carriers 40 are employed, including instant heating through photothermal conversion or magnetic hyperthermia under irradiations with the specific external energy. The solid-phase carrier 40 composed of specific material deposition or structure can be heated by using plasmonic heating under the light irradiations with a specific spectrum, or the solid-phase carrier 40 decorated with an organic photothermal conversion coating on the surface can be heated; alternatively, the solid-phase carrier 40 can be a magnetic carrier, enabling to produce the magnetically induced heating by magnetic solid carrier-mediated conversion of the AMF (alternating magnetic field). By placing the magnetic solid-phase carrier 40 in an externally applied magnetic field with an amplitude ranging from 0.5 to 550 kA/m and a frequency ranging from 3 to 3,500 kHz, the required localized temperature range on solid-phase carriers 40 for performing the nucleic acid denaturation, primer annealing, and polymerase extension can be achieved.

Furthermore, the multifunctional body 41, enrichment body, and amplification body 42 in the solid-phase carrier 40 can be of different configurations. The solid-phase carrier 40 can be implemented in the form of a spherical structure that includes a magnetic core 401 and an intermediate layer 402, which ensures the stability and maintenance of the outer photothermal conversion layer and can be made from metal or composite materials. The intermediate layer 402 can be a silicon shell, and its overall configuration can be spheres, ellipsoids, discs, stars, rods, squares, anisotropic spike structures (i.e., nanostar), nanoshells, nanocages, bipyramidal structures, micro filaments or a combination of two or more of these, but is not limited thereto; the magnetic core 401 includes transitional metals and their oxides, such as FeO, $Fe_2O_3$, $Fe_3O_4$, FeO(OH), $Fe(OH)_2$, $Fe(OH)_3$, CoO, CoO(OH), $Co_3O_4$ and their derivatives or their mixture, but the present invention is not limited thereto. The surface of the solid-phase carrier 40 is composed of a noble metal layer localized surface plasmon resonance (LSPR) effects, such as gold, silver, palladium, platinum, or a combination thereof, or an organic layer with efficient photothermal conversion, which may also incorporate materials that exhibit near-infrared spectroscopy (NIR) absorption spectra, such as cyanine, polypyrrole, or graphene.

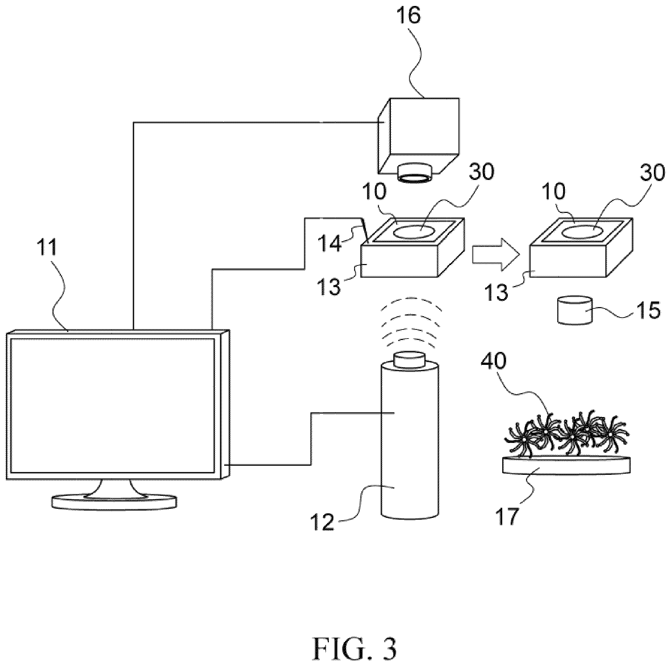

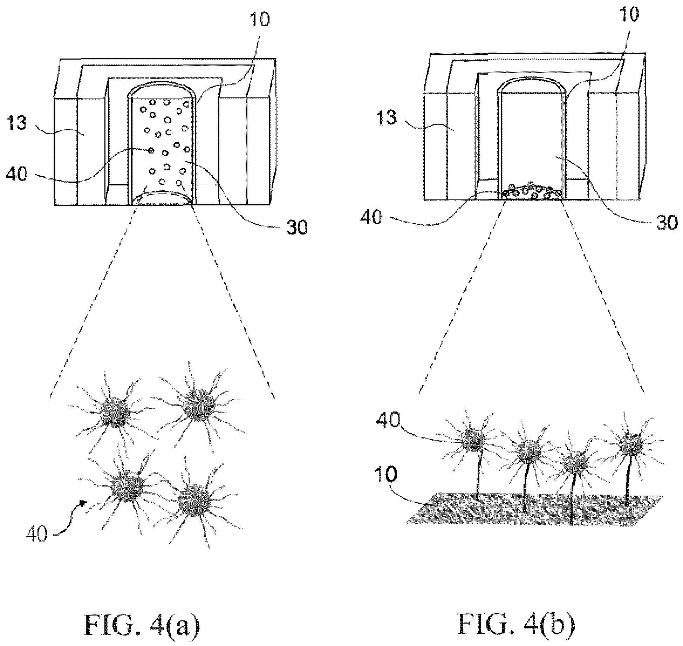

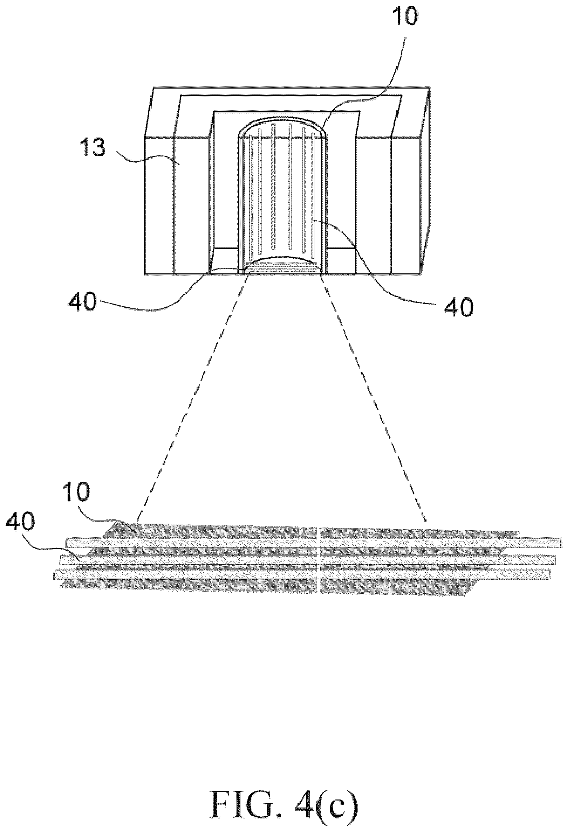

Please refer to FIG. 3, the present invention provides a rapid nucleic acid amplification system, including a reaction unit 10, an integrated driver 11, an energy excitation unit 12, and an auxiliary cooling unit. Among them, the reaction unit 10 is configured to accommodate the nucleic acid amplification solution 30 and a plurality of solid-phase carriers 40, wherein the solid-phase carriers 40 are suspended in nucleic acid amplification solution 30 [FIG. 4(a)], tethered to the inner wall of the reaction unit 10 [FIG. 4(b)], or embedded into the inner wall of the reaction unit 10 [FIG. 4(c)]. The auxiliary cooling unit can be in the following forms:

1. The nucleic acid amplification solution 30 (with a volume of at least 100 μL) serves as the auxiliary cooling unit. The nucleic acid amplification solution 30 is pre-cooled to the cooling temperature by using external auxiliary cooling unit 13 followed by being introduced into reaction unit 10, thus the nucleic acid amplification solution 30 in the reaction unit 10 can serve as the auxiliary cooling unit. In this formation, after the external energy is provided, the temperature of the nucleic acid amplification solution 30 will gradually increase but still remain at the cooling temperature during the reaction. Therefore, the efficiency of the nucleic acid amplification is worse than that of the auxiliary cooling unit described below, but the overall operation time is still shorter than the nucleic acid amplification of the conventional polymerase chain reaction.

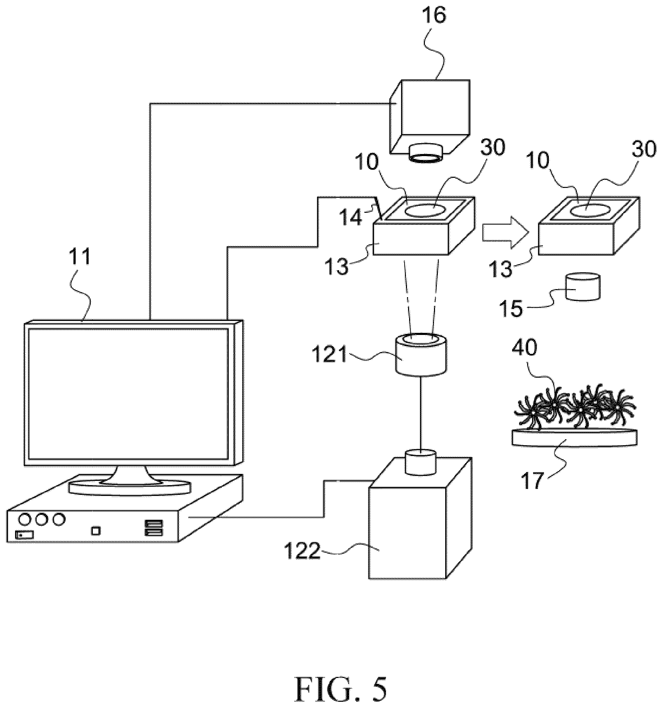

2. The nucleic acid amplification solution 30 and the external auxiliary cooling unit 13 collaborate with each other to form an auxiliary cooling unit, the external auxiliary cooling unit 13 is arranged around the reaction unit 10 (as shown in FIG. 5), and it continuously provides a cooling temperature to the reaction unit 10, resulting in the temperature of the nucleic acid amplification solution 30 within the reaction unit 10 drops and maintains at the cooling temperature, Besides, the temperature detection unit 14 (e.g., a K-type thermocouple) is integrated to the external auxiliary cooling unit 13 for performing instant temperature monitoring of the external auxiliary cooling unit 13 and temperature feedback control. This ensures that the nucleic acid amplification solution 30 inside the reaction unit 10 remains at a constant cooling temperature, enabling rapid nucleic acid amplification.

Moreover, the external auxiliary cooling unit 13 can be selected to use ice or the water-swelling macromolecular polymer with high specific heat capacity (e.g., carboxymethyl cellulose) or thermoelectric cooling chips (e.g., Peltier Cooler). The external auxiliary cooling unit 13 can also be water (i.e., 0.5 mL to 1 mL) or can be selected to contain the chemical endothermic reactants, the chemical endothermic reaction caused by its crystal dissolving in water, [e.g., ammonium nitrate molar heat of solution ($\Delta$Hsol) = 26.2 KJ, urea ($\Delta$Hsol = 15 KJ].

Please refer to FIG. 3, the energy excitation unit 12 directly and efficiently provides external energy to the solid-phase carrier 40, and converts it into thermal energy in a either contact or non-contact energy transfer manner. When the energy excitation unit 12 pauses to provide energy to the solid-phase carrier 40, the cooling temperature provided by the auxiliary cooling unit is used to rapidly dissipate the heat from the solid-phase carrier 40 and restrict the thermal radiation field generated by solid-phase carrier 40 upon the external energy irradiations. Through the coordinated control between the energy excitation unit 12 and the auxiliary cooling unit, the present invention enables the fast formation of the required temperature (thermal) cycling for performing nucleic acid amplification in the *in-situ* environment 50 surrounding the solid-phase carrier 40 and achieves *in-situ* nucleic acid amplification on the surface of the solid-phase carrier 40.

In the present invention, the contact excitation mode is electric heating. The electric energy transmission can be achieved through electric circuits or electromagnetic induction (wireless charging). The electric heating module can be Joule heating, thermoelectric heating, and surface acoustic waves (SAWs) to generate heat energy.

In the present invention, the preferred mode for the non-contact excitation to the solid-phase carrier 40 is plasmonic heating. As shown in FIG. 3. the energy excitation unit 12 is driven by an integrated driver 11 (e.g., comprising a computer and LabVIEW controlling software) to control the energy output and the timing sequence of laser on/off, resulting in the solid-phase carrier 40 to generate thermal change within localized thermal radiation field in the reaction unit 10.

Furthermore, the energy excitation unit 12 can trigger the solid-phase carrier 40 in a non-contact manner and it can be selected as photothermal irradiation mode. As shown in FIG. 5, the energy excitation unit 12 consists of a laser collimator 121 and a laser emitter 122, or an LED diode light source. Alternatively, the energy excitation unit 12 can use magnetically induced heating mode (e.g., magnetic solid carrier-mediated conversion of the AMF energy into induction heating) by using a high-frequency alternating magnetic field generator, but the actual implementation of the present invention is not limited thereto. The energy excitation unit 12 is primarily used for performing localized heating of the solid-phase carrier 40 itself and the surrounding *in-situ* environment 50 [i.e., within a range of hundreds of nanometers (nm) to hundreds of micrometers (μm)].

In the present invention, the rapid nucleic acid amplification system further includes a temperature detection unit 14 (e.g., K-type thermocouple) to detect the temperature feedback of the external auxiliary cooling unit 13; under the collaborated control of the nucleic acid amplification reaction in the auxiliary cooling unit and the heating provided by the energy excitation unit 12 to the solid-phase carrier 40, the required localized temperature (thermal) cycling for performing PCR is rapidly achieved, enabling the rapid completion of nucleic acid amplification.

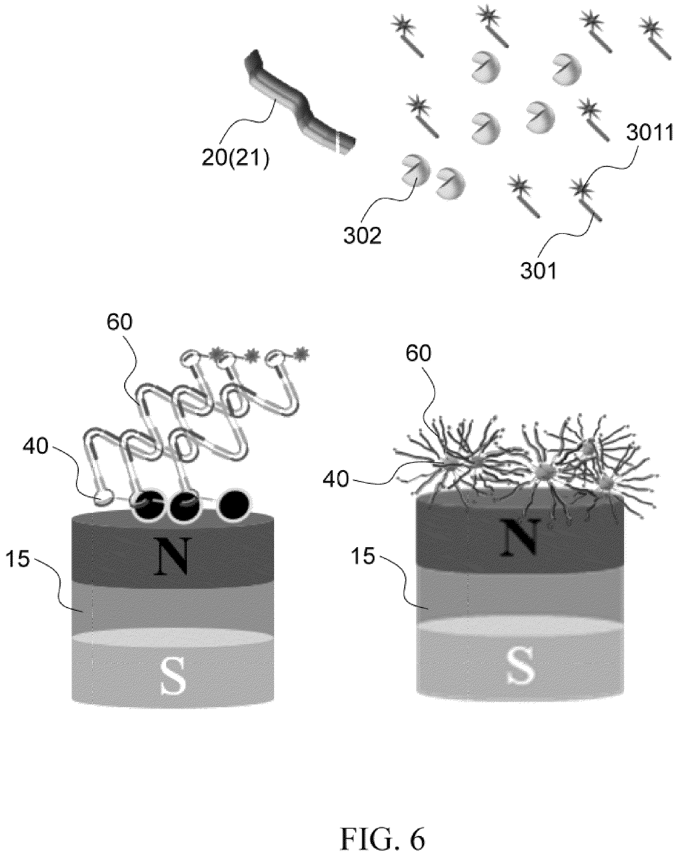

In the present invention, the rapid nucleic acid amplification system further includes an operation unit 15, which can be a permanent magnet, an electromagnet, or a combination thereof, but the present invention is not limited thereto; the operation unit 15 allows the solid-phase carrier 40 in the reaction unit 10 for performing the procedures of purification, separation, and concentration/enrichment via a simple magnetic manipulation, which will facilitate the subsequent detection and analysis of the amplicons 60 immobilized on the solid-phase carrier 40 (i.e., enhancing the signal readouts of the amplicons 60 in detection), the present invention is not limited thereto. As shown in FIG. 6, during the subsequent operation for the concentration/enrichment of the amplicons 60 immobilized on the solid-phase carrier 40, the amplicons 60 immobilized on the solid-phase carrier 40 can be separated from the nucleic acid amplification solution 30 through a simple magnetic capture. This purification and separation process not only removes interfering substances (e.g., non-specific amplicons, primers 301, or polymerase 302) that may affect the detection of nucleic acid molecules in the downstream detection analysis but also enhances the performance for subsequent molecular detection (e.g., analytical sensitivity).

It should be specifically noted that this invention can simplify temperature control devices and achieve localized temperature control with only a single energy excitation unit 12 and an external energy calibrator 16. The external energy calibrator 16 can be a light intensity meter calibrating the energy output of the energy excitation unit 12. By controlling the energy output and excitation timing sequence of the energy excitation unit 12 to achieve localized temperature (thermal) cycling control around the solid-phase carrier 40, forming an *in-situ* environment 50 around all the solid-phase carriers 40. This invention eliminates the need of the two-dimensional laser scanning with a two-dimensional mirror scanner, as disclosed in GNA's patent with the publication number US20140377764A1 (titled "Method for the amplification of nucleic acids using heat transfer for nanoparticles"), in which laser beams are selectively and specifically focused on a portion of nanoparticles in a PCR sample for 10 nanoseconds to 500 milliseconds.

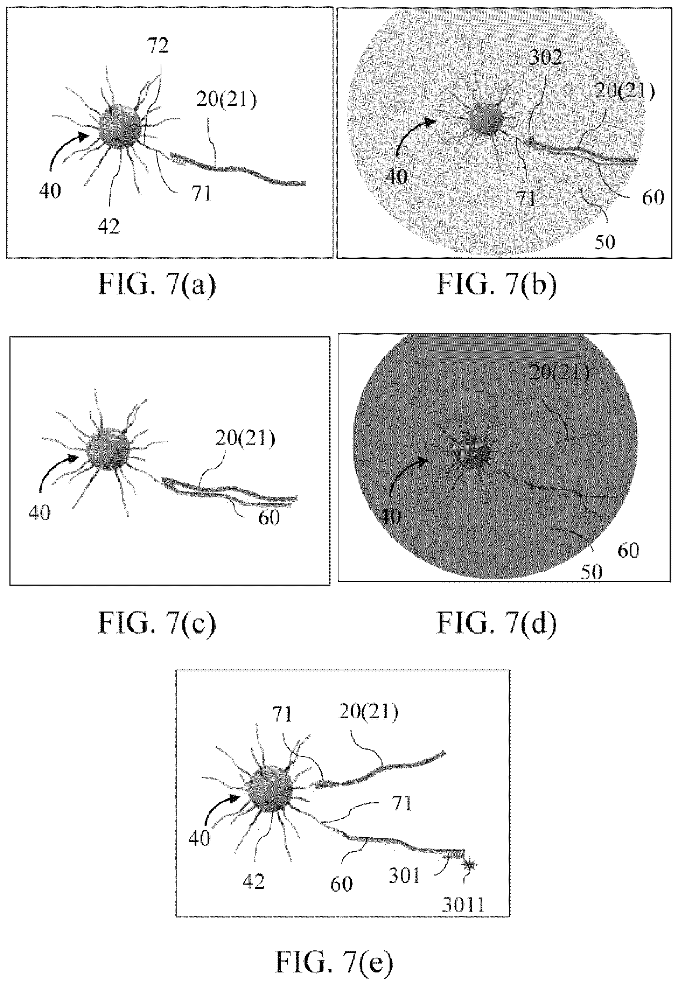

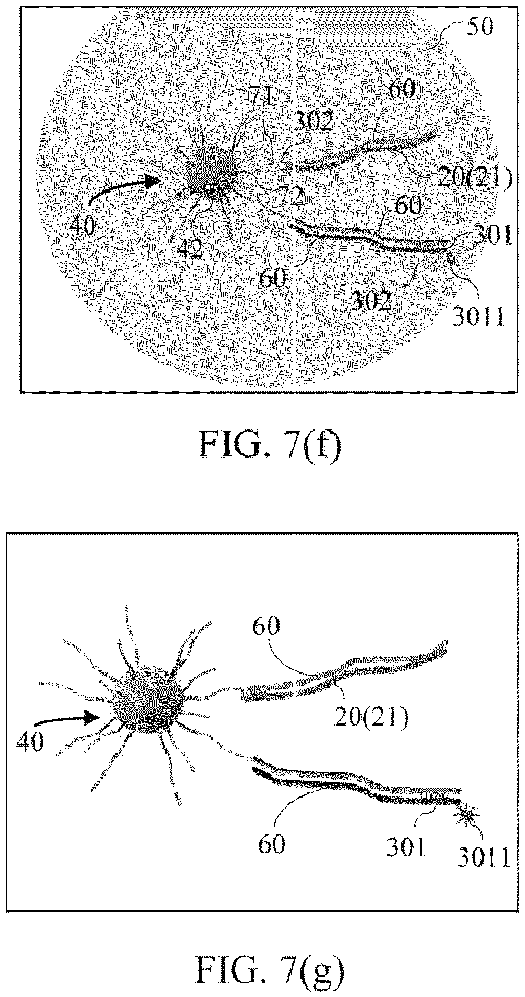

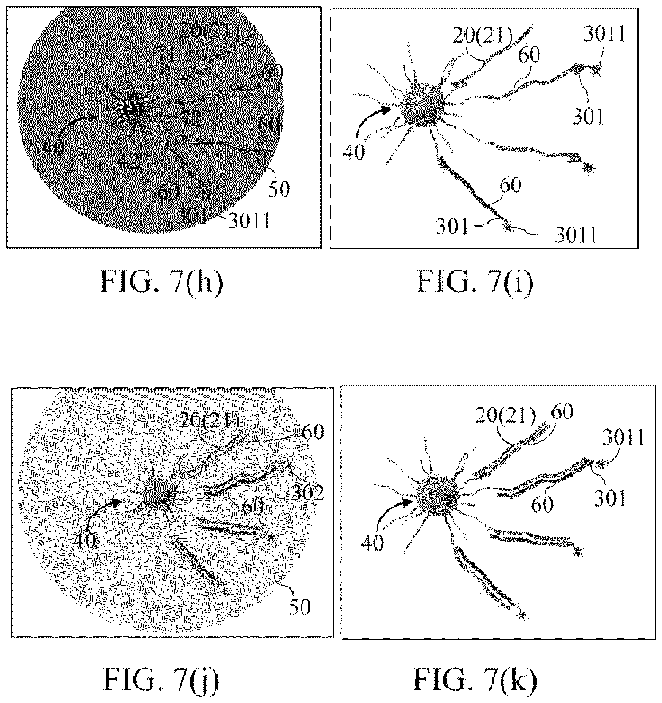

In one embodiment of the present invention, the analyte 20 is a free deoxyribonucleic acid, ribonucleic acid, or a biological substance 21 released from the analyte 20, and the solid-phase carrier 40 acts as an amplification body 42. Please refer to FIG. 7(a), each amplification ligand 71 is immobilized on the surface of the amplification body 42, and the amplification ligand 71 binds to the single strand of analyte 20 or biological substance 21 through hybridization capture. Please refer to FIG. 7(b), when the external energy triggers the solid-phase carrier 40 to generate an *in-situ* environment 50 for performing the first extension with the polymerase 302, the polymerase 302 in the nucleic acid amplification solution 30 used the single strand of analyte 20/biological substance 21 and the amplification ligand 71 as the DNA template and primer respectively to initiate the first extension, and then generating newly-synthesized amplicons 60. As shown in FIG. 7(c), after a brief pause of the external energy, the thermal field surrounding the solid-phase carrier 40 is rapidly cooled by the auxiliary cooling unit, resulting in the *in-situ* environment 50 dissipating rapidly. Referring to FIG. 7(d), the external energy source is turned on again to generate form the *in-situ* environment 50 for performing the denaturation step, causing the double-strand newly-synthesized amplicons 60 separate to form the single-strand analyte 20 and the single-strand amplicons 60. Please refer to FIG. 7(e), a brief pause of the external energy, which contributes to the *in-situ* environment 50 dissipating rapidly. The free primer 301 with the fluorophore 3011 in the nucleic acid amplification solution 30 is hybridized with the single strand of newly synthesized amplicons 60 immobilized on the solid-phase carriers 40, and the single strand originating from the analyte 20 re-annealed with another amplification ligands 71 immobilized on the solid-phase carriers 40.

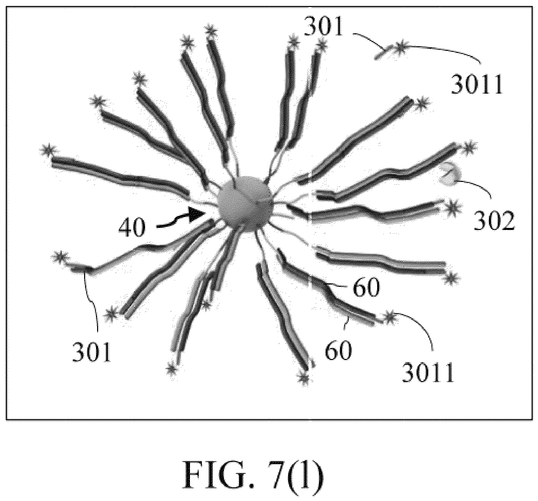

Please refer to FIG. 7(f) to (g), the external energy source is turned on again to generate the *in-situ* environment 50 for performing the primer annealing/extension step, the *in-situ* environment 50 could achieve and maintain the temperature higher the Tm (i.e., melting temperature) of free primer 301 and further reduce the non-specific primer binding might occur in FIG. 7(e). The single strand of the newly synthesized amplicons 60 immobilized to the amplification ligand 71, and the single-strand of analyte 20 re-annealed with another amplification ligands 71 act as the DNA templates for the polymerase 302, and the free primer 301 and amplification ligand 71 act as primers to initiate the extension of the polymerase 302 for another round of PCR nucleic acid amplification, resulting in more newly synthesized amplicon 60 generated on the solid-phase carriers 40. As shown in FIG. 7(h) to (k), another thermal cycle of *in-situ* environment 50 triggered by the energy excitation unit 12 (e.g., energy output and timing sequence of on/off for the external energy), initiating another round of nucleic acid amplification to generate the amplicons 60. As shown in FIG. 7(l), it represents the amount of the amplicons 60 is close to the saturation status of amplification ligand capacity on the solid-phase carrier 40 after performing multiple cycles of *in-situ* environment 50.

In one embodiment of the present invention, regarding the "photothermal" excitation of the solid-phase carrier 40, the surface of the solid-phase carrier 40 is preferably coated with a gold nanoshell 403. The gold nanoshell 403 includes a magnetic core 401 and an intermediate layer 402 between the gold nanoshell 403 and the magnetic core 401, collectively referred to as "magnetic gold nanoshell, MGN." The photothermal excitation can be achieved using a laser, an LED array, or a combination of both, the preferred external energy source is a laser, and the laser wavelength mainly ranges from visible spectrum to near-infrared spectrum (380 nm to 1.4 $\mu$m). Besides, the monodispersed suspension of magnetic gold nanoshells (MGNs) exhibits the characteristic of near-infrared absorbance (750 nm to 1.4 $\mu$m), particularly, the LSPR (localized surface plasmon resonance) absorbance peak is closed to the wavelength at 808 nm. Under irradiations with an 808nm laser, the MGNs can generate the photothermal conversion highly efficiently (i.e., plasmonic heating).

In another embodiment of the present invention, regarding the "alternating magnetic field (AMF)-induced heating of the solid-phase carrier 40", the solid-phase carrier 40 comprises a magnetic core 401. Under an applied alternating magnetic field, the solid-phase carrier 40 exhibits an induced heating effect that is mainly attributed to the magnetization reversal of the nanoscale structure, including the Néel relaxation mechanism (reorientation of the magnetization of the solid-phase carrier 40) and the Brown relaxation mechanism (physical rotation of the solid-phase carrier 40 in aqueous medium ). This heating capability depends on the characteristics of nanoscale structures, (e.g., average size, configuration, magnetization intensity, magnetic anisotropy), and the amplitude (Hac) and frequency (f) of the applied alternating magnetic field. The alternating magnetic field is generated by an alternating magnetic field generator, and the amplitude and frequency of the alternating magnetic field are set based on the field conditions required for the temperature needed for forming in-situ environment 50 by each solid-phase carrier 40. Moreover, the amplitude of the alternating magnetic field ranges from 0.5 to 550 kiloamps per meter (kA/m), and the frequency of the alternating magnetic field ranges from 3 to 3,500 kilohertz (kHz).

Furthermore, the solid-phase carrier 40 can be suspended with the nucleic acid amplification solution 30 and be a stable colloidal monodispersed suspension, in which the monodispersed status refers to a single dispersed suspension, and if the size of the solid-phase carrier 40 reaches the nanoscale, due to the surface potential of solid-phase carriers 40 it forms a stable colloidal monodispersed solution; the solid-phase carrier 40 can be a strong magnetic or superparamagnetic iron oxide solid-phase carrier (i.e., Superparamagnetic Iron Oxide Nanoparticles; SPIONs), especially $Fe_3O_4$ (magnetite), $\gamma$-$Fe_2O_3$, (maghemite) or spinel ferrite nanomaterials including $MIIFe_2O_4$ spinel ferrites (in which MII = $Co^{2+}$, $Ni^{2+}$, $Zn^{2+}$, $Mn^{2+}$). The addition of transition metal elements such as Ni, Co, etc mixed into the ferrite nanomaterials, can obtain a larger saturation magnetization, stable effective magnetic anisotropy (Keff), and stronger magnetization loss under the external alternating magnetic field.

The size of solid-phase carriers 40 for alternating magnetic field (AMF)-induced heating ranges from 8 to 1,000 nm. In addition to the common spheres, its shape type includes nanocubes, nanoctahedra, rods, disks, hollow spheres, stars, tetrapods, and so on, but not limited thereto. The amplitude (Hac) and frequency (f) of the applied alternating magnetic field can be set to a wide range with wide frequencies (ranging from 3 to 3,500 kHz) and field amplitudes (ranging from 0.5 to 550 kA/m), to generate the field conditions required for the temperature needed for forming in-situ environment 50 by each solid-phase carrier 40.

In the above embodiments, the surface modification of the multifunctional body 41, enrichment body, or amplification body 42 of the solid-phase carrier 40 further includes a surface-filling substance. The surface-filling substance is used to prevent aggregation of the solid-phase carriers 40, improve the hydrophilicity and confer the water-solubility of solid-phase carriers 40, reduce the adsorption inhibition of polymerase 302, and prevent non-specific adsorptions of proteins and primer 301 on the solid-phase carrier 40. The surface-filling substances can be polyethylene glycol (PEG) 72 with a molecular weight of 5 to 10 kDa and bovine serum albumin (BSA) 73. The enrichment ligand 70 or amplification ligand 71 can form stable covalent bonds with the functional groups provided by the surface-filling substances on the solid-phase carriers 40, the functional groups provided by surface-filling substance are preferred for carboxyl, amine, or thiol groups for perming chemical crosslinking in this embodiment.

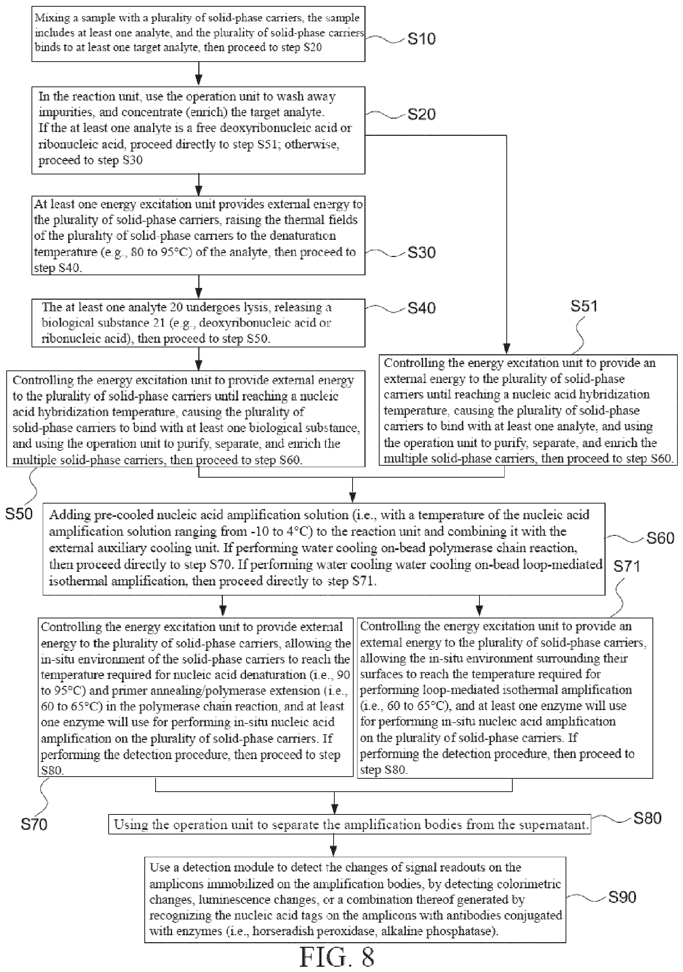

As shown in FIG. 8, the nucleic acid amplification method of the present invention can be performed using either water cooling on-bead polymerase chain reaction or water cooling on-bead loop-mediated isothermal amplification. The term "water cooling" refers to the cooling is achieved using the assistance of the aforementioned auxiliary cooling unit. The term "on-bead "refers to the performing in-situ nucleic acid amplification on the solid-phase carrier 40. The nucleic acid amplification method includes the following steps:

Step S10: Mixing a sample with a plurality of solid-phase carriers 40, the sample includes at least one analyte 20, and the plurality of solid-phase carriers 40 binds to at least one target analyte 20, then proceed to step S20.

Step S20: In the reaction unit 10, use the operation unit 15 to wash away impurities, and concentrate (enrich) the target analyte 20. If the at least one analyte 20 is a free deoxyribonucleic acid or ribonucleic acid, proceed directly to step S51; otherwise, proceed to step S30.

Step S30: At least one energy excitation unit 12 provides external energy to the plurality of solid-phase carriers 40, raising the thermal fields of the plurality of solid-phase carriers 40 to the denaturation temperature (e.g., 80 to 95°C) of the analyte 20, then proceed to step S40.

Step S40: The at least one analyte 20 undergoes lysis, releasing a biological substance 21 (e.g., deoxyribonucleic acid or ribonucleic acid), then proceed to step S50.

Step S50: Controlling the energy excitation unit 12 to provide external energy to the plurality of solid-phase carriers 40 until reaching a nucleic acid hybridization temperature, causing the plurality of solid-phase carriers 40 to bind with at least one biological substance 21, and using the operation unit 15 to purify, separate, and enrich the multiple solid-phase carriers 40, then proceed to step S60.

Step S51: Controlling the energy excitation unit 12 to provide an external energy to the plurality of solid-phase carriers 40 until reaching a nucleic acid hybridization temperature, causing the plurality of solid-phase carriers 40 to bind with at least one analyte 20, and using the operation unit 15 to purify, separate, and enrich the multiple solid-phase carriers 40, then proceed to step S60.

Step S60: Adding pre-cooled nucleic acid amplification solution 30 (i.e., with a temperature of the nucleic acid amplification solution 30 ranging from -10 to 4°C) to the reaction unit 10 and combining it with the external auxiliary cooling unit 13. If performing water cooling on-bead polymerase chain reaction, then proceed directly to step S70. If performing water cooling water cooling on-bead loop-mediated isothermal amplification, then proceed directly to step S71.

Step S70: Controlling the energy excitation unit 12 to provide external energy to the plurality of solid-phase carriers 40, allowing the *in-situ* environment 50 of the solid-phase carriers 40 to reach the temperature required for nucleic acid denaturation (i.e., 90 to 95°C) and primer annealing/polymerase extension (i.e., 60 to 65°C) in the polymerase chain reaction, and at least one enzyme will use for performing *in-situ* nucleic acid amplification on the plurality of solid-phase carriers 40. If performing the detection procedure, then proceed to step S80.

Step S71: Controlling the energy excitation unit 12 to provide an external energy to the plurality of solid-phase carriers 40, allowing the *in-situ* environment 50 surrounding their surfaces to reach the temperature required for performing loop-mediated isothermal amplification (i.e., 60 to 65°C), and at least one enzyme will use for performing *in-situ* nucleic acid amplification on the plurality of solid-phase carriers 40. If performing the detection procedure, then proceed to step S80.

Step S80: Using the operation unit 15 to separate the amplification bodies 42 from the supernatant.

Step S90: Use a detection module 17 to detect the changes of signal readouts on the amplicons 60 immobilized on the amplification bodies 42, by detecting colorimetric changes, luminescence changes, or a combination thereof generated by recognizing the nucleic acid tags on the amplicons 60 with antibodies conjugated with enzymes (i.e., horseradish peroxidase, alkaline phosphatase).

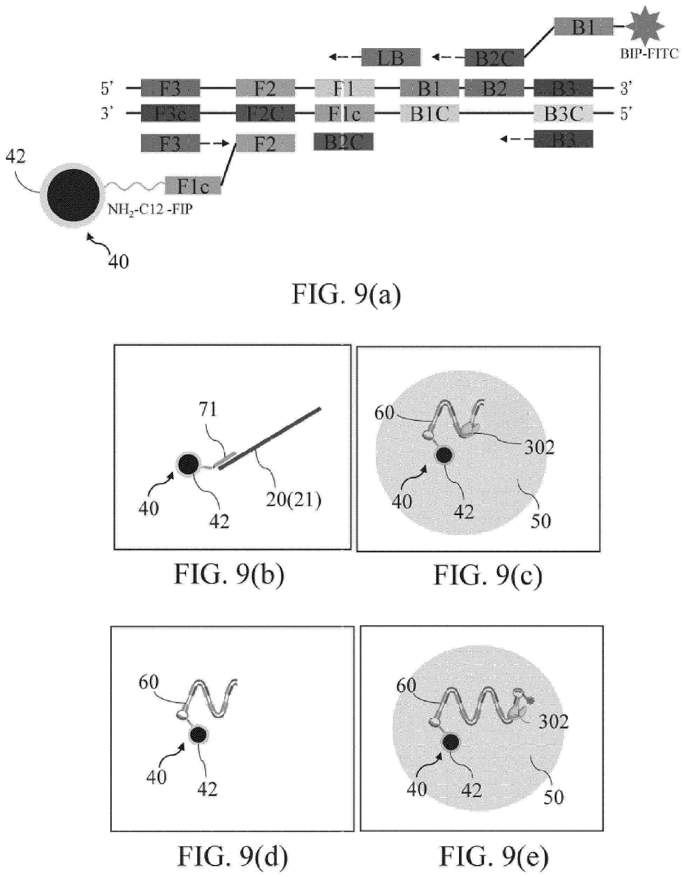

In one embodiment, the water cooling on-bead loop-mediated isothermal amplification (LAMP) of the present invention is performed. As shown in FIG.9 (a), the solid-phase carriers functionalized with at least one of specific primers 301 (i.e., FIP, BIP) and act as the amplification body 42 for performing water cooling on-bead loop-mediated isothermal amplification. As shown in FIG.9 (b), the target nucleic acid sequence can be released from the analyte 20 or the biological substance 21. In this embodiment, the at least one pair of specific primers 301 includes three primer pairs (FIP, BIP, LF, LB, B3, and F3), enabling water cooling on-bead loop-mediated isothermal amplification on the amplification body 42. Firstly, the external energy excitation unit 12 is turned off, and the amplification body 42 will capture the target nucleic acid sequence through DNA hybridization by the functionalized specific primers 301. The nucleic acid sequence can be the analyte 20 or the biological substance 21 [as shown in FIG. 9(b)]. Then the external energy excitation unit 12 turns on for short intervals (e.g., 0.5 to 3 seconds), the amplification body 42 generates instantly a thermal field with a temperature range suitable for performing loop-mediated isothermal amplification, and the initial extension is performed by the polymerase 302 [as shown in FIG. 9(c)]. Subsequently, the external energy excitation unit 12 is turned off for short intervals (e.g., 0.5 to 1 second), and the thermal field generated by the amplification body 42 will dissipate rapidly and stop the enzyme activity of polymerase 302 for performing the loop-mediated isothermal amplification [as shown in FIG. 9 (d)]. The steps from FIG. 9(b) to FIG. 9(d) are cyclically repeated until the amplicon 60 reaches saturation status of the capacity of the amplification body 42 [as shown in FIG. 9(e)]. After the water cooling on-bead LAMP is completed, the amplicons 60 of the amplification body 42 can be concentrated (enriched) and purified by the operation unit 15 subsequently. This purification and separation procedure for the amplification body 42 is also used to assist in the specific detection of the amplicons 60 after water cooling on-bead LAMP/RT-LAMP. True positive" amplicons 60 with the "inner region" between the 3' ends of FIP and BIP primers for LAMP/RT-LAMP reactions can be isolated to move away nonspecific detection through magnetic capture, which will enhance the analytical specificity for the detection of the amplicons 60 generated by water cooling on-bead LAMP/RT-LAMP and reduce the occurrence of false positives in detection.

Furthermore, according to the disclosure of U.S. patent with publication number US20140377764A1 by GNA Biosolutions, the occurrence of "localized heating" on the photothermal solid-phase carrier 40 is based on the following conditions. When the interval of energy excitation is shorter or equal to the critical excitation interval t1, the critical excitation interval t1 can be represented by the following equation:

$$t1 = \frac{(s1 \times |x|)^2}{D} \; ;$$

t1: represents the time required for heat to diffuse from one nanoparticle to the next nanoparticle at the average distance between nanoparticles;

s1: represents a scaling factor that measures how far the heat front of a solid-phase carrier 40 spreads during the excitation duration (i.e., the critical excitation interval t1). When localized heating occurs on the solid-phase carrier 40 upon light irradiation, s1 is less than or equal to 1;

|X| represents the average distance between each solid-phase carrier 40;

D represents the thermal diffusion coefficient of the medium between solid-phase carriers 40.

According to the conditions disclosed in the aforementioned patent, it is required to use the two-dimensional laser scanning controlled with a complex and sophisticated two-dimensional mirror scanner, in which laser beams are selectively and specifically focused on a portion of plasmonic nanoparticles within the nucleic acid solution 30 for 10 nanoseconds to 500 milliseconds. Therefore, in the overall implementation of the aforementioned patent, it is necessary to precisely control the actions as mentioned above to control the time and relative positions of light irradiations of the plasmonic particles within the nucleic acid solution 30, which are critical factors for the successful implementation of the PCA technology.

In contrast, the present invention provides a relatively easy strategy to control the excitation of the solid-phase carrier 40 by external energy, resulting in the generation of localized thermal radiation surrounding the solid-phase carrier 40. Firstly, the present invention uses solid-phase carriers 40 suspended in the nucleic acid amplification solution 30, with a ratio of the total volume of solid-phase carriers 40 to the volume of the nucleic acid solution 30, ranging from 1:200 to $1:1 \times 10^9$. The preferred volume ratio is $1:1 \times 10^4$ to $1:1 \times 10^8$, meaning that the distance |X| between the solid-phase carriers 40 and the distance of heat front of the solid-phase carrier 40 spreading during the critical excitation interval t1 excitation (i.e., the s ; the scaling factor) are significantly increased. Therefore, the longer critical excitation intervals t1 can be used to modulate the laser irradiations on the solid-phase carriers 40 to generate localized thermal radiation surrounding the solid-phase carriers 40.

Moreover, the nucleic acid amplification solution 30 is placed within an external auxiliary cooling unit 13, which provides a cooling environment to significantly constrict the spreading of localized thermal field (i.e., in-situ environment 50) generated by the solid-phase carriers 40 upon laser irradiations, ensuring that the localized thermal fields generated by different solid-phase carriers 40 do not overlap each other and remain in an independent state. In view of this, the present invention does not require controlling for the relative movement between the energy excitation unit 12 and the reaction unit 10 (i.e., To control the irradiation direction of laser beams on a portion of plasmonic nanoparticles within the nucleic acid solution 30 with very short intervals), and can use a relatively long excitation intervals (ranging from one second to several tens of seconds) to excite "all "suspended solid-phase carriers 40 and the solid-phase carriers 40 are tethered or the solid-phase carriers 40 are embedded in the inner wall of the reaction unit 10. Additionally, during each activation of the energy excitation unit 12, there is no implementation of controlling relative movement between the energy excitation unit 12 and the reaction unit 10. The above-mentioned characteristics have a distinct difference from those disclosed in the U.S. patent (with publication number US20140377764A1) of the GNA Biosolutions.

Due to the high surface area to volume ratio of the solid-phase carriers 40, the solid-phase carriers 40 not only serve as efficient platforms for separating/concentrating the amplicons 60 of the analyte 20 but also can be used to remove potential inhibitory substances originating from the analyte 20. In addition, the in-situ nucleic acid amplification is performed in the in-situ environments 50 formed on the surrounding space of the solid-phase carriers 40. Since the limited capacity of immobilized amplicons 60 on the surface of the solid-phase carriers 40, which enables to achieve saturation status of the capacity of the solid-phase carriers 40 in relatively short operation times (or a relatively small number of nucleic acid amplification cycles). This will facilitate the subsequent detection of amplicons 60.

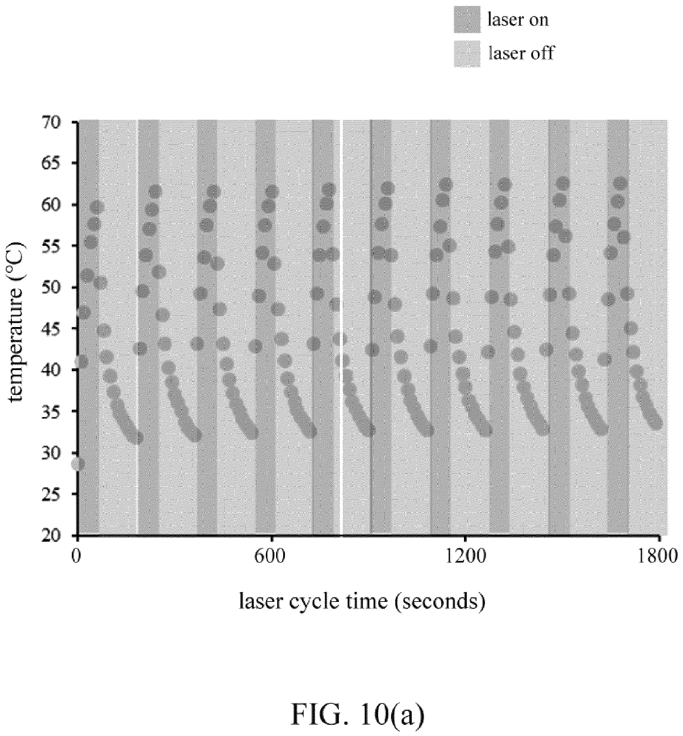

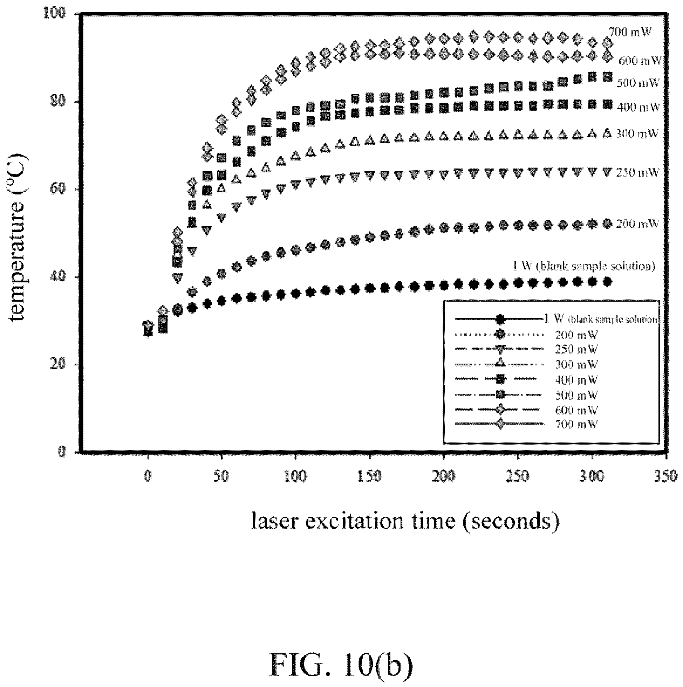

Please refer to FIG. 10(a) and FIG. 10(b), in the embodiments of the present invention, the selected magnetic gold nanoshells (MGNs) are selected as the solid-phase carriers 40, which exhibit excellent characteristics of photothermal conversion and photothermal stability, and the optimal localized surface plasmon resonance (LSPR) wavelength of MGNs is located from 780 to 1,000 nm, with the preferred range from 800 to 950 nm. In this embodiment, the evaluation of the micro-scale photothermal temperature measurement of monodispersed MGN suspensions (i.e. with a particles concentration at $2.9 \times 10^{10}$ particles/mL, 20ul) was performed under a 808nm laser irradiations with consecutive 10 photonic cycles. As shown in FIG. 10(a), the 808 nm fiber-coupled laser is controlled by the integrated driver 11 driven by the LabVIEW software to performed a consecutive 10 photonic cycles (i.e., the setup of the photonic cycle, NIR laser on at 400mW/0.16cm$^2$ for 60 seconds, and laser off for 120 seconds). The highest photothermal converted temperature of the monodispersed MGN suspension was measured at 62.29 ± 1.06°C during consecutive 10 photonic cycles, and its coefficient of variation of 1.70%. This result demonstrates that MGNs exhibit excellent photothermal stability, no significant change on photothermal temperature caused by the configuration changes of the solid-phase carriers 40 under laser irradiations with high-power. As shown in FIG. 10(b), the result of the micro-scale photothermal temperature measurement of the solid-phase carriers (i.e. magnetic gold nanoshell, $2.9 \times 10^{10}$ particles /ml, 20 μL) under NIR laser irradiations with different excitation powers (i.e., 200 mW/0.16 cm$^2$, 250 mW/0.16 cm$^2$, 300 mW/0.16 cm$^2$, 400 mW/0.16 cm$^2$, 500 mW/0.16 cm$^2$, 600 mW/0.16 cm$^2$, 700 mW/0.16 cm$^2$) for 300 seconds. Besides, the micro-scale photothermal temperature measurement of the blank sample solution without MGNs under laser irradiations with 1 W/0.16 cm$^2$ was also included as a control group. The photothermal temperatures monitoring of the monodispersed MGN suspension was measured to steady-state heating (i.e., the time of laser irradiations is more than 180 seconds). The results demonstrate that MGNs has the excellent photothermal conversion characteristics of the MGNs, and the photothermal temperature at steady state is proportional to the input laser power.

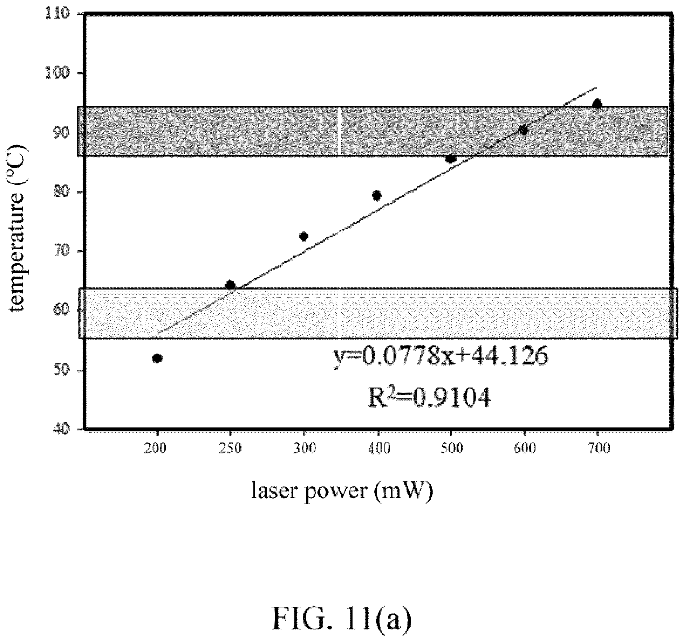

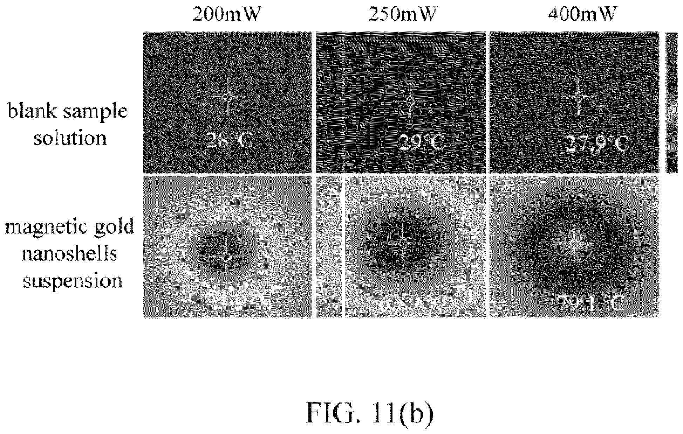

Please refer to FIG. 11(a) and FIG. 11(b), by means of photothermal temperature measurements at the microscale of the monodispersed MGNs suspensions, based on the result of the micro-scale photothermal temperature measurement of the solid-phase carriers as mentioned in FIG. 10(b). We established the linear calibration curve of the applied NIR laser power intensity and the micro-scale photothermal converted temperature of the monodispersed MGNs suspensions (i.e., the linear equation: y = 0.0778x + 44.126; $R^2$ = 0.9104). The upper panel of FIG. 11(a), which represents the temperature range required for performing the cell thermal lysis and DNA denaturation (85 to 95 °C), and the lower panel of FIG. 11(a) represents the temperature range required for performing the primer annealing/polymerase extension in PCR or polymerase extension in loop-mediated isothermal amplification (LAMP) (55 to 65 °C) in one embodiment of the present invention. Furthermore, the photothermal converted temperature at the microscale will be used to estimate the actual temperature range of the *in-situ* environment 50 for performing nucleic acid amplification. As shown in FIG. 11(b), the infrared thermal images of the monodispersed MGN suspension under NIR laser irradiations with different powers (i.e., 200 mW/0.16 cm$^2$, 250 mW/0.16 cm$^2$, 400 mW/0.16 cm$^2$). The photothermal converted temperature at the steady state of the MGN suspensions in the reaction unit10 was measured and recorded immediately with the thermal camera under laser irradiations. The upper panel of FIG. 11(b) shows the infrared thermography results of the blank sample solution irradiated with an 808 nm laser powers (i.e., 200 mW/0.16 cm$^2$, 250 mW/0.16 cm$^2$, 400 mW/0.16 cm$^2$), and the photothermal

converted temperatures at the steady-state range from 27.9 to 29 °C, which is close to the ambient temperature of the environment. The photothermal converted temperature at the steady-state of the monodispersed MGN suspension is 51.6°C, 63.9°C and 79.1°C respectively under laser irradiations with different powers (i.e., 200 mW/0.16 cm$^2$, 250 mW/0.16 cm$^2$, 400 mW/0.16 cm$^2$).

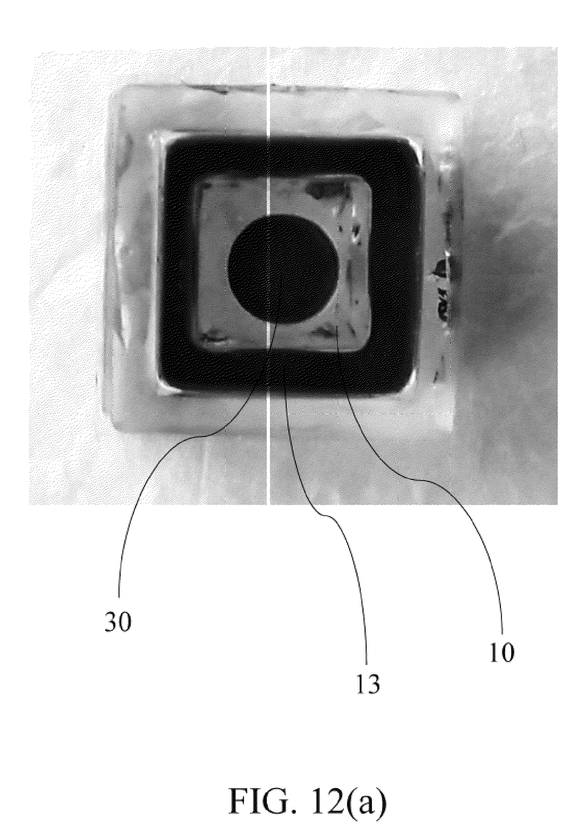

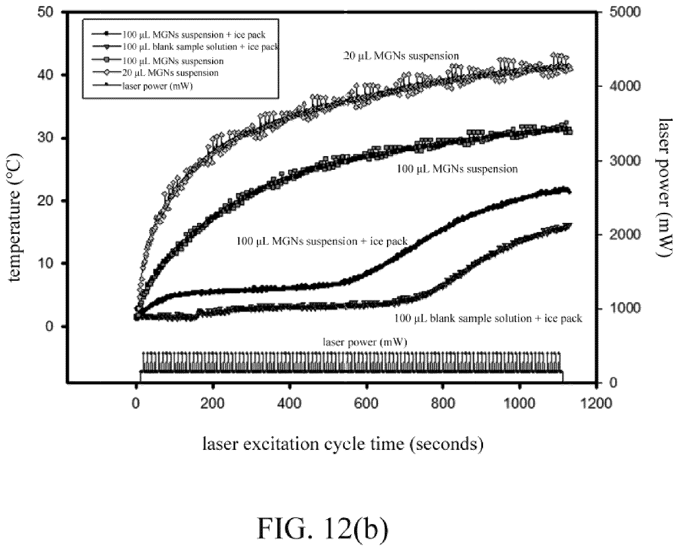

Please refer to FIG. 12(a) and FIG. 12(b). This is an embodiment of the present invention regarding the layout of the auxiliary cooling unit. The overall temperature changes of the nucleic acid amplification solution including monodispersed MGNs under the NIR laser irradiations with 100 photonic cycles (i.e., each photonic cycle is modulated by the integrated driver at 400 mW/0.16 cm$^2$ for 1.25 seconds, followed by 0.5 seconds of laser off, and then at 150 mW/0.16 cm$^2$ for 7.5 seconds of irradiation). As shown in FIG. 12(a), the layout of the auxiliary cooling unit in this embodiment includes a large volume of nucleic acid amplification solution 30 (i.e., the volume is 100 μL) and the external auxiliary cooling unit 13. In this embodiment, the external auxiliary cooling unit 13 uses the material with high heat capacity (i.e., ice, from 550 μL of water) to surround the reaction unit 10 including the nucleic acid amplification solution 30, which is facilitated to maintain the cooling temperature (i.e., 2 to 10 °C) of the nucleic acid amplification solution 30 within the reaction unit 10. As shown in FIG. 12(b), the overall temperature monitoring of the nucleic acid amplification solution suspended with MGNs at the number of $5.8 \times 10^8$ particles is measured and recorded on the nucleic acid solution 30 with different volumes of the pre-cooled nucleic acid solution 30 (i.e., 20 and 100 uL) during the periods of 100 photonic cycles of laser irradiations. Grouping is based on whether the external auxiliary cooling unit 13, as depicted in FIG. 12 (a), is utilized. Besides the nucleic acid amplification solution 30 acts as a control group. And then, it is also evaluated whether the above-mentioned combination of operating conditions can maintain the temperature of the nucleic acid amplification solution 30 within range of the cooling temperature (i.e., 2 to 10 °C) under how many external laser irradiation cycles (i.e., photonic cycle). Furthermore, it also evaluated whether the temperature of the nucleic acid amplification solution 30 changes along with the cycle of the NIR laser irradiations. The experimental groups included 100 μL MGNs suspension with the external auxiliary cooling unit 13, 100 μL blank sample solution with the external auxiliary cooling unit 13, 100 μL MGNs suspension without the external auxiliary cooling unit 13, and 20 μL MGNs suspension without the external auxiliary cooling unit. As shown in FIG.12 (b), the temperatures of the nucleic acid amplification solutions and the laser irradiation cycle correspond to the Y-axes on the left and right, respectively, and the Y-axes on the left and right correspond to the temperature of the nucleic acid amplification solution (°C) and the laser power (mW). The results show that the experimental control group of the large volume nucleic acid amplification solution 30 (i.e., 100 μLMGNs suspen-

sion) with the external auxiliary cooling unit 13, which can maintain the temperature of the nucleic acid amplification solution 30 within the cooling temperature range (2 to 10 °C) and no significant temperature fluctuation during the period of 65 photonic cycles. Furthermore, under the coordination of operating conditions of the photonic cycle, a large volume of nucleic acid amplification solution 30 and the external auxiliary cooling unit 13, our results demonstrate that the heating mode of the MGNs induced by laser irradiations belongs to a "localized heating" mode, in which the heating is constricted to the *in-situ* environment 50.

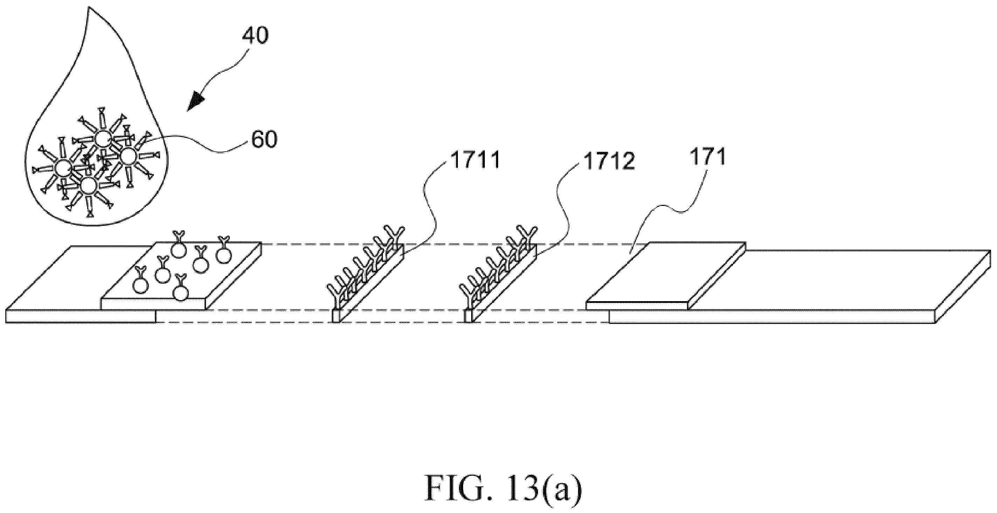

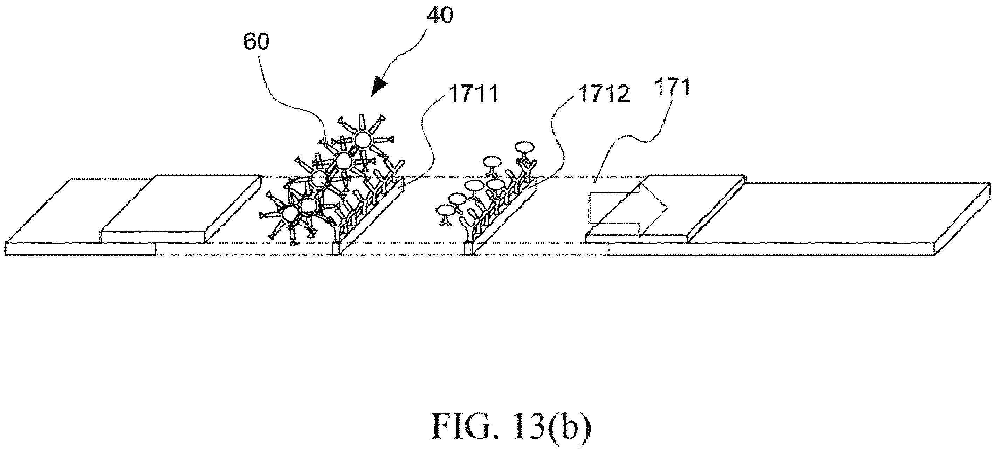

FIG. 13(a) and FIG. 13(b) illustrates an embodiment of the present invention, using a lateral flow immunoassay strip 171 to detect the amplicons 60 generated by the water cooling on-bead polymerase chain reaction. As shown in FIG. 13(a), after the water cooling on-bead polymerase chain reaction, owing to the magnetic properties of MGNs, the amplicons 60 immobilized on the MGNs not only be used to purify and concentrate/enrich by the magnetic operation unit 15 but also can remove the non-specific nucleic acid products and other substances (e.g., primers 301 or polymerase 302) present in the nucleic acid amplification solution 30. After the procedure of magnetic separation, the purified and concentrated amplicons 60-immobilized MGNs were resuspended with TBST buffer, and then the fast detection was performed using a lateral flow immunoassay strip 171. The lateral flow immunoassay strip 171 consists of different porous membranes on which liquids migrate by capillarity. As shown in FIG.13(a) and FIG. 13(b), in this embodiment, the amplicons 60 immobilized on the MGNs are incorporated with nucleic acid tags, which are the primers 301 labeled with fluorescein isothiocyanate (FITC). During the detection with the lateral flow immunoassay strip 171, the liquids containing the amplicons 60 immobilized on the MGNs are migrated through the conjugation pad containing specific antibodies conjugated colloidal golds, the liquid mixture comprises the released antibodies conjugated colloidal golds and the amplicons 60 immobilized on the MGNs migrate along the strips into the detection zone, in which the specific capture antibodies are dispensed onto the porous membrane to form the test line 1711 (i.e., the depositions of anti-fluorescein isothiocyanate antibodies) and the control line 1712 (i.e., the depositions of anti-mouse antibodies). While the antibody recognition of the above-mentioned liquid mixture occurs on the test line and the control line, resulting in the readouts for visual inspection occurring on its corresponding regions (e.g., the line formed at the test line 1711 or control line 1712). This detection procedure can be completed within 3 to 5 minutes, and the response on the control line indicates the proper liquid flow through the lateral flow immunoassay strip 171.

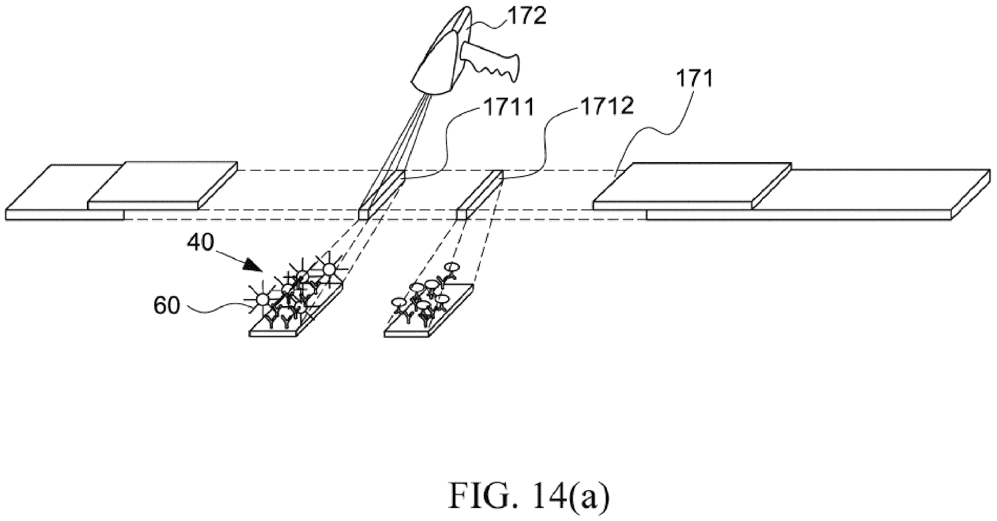

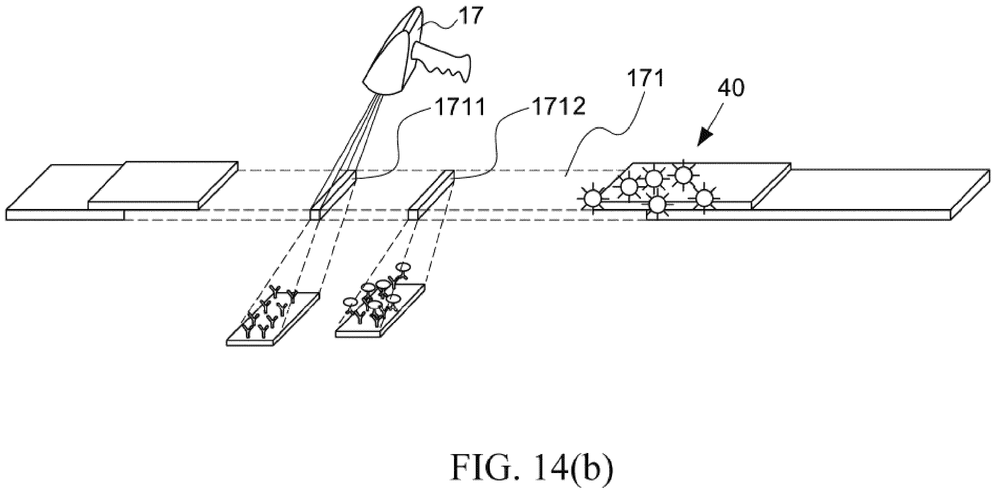

FIG. 14(a) and FIG. 14(b) represents another embodiment of the present invention, by integrating the plasmonic thermal sensing and the lateral flow immunoassay strip 171 as illustrated in FIG.13(b). The technology of plasmonic thermal sensing can improve the analytical sensitivity of conventional lateral flow immunoassay strip 171. FIG. 14 (a) shows that the amplicons 60 immobilized on the MGNs are incorporated with the primer 301 labeled with fluorescein isothiocyanate (FITC). In the detection using lateral flow immunoassay strip 171, the nucleic acid tags of amplicon 60 immobilized on the MGNs are recognized and captured by the anti-fluorescein isothiocyanate antibody dispensed on the test line 1711 of the lateral flow strip 171, resulting in the readouts for visual inspection. Owing to the unique LSPR property of MGNs in the near-infrared region, the MGNs deposited on the test line 1711 of the lateral flow immunoassay strips 171 can generate plasmonic heating under the NIR laser irradiations. In this embodiment of the present invention, the NIR laser-coupled infrared thermal sensor 172 is integrated with the lateral flow immunoassay strip 171 for performing plasmonic thermal sensing. As compared with the analytical sensitivity of the lateral flow immunoassay strip 171, the plasmonic thermal sensing has better sensitivity on result interpretation in the lateral flow immunoassay strip 171. FIG. 14(b) is a schematic diagram of a negative result of water cooling on-bead nucleic acid amplification detected by integrating the plasmonic thermal sensing and the lateral flow immunoassay strip as illustrated in FIG. 13(b). Since no amplicon 60 immobilized on the MGNs are recognized and captured by the anti-fluorescein isothiocyanate antibody dispensed on the test line 1711, no significant plasmonic heating occurring on the test line 1711 under detection with the NIR laser-coupled infrared thermal sensor 172.

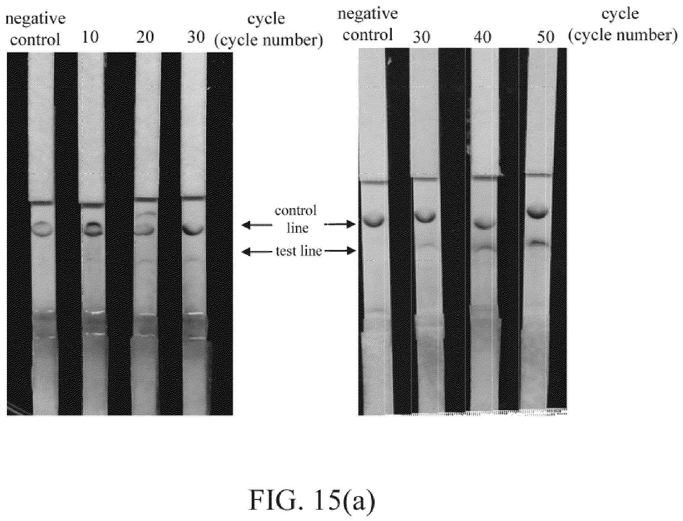

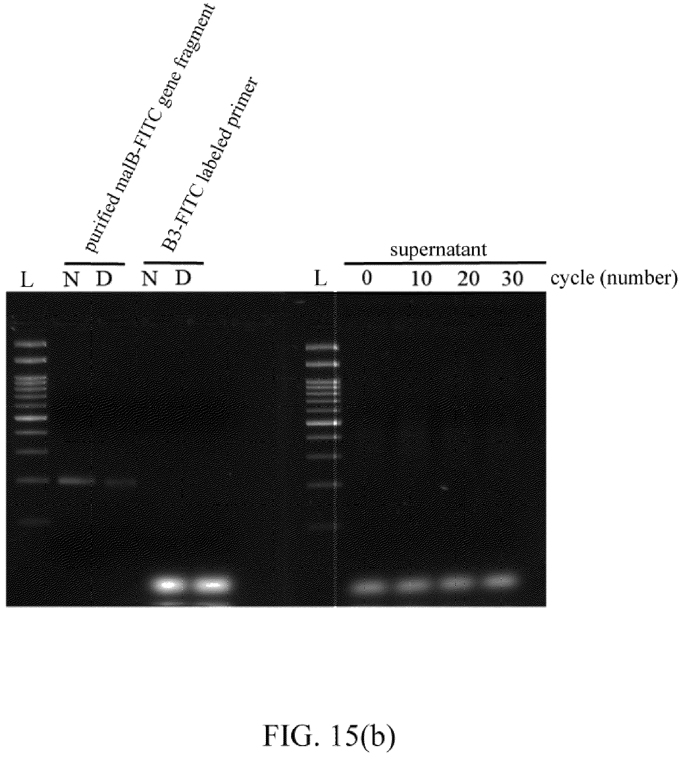

Please refer to FIG. 15(a) and FIG. 15(b). This is an embodiment of the present invention for performing *in-situ* amplification and subsequent detection with the water cooling on-bead PCR and the lateral flow immunoassay strips. In this embodiment, the *malB* gene fragment used for conventional PCR detection of *Escherichia coli* (*E. coli* ATCC35218) is selected as the detection target. The performance evaluation of the water cooling on-bead PCR at various numbers of photonic cycles (i.e., ranging from 10 to 50 cycles; each photonic cycle is modulated by the integrated driver at 400 mW/0.16 cm² for 1.25 seconds, followed by 0.5 seconds of laser off, and then at 150 mW/0.16 cm² for 7.5 seconds of irradiation) is analyzed with the lateral flow immunoassay strip 171. In addition to analyzing the amplicons derived from the *malB* gene on the MGNs (i.e., the solid-phase carriers 40), the amplicons 60 derived from the *malB* gene in the nucleic acid amplification solution 30 are also analyzed with the urea agarose gel. As shown in FIG. 15(a), as the number of photonic cycles increases, the visual inspection readouts on the test line 1711 of the lateral flow immunoassay strip 171 are also gradually increased. The issue of the low amplification performance results from the very short time intervals for NIR laser modulations in the photonic cycle in super amplification, which can be improved by increasing the number of photonic cycles. As shown in FIG. 15(b), the amplicons derived from the *malB* gene in the nucleic acid amplification solution 30 are also analyzed with the urea agarose gel. Firstly, the operation unit 15 is used to separate the MGNs and the supernatant of the nucleic acid amplification solution 30, the DNA in the supernatant of nucleic acid solution 30 is concentrated with the ethanol precipitation, and then the concentrated DNA is treated with high-concentration urea and heat to denature the DNA. Subsequently, the denaturing urea agarose gel electrophoresis is performed to analyze whether any residual amplicons 60 are in the supernatant of the nucleic acid amplification solution 30. The purified *malB*-FITC fragments are the amplicons of the *malB* gene labeled with FITC, used as a positive control for the amplicon 60. The purified B3-FITC labeled primer is the B3 primer labeled with FITC, used as a positive control of the primer. Our results showed that only free primer 301 was detected in the supernatant after various photonic cycles ranging from 10 to 30 cycles, and no amplicon 60 was present in the supernatant of the nucleic acid amplification solutions 30. This indirectly proves that the nucleic acid amplification occurred on the MGNs; In FIG. 15(b), the DNA ladder is added as a reference marker for DNA molecular weight, indicated by "L"; additionally, the samples without heating and denaturation were labeled as "N" in FIG. 15(b), while those subjected to heating and denaturation were labeled as "D. "

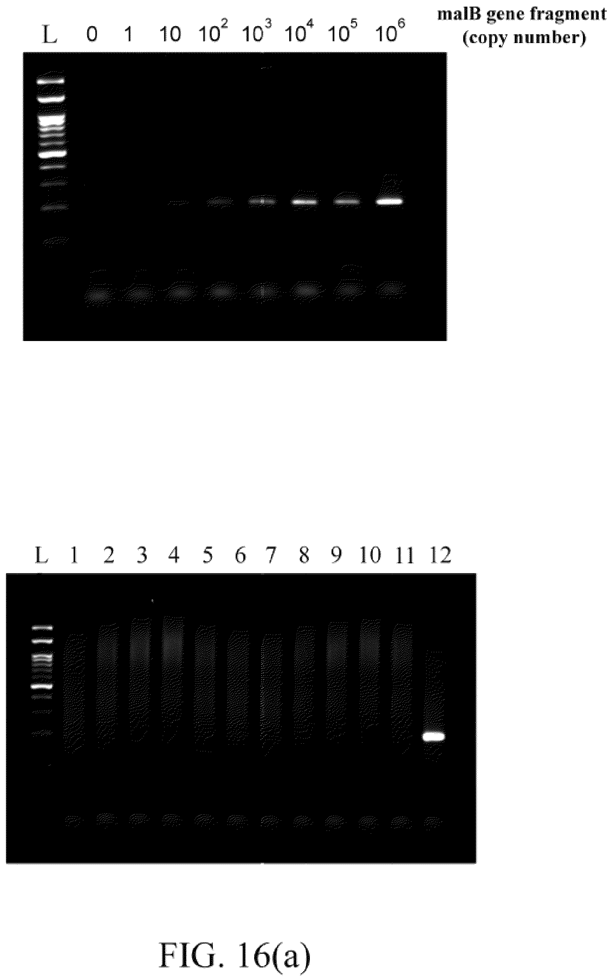

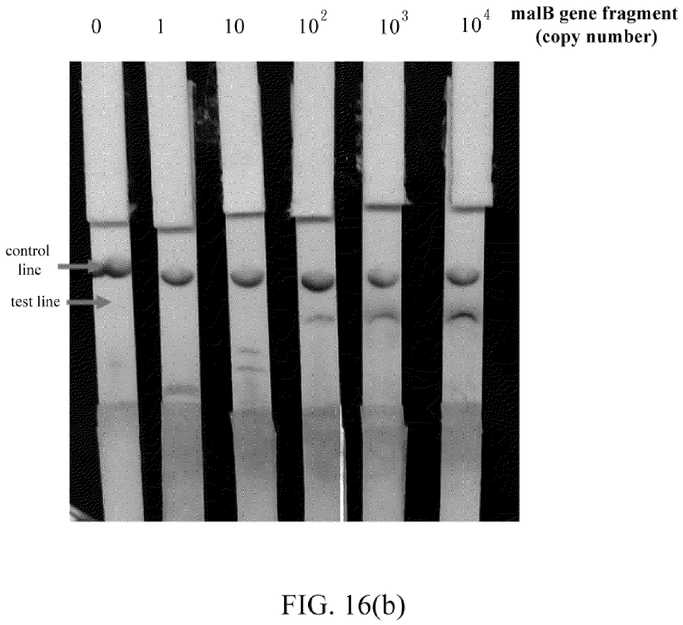

Please refer to FIG. 16(a) and FIG. 16(b). This is an embodiment of the present invention. The performance comparison of the conventional polymerase chain reaction and the water-cooling on-bead polymerase chain reaction for detecting the *malB* gene was evaluated with the simple DNA samples. The simple DNA samples mean that the genomic DNAs are derived from their corresponding microbial strains. As shown in the upper panel of FIG. 16 (a), the analytical sensitivity of the conventional polymerase chain reaction was evaluated with the genomic DNA containing various copy numbers (i.e., 0, 10, $10^2$, $10^3$, $10^4$, $10^5$, and $10^6$ copies of the *malB* gene of *Escherichia coli* ) under 40 thermal cycling (i.e., the setup of each thermal cycling: 95°C for 15 seconds, followed by 60°C for 30 seconds), and then the *malB* amplicons 60 were analyzed with the agarose gel. As shown in the lower panel of FIG. 16(a), besides the analytical specificity of the conventional polymerase chain reaction for detecting the *malB* gene was also evaluate with no template control (NTC) and genomic DNAs derived from other microbes. The lower panel of FIG. 16(a) shows the DNA molecular weight standard on the left, followed by simple samples no template control, *Acinetobacter baumannii, Pseudomonas aeruginosa, Klebsiella pneumoniae, Staphylococcus epidermidis, Staphylococcus aureus* ATCC: BAA977, *Streptococcus agalactiae, Enterococcus faecalis, Mycobacterium abscessus, Candida albicans, Aspergillus niger,* and *Escherichia coli* ATCC: 35218 from left to right. Simple samples are labelled 1 to 12 from left to right. Our results showed that the LOD (limit of detection) of the conventional polymerase chain reaction for detecting the *malB* gene with the simple DNA samples ranged from 10 to $10^2$ copies per reaction and the conventional polymerase chain reaction for detecting the *malB* gene has superior analytical specificity. Similarly, the analytical sensitivity of the water cooling on-bead polymerase chain reaction combined with the lateral flow immunoassay strip was evaluated with the genomic DNA containing various copy numbers of the *malB* gene from 0, 1, 10, $10^2$, $10^3$, and $10^4$ copies, which corresponded to 0, 0.00675, 0.0675, 0.675, 6.75, and 67.5 picograms (pg) of genomic DNA derived from the *Escherichia coli*. As shown in FIG. 16(b), the results showed that the LOD of the water cooling on-bead PCR combined with the lateral flow immunoassay strip ranged from 10 to $10^2$ copies per reaction, which is comparable to the analytical sensitivity of the conventional PCR for detecting *malB* gene.

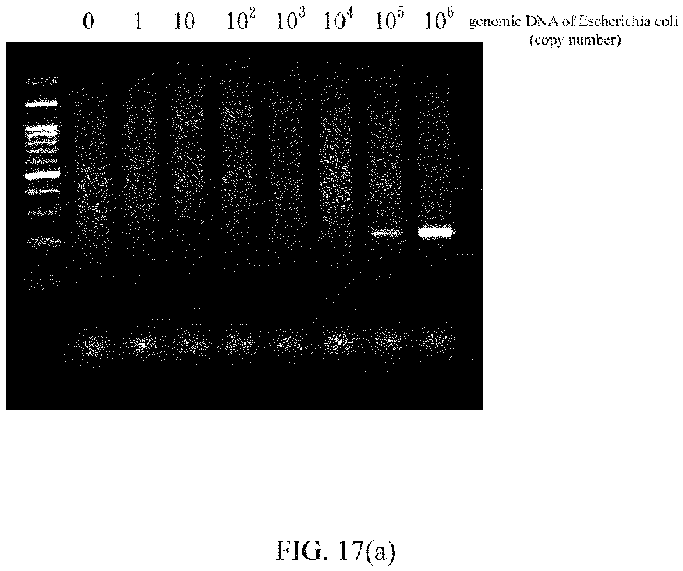

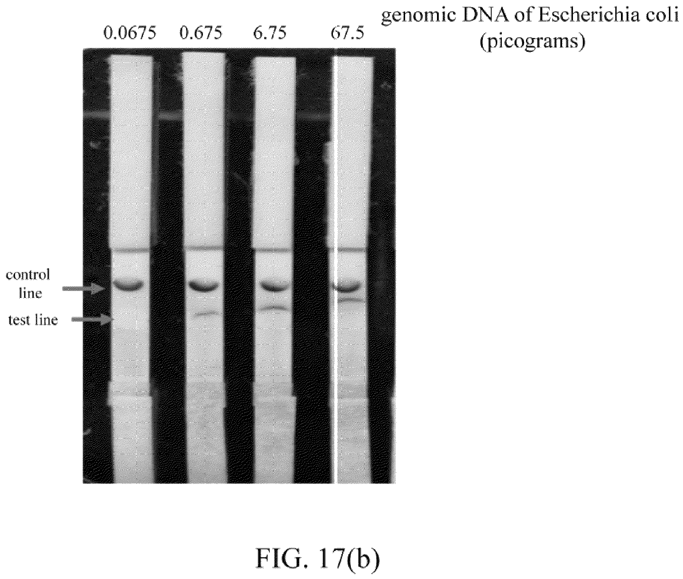

Please refer to FIG. 17(a) and FIG. 17(b). This is an embodiment of the present invention. The performance comparison of the conventional polymerase chain reaction and the water-cooling on-bead polymerase chain reaction was evaluated for detecting the *malB* gene with the complex DNA samples. The complex DNA samples include apart from the genomic DNA of *E. coli* (ATCC: 35218), the genomic DNAs derived from five other common pathogenic microorganisms in humans, including *Acinetobacter baumannii, Pseudomonas aeruginosa, Staphylococcus aureus* (ATCC: BAA977), *Streptococcus agalactiae,* and *Enterococcus faecalis.* As shown in FIG. 17 (a), the analytical sensitivity comparison of conventional polymerase chain reaction for detecting the *malB* gene was evaluated with the genomic DNA containing various copy numbers of the *malB* gene (i.e., 0, 1, 10, $10^2$, $10^3$, $10^4$, $10^5$ and $10^6$ copies) mixed with 6.75 ng (i.e., equivalent to $10^6$ copies) of the genomic DNA derived from the above-mentioned five other common pathogenic microorganisms. After 40 thermal cyclings (i.e., the setup of each thermal cycling: 95°C for 15 seconds, followed by 60°C for 30 seconds), and then the *malB* amplicons 60 were analyzed with the agarose gel. Our results showed that the LOD of the conventional polymerase chain reaction for detecting the *malB* gene is significantly decrease by as low as $10^5$ copies per reaction. As shown in FIG.17 (b), similarly, the analytical sensitivity of the water cooling on-bead polymerase chain reaction combined with the lateral flow immunoassay strip 171 for detecting the *malB* gene was evaluated with the above-mentioned complex DNA samples. Our results showed that the LOD of the water cooling on-bead polymerase chain reaction combined with the lateral flow immunoassay strip still remained at 10 and $10^2$ copies per reaction. Furthermore, our result demonstrated that the analytical sensitivity of the water cooling on-bead polymerase chain reaction combined with the lateral flow immunoassay strips 171 is not affected by the complexity of nucleic acids originating from the analytes 20.

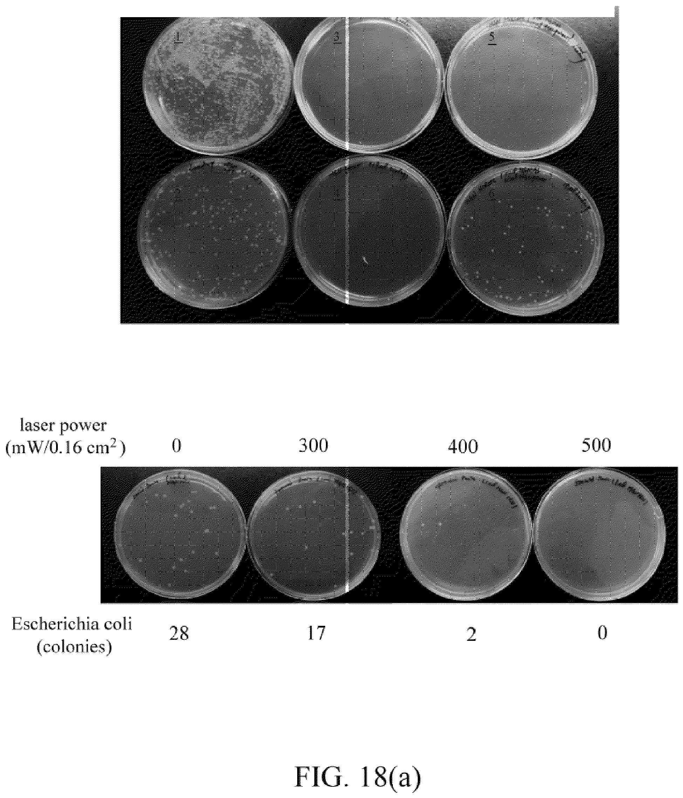

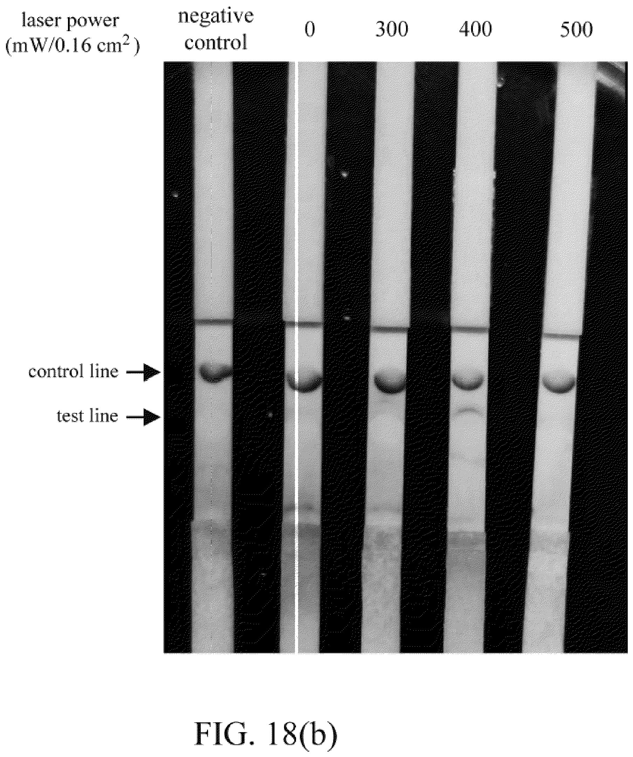

Please refer to FIG. 18(a) and FIG. 18(b). This is an embodiment of the present invention, which is the target enrichment and photothermal lysis performance evaluation of the suspensions mixing with the functional ligands immobilized solid-phase carriers 40 (i.e., the enrichment bodies and the amplification bodies 42). The colony-forming-units (CFUs) testing is used to assess the capture efficiency rate of target analytes 20 (e.g., *E. coli*) and the photothermal lysis efficiency under NIR irradiations with different power densities (i.e., 0 to 500 mW/0.16 $cm^2$ for 300 seconds). Firstly, in one embodiment of the present invention, the experiment was performed by using a mixed suspension including the enrichment bodies and the amplification bodies 42. Both the enrichment bodies and the amplification bodies 42 belong to the magnetic gold nanoshells (MGNs). The enrichment bodies are used to capture specifically the *E. coli* cells (i.e., the target analytes 20), and the captured *E.coli* on the enrichment bodies are purified and separated through magnetic capture with the operation unit 15, and then the captured target analytes 20 on the enrichment bodies are subjected to photothermal lysis, wherein the analytes 20 is the *E. coli* cells (ATCC:35218) suspended in buffer or biosamples, the procedures of photothermal lysis results in the release of biological substances 21 from the *E. coli.* The amplification bodies 42 are used to bind the biological substances 21 released from *E. coli* through the hybridization capture. As shown in FIG. 18(a), the mixed suspension includes the enrichment bodies and amplification bodies 42. The enrichment bodies are functionalized with the enrichment ligands 70 (i.e., antibodies specifically against the O and K antigens of *E. coli*), and the amplification bodies 42 are functionalized the amplification ligands 71 (i.e., the *malB* F3 primer 301 can specifically hybridize the sequences within the *malB* gene unique to *E. coli*). As shown in the upper panel of FIG.18(a), in this embodiment, a mixture of 20 μL of the mixed suspensions includes an equal amount of enrichment bodies and amplification bodies 42, 1 μL of *E. coli* solution (i.e., at $OD_{600}$ at 0.01; $8 \times 10^3$ CFUs ), and 19 μL of TBST buffer containing 1% BSA. After the procedures of the immunocapture at room temperature for 20 minutes, the enrichment bodies and amplification bodies 42 will be purified and concentrated with the magnetic operation unit 15, followed by washing with TBST buffer. The captured *E.coli* on the enrichment bodies will be evaluated by using the colony-forming unit testings, our results confirm the specific enrichment with the enrichment bodies can achieve low background and highly specific capture of *E. coli.* However, if the above-mentioned steps of this embodiment are performed with a suspension containing only the amplification bodies 42, the *E. coli* cannot be effectively captured, which provides evidence that the specific capture of E. *coli* merely occurs on the enrichment bodies. As shown in the lower panel of FIG. 18(a), in another embodiment of the present invention, the photothermal lysis performance of the enrichment bodies was evaluated by using the suspensions containing the captured *E. coli* on the enrichment bodies triggered under 808 nm laser irradiations at different powers (i.e., 0 mW/0.16 $cm^2$, 300 mW/0.16 $cm^2$, 400 mW/0.16 $cm^2$, and 500 mW/0.16 $cm^2$) for 5 minutes. After the procedure of photothermal lysis, the suspensions containing the captured *E. coli* on the enrichment bodies and free enrichment bodies were cultured on the LB plates, and the survival rate of the captured *E.coli* on the enrichment bodies was determined by directly counting visible colonies of bacterial growth on the plate. The efficiency of photothermal lysis of the enrichment bodies on thermally induced cell death of *E. coli* was evaluated by using the colony-forming units testing. If no CFUs on the culture plates, it indicates that the captured *E. coli* on the enrichment bodies had been completely lysed through the procedure of photothermal lysis. Our result showed that the continuous laser irradiation with 400 mW/0.16 $cm^2$ for 5 minutes is sufficient to lyse the most of captured *E. coli* on the enrichment bodies. As shown in FIG. 18 (b), the photothermal lysis performance of the mixed suspensions with the enrichment bodies and the amplification bodies 42 under various laser powers are evaluated, in this embodiment, the performance evaluation integrates the water cooling on-bead PCR with the lateral flow immunoassay strip 171. After the procedures of photothermal lysis under various laser powers, the biological substances 21 containing the *malB* gene fragments are released from *E. coli* (ATCC: 35218). The *malB* gene fragments are further recognized and captured by the amplification ligands 71 functionalized on the amplification bodies 42, and the *malB*-captured amplification bodies 71 are subjected to laser excitation for performing the water cooling on-bead polymerase chain reaction. Subsequently, the signal readouts on the test lines of the lateral flow immunoassay strips 171 are utilized to assess the photothermal lysis efficiency of the enrichment bodies and the amplification bodies 42 under the NIR laser irradiations with different powers. Our result showed that continuous laser irradiation with 400 mW/0.16 $cm^2$ for 5 minutes is the best condition for performing the photothermal lysis. This result is consistent with the result of above-mentioned colony-forming-units (CFUs) testing in FIG.18 (a).

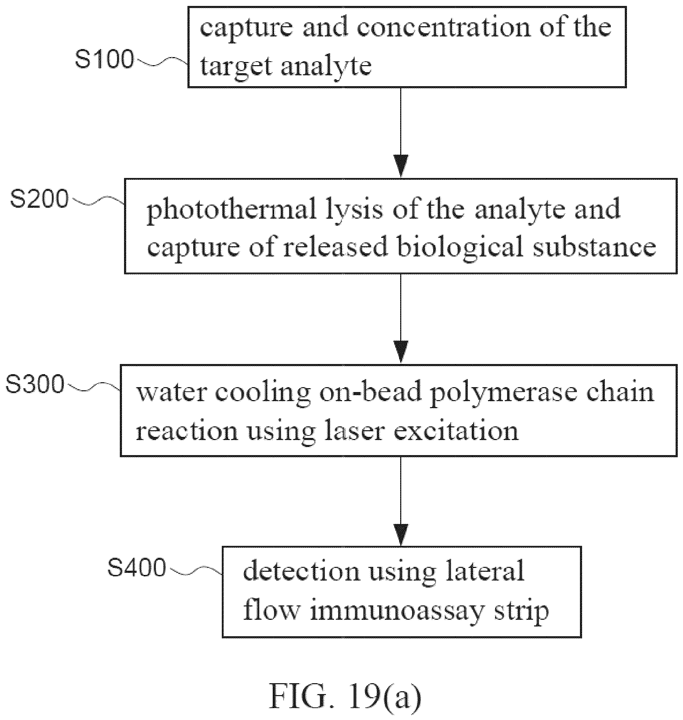

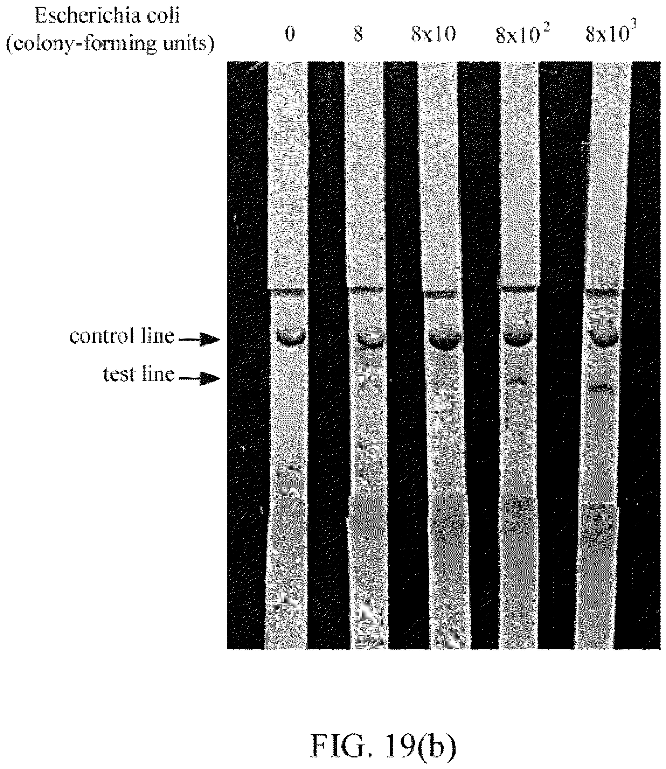

Please refer to FIG. 19(a) and FIG. 19(b). This is an embodiment of the present invention using the suspension includes the mixed suspensions of enrichment bodies and amplification bodies 42 for performing the overall procedures of sample pretreatment (i.e., the processes of target analyte 20 enrichment and photothermal lysis), the water cooling on-bead PCR, and the lateral flow immunoassay strips 171. In this integrated platform, the main bodies of the enrichment bodies and the amplification bodies 42 belong to the magnetic gold nanoshells (MGNs), and the enrichment ligands 70 are functionalized with the antibodies specifically against the O antigens and K antigens of *E. coli,* and the amplification ligands 71 are functionalized with the F3 primer 301 (i.e., the *malB* F3 primer) capable of recognizing and capturing the sequences of the *malB* gene. The cell number of *E.coli* (*E.coli* ATCC: 35218) was estimated from the optical density at 600nm (i.e., OD600=1, $8 \times 10^8$ CFU/ml), and then various amounts of *E. coli* were spiked into the TBST buffer as the analytes 20 (i.e., 0 CFUs, 8 CFUs, $8 \times 10$ CFUs, $8 \times 10^2$ CFUs, and $8 \times 10^3$ CFUs). The water cooling on-bead polymerase chain reaction combined with the lateral flow immunoassay strip 171 was utilized to evaluate the analytical sensitivity and overall turnaround time of the integrated platform. As shown in FIG. 19 (a), the flowchart of operating procedures of the integrated platform includes at least four key steps: "the process of capture and concentration of the target analyte 20," "the process of the photothermal lysis of the analyte and capture of released biological substance 21," "water cooling on-bead polymerase chain reaction using laser excitation," and "detection using the lateral flow immunoassay strip 171."

In the process of "capture and concentration of the target analyte 20," 5 μL of the suspension of the enrichment bodies, 15 μL of the suspension of the amplification bodies 42, and 1 μL of the analyte 20, were added into 19 μL of TBST buffer including 1% bovine serum albumin, and then incubated at 25°C with shaking for 20 minutes. This allows the amplification ligands 71 functionalized on the amplification bodies 42 to capture the cells of *E. coli,* subsequently, the magnetic separation is used to concentrate (enrich) the enrichment bodies and amplification bodies 42 with the operation unit 15, and remove the supernatant and wash away impurities with the TBST buffer. This process can be completed within 25 minutes.

In the process of "photothermal lysis of the analyte and capture of released biological substance 21". After the process of "capture and concentration of the target analyte 20," the mixed enrichment bodies and the amplification bodies 42 were resuspended in 20 μL of TBST buffer, and then those were subjected to continuous 808 nm laser irradiations at 400 mW for 5 minutes to perform photothermal lysis, and then the biological substances 21 are released from the lysed analyte 20. The released biological substances 21 are bound by the amplification ligands 71 on the amplification bodies 42 through the hybridization capture. The magnetic operation unit 15 is used to concentrate the enrichment bodies and amplification bodies 42, and remove the putative PCR inhibitory substances originating from target analytes 20 in the supernatant. Subsequently, add pre-cooled 100 μL of nucleic acid amplification solution 30 to resuspend the mixed enrichment bodies and amplification bodies 42.

In the process of "water cooling on-bead polymerase chain reaction using laser excitation". After the process of "photothermal lysis of the analyte and capture of released biological substance 21", the mixed enrichment bodies and the amplification bodies 42 are resuspended in the pre-cooled nucleic acid amplification solution 30 or placed in an external auxiliary cooling unit 13, wherein the pre-cooled reaction unit 10 or the external auxiliary cooling unit 13 maintains the cooling temperature, and then those are excited under the NIR laser irradiations with 40 photonic cycles (i.e., each photonic cycle is modulated by the integrated driver at 400 mW/0.16 cm$^2$ for 1.25 seconds, followed by 0.5 seconds of laser off, and then at 150 mW/0.16 cm$^2$ for 7.5 seconds of irradiation). After the water cooling on-bead polymerase chain reaction. The magnetic operation unit 15 is then used to concentrate the enrichment bodies and amplification bodies 42 and remove the supernatant, subsequently, add 20 μL of TBST buffer to resuspend the mixed enrichment bodies and the amplification bodies 42. This process can be completed within 6 minutes.

In the process of "detection using lateral flow immunoassay strip 171". After the process of "the water cooling on-bead polymerase chain reaction using laser excitation", the mixed enrichment bodies and the amplification bodies 42 are resuspended in TBST buffer, those are subjected to detection using the lateral flow immunoassay strip 171. The signal readouts on the test line 1711 of the lateral flow immunoassay strip 171 can be directly observed with the visual inspection or those can be detected using plasmonic thermal sensing with the NIR laser-coupled infrared thermal sensor 172. This process can be completed within 3 to 5 minutes. The overall process of the integrated platform can be completed within 45 minutes.

As shown in FIG. 19(b), the overall process illustrated in FIG. 19(a) were completed, including the process of the water cooling on-bead polymerase chain reaction and subsequent detection with the lateral flow immunoassay strip 171. Our results revealed that the signal readouts could be visually observed on the test line 1711 of the lateral flow immunoassay strip 171 with the analytical sensitivity at 8 CFU of *E. coli* per reaction.

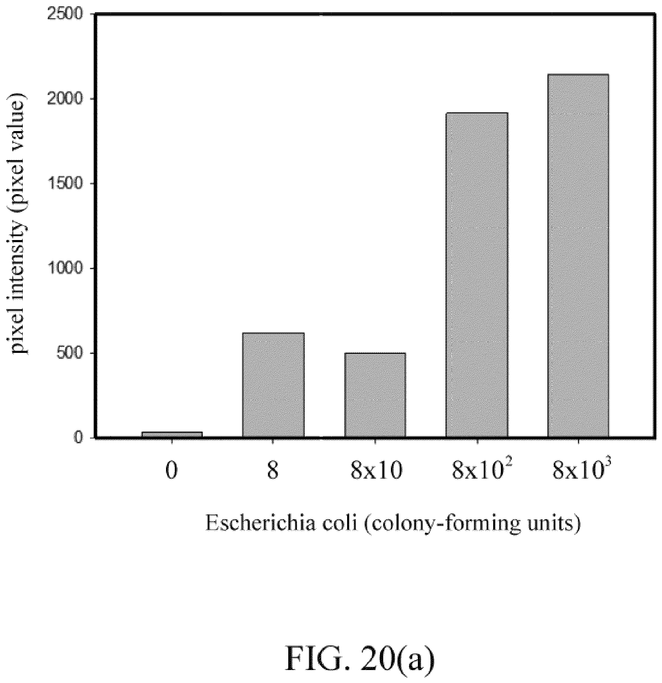

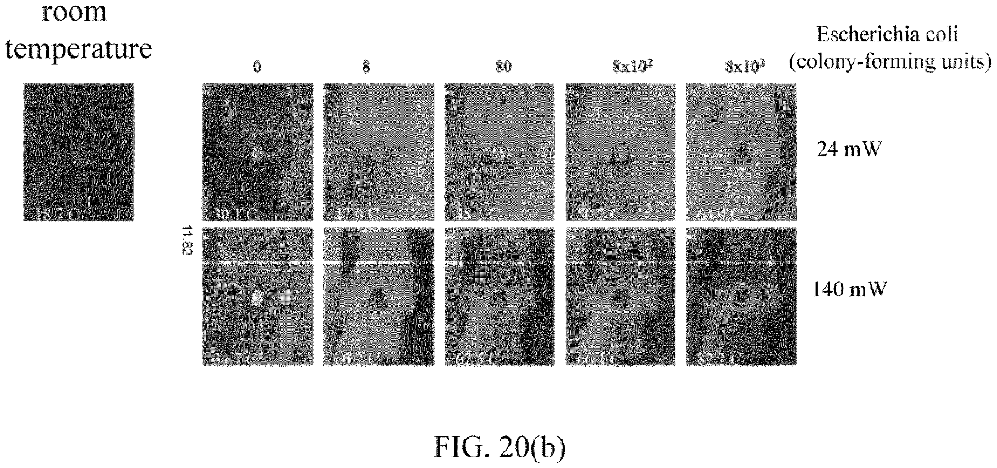

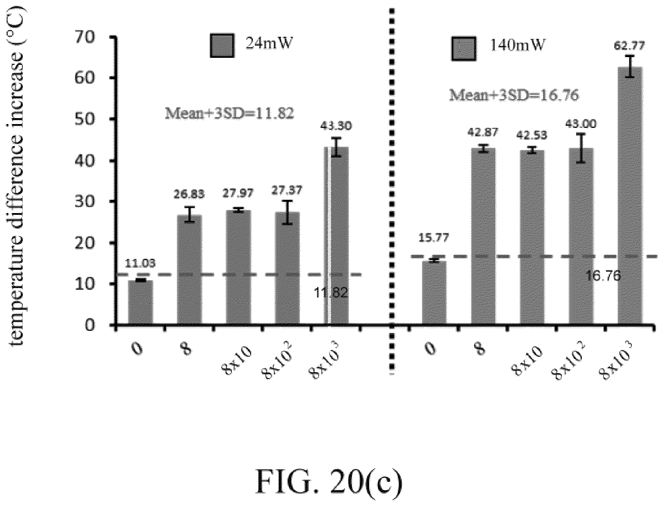

Please refer to FIG. 20(a), FIG. 20(b), and FIG. 20(c). This is another embodiment of the present invention, the analytical performance comparison of the plasmonic thermal sensing combined with lateral flow immunoassay strips 171 and the conventional lateral flow immunoassay strips 171, after the procedures of target analyte enrichment, the photothermal lysis and water cooling on-bead PCR. As shown in FIG. 19(b), the band intensity (i.e., pixel intensity of test line) at the test line 1711 of the lateral flow immunoassay strip 171 was analyzed by using the image analysis software (i.e., Image J). The quantification results of band intensities at the test lines 1711 be analyzed under various *E. coli* loadings (i.e., at 0 CFUs, 8 CFUs, $8 \times 10$ CFUs, $8 \times 10^2$ CFUs, and $8 \times 10^3$ CFUs of *E. coli* per reaction) are shown in FIG. 20(a). Although the image quantitative analyses revealed that the analytical sensitivity of the lateral flow immunoassay strip 171 combined with the image analysis could achieve 8 CFU of *E.coli* per reaction, the band intensities at the test lines 1711 of the lateral flow immunoassay strips 171 tested at 8 to $8 \times 10$ CFUs of the *E.coli* per reaction were challenging through the visual inspection. In the embodiment of the present invention, the method of integrating the plasmonic thermal sensing with the lateral flow immunoassay strip 171 can significantly improve the discrimination rate as compared those in the conventional lateral flow immunoassay strips 171. As shown in the FIG. 20(b), the detection regions including the test lines 1711 of the lateral flow immunoassay strips 171 were irradiated with the 808 nm laser powers at 24 mW/0.16cm$^2$ and 140 mW/0.16cm$^2$ respectively generated by the NIR laser-coupled infrared thermal sensor 172, and then the NIR laser-coupled infrared thermal sensor 172 is used to focus on the test line 1711 of the lateral flow immunoassay strip 171 to capture the thermal imaging. As shown in the FIG. 20(c), the quantitative results of the photothermal temperature difference were obtained by thermal imaging under 808 nm laser irradiations with energy powers at 24 mW/0.16cm$^2$ and 140 mW/0.16cm$^2$ at least for 120 seconds respectively, after subtracting the ambient temperature of the background environment. The LOD of the NIR-laser coupled infrared thermal sensor combined with the lateral flow immunoassay strip 171 is estimated from the mean of the blank (i.e., 0 CFU of the *E. coli*) plus the triple times of standard deviation of the blank. The value of LOD (i.e., the lowest quantity of photothermal temperature difference that can be distinguished from the absence of that blank with a stated confidence level) is represented as the red dashed line in the FIG.20 (c). Our results revealed that the photothermal temperature difference of the NIR-laser coupled infrared thermal sensor combined with the lateral flow immunoassay strip 171 at 8 CFU per reaction are 26.83°C and 42.87°C respectively under laser irradiations with powers of 24 mW/0.16cm$^2$ and 140 mW/0.16cm$^2$. The photothermal temperature difference is significantly higher than the LOD values (i.e., LOD=11.82°C, 24mW/0.16cm$^2$; LOD=16.76°C, 140mW/0.16cm$^2$) under various laser irradiations. Our results demonstrated that the method of integrating the plasmonic thermal sensing with the lateral flow immunoassay strip 171 can significantly improve the discrimination rate of the sample containing a relatively low amount of target analytes 20.

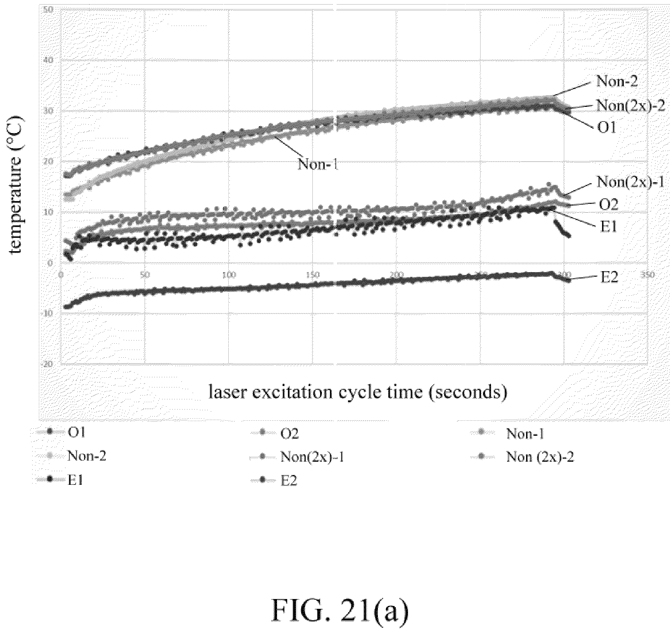

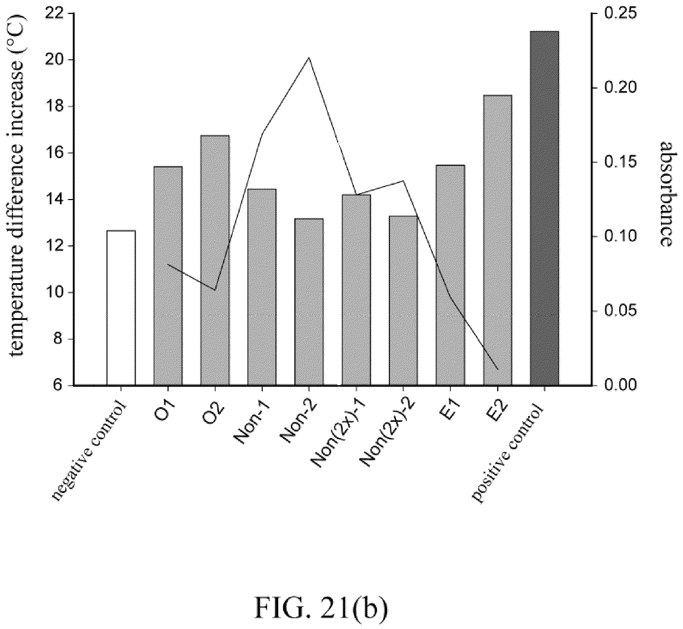

To understand the effect of the cooling temperature of the nucleic acid amplification solution 30 on the *in-situ* amplification performance of the water-cooling on-bead PCR, the issues about the influence of rapid heat dissipation under laser excitation were further investigated by testing the presence or absence of the external auxiliary cooling units 13 (e.g., an ice pack or an enlarged ice pack), the specific heat capacity of endothermic substances (e.g., the size of the ice pack, and the volume of the nucleic acid amplification solution 30). The overall temperature difference ($\Delta T$; °C) of the nucleic acid amplification solution 30 was monitored under various above-mentioned conditions during the performing of the water cooling on-bead PCR. All experiment groups had been classified depicted as the "O group" (i.e., with an ice pack as an external axillary cooling unit 13, 100 $\mu$L nucleic acid amplification solution 30), the "Non group" (i.e., without an ice pack, 100 $\mu$L nucleic acid amplification solution 30), the "Non (2x) group" (i.e., without an ice pack, 200 $\mu$L nucleic acid amplification solution 30), and the "E group" (i.e., with an enlarged ice pack, 100 $\mu$L nucleic acid amplification solution 30). Each experiment group contains two duplicated experiments and each experiment has equal amounts of MGNs at $2.8\times10^8$ particles. The ice pack was prepared by freezing 550 $\mu$L of water, while the enlarged ice pack was prepared by freezing 2,200 $\mu$L of water. Please refer to FIG. 21(a), in this embodiment of the present invention, the 190 nm diameter MGNs were used as the amplification bodies 42, and the reactions of water cooling on-bead PCR were performed at different initial temperatures according to the aforementioned groupings under irradiations with 50 photonic cycling (i.e., the setup of each photonic cycle: the NIR laser irradiations at 690 mW for 1.25 seconds, followed by laser irradiations at 360 mW for 7.5 seconds). The overall temperature difference of the nucleic acid amplification solution 30 was monitored and recorded instantly with the thermocouple sensor during the performing of the water cooling on-bead PCR. Please refer to FIG. 21(b), the *in-situ* amplification performance of the water cooling on-bead PCR on the cooling temperature of the nucleic acid solutions 30 was evaluated by the bead-based enzyme-linked immunosorbent assay (bead-based ELISA) for each experiment of the above-mentioned groups. The Y-axis on the left corresponds to the scatter plot, representing the temperature difference of the nucleic acid amplification solution 30 after performing the water cooling on-bead PCR. The temperature difference in the "O1 group" was 11.2 °C (from 3.8 to 15 °C), the "O2 group" was 10.1 °C (from 2.1 to 12.2 °C), the temperature difference in the "Non-1 group" was 16.8 °C (from 13.5 to 30.3 °C) , the temperature difference in the "Non-2 group" was 20.1 °C (from 12.6 to 32.7 °C), the temperature difference in the "Non (2x)-1 group " was 14.2 °C (from 17.2 to 31.4 °C) , the temperature difference in the "Non (2x)-2 group" was 14.8 °C (from 17.5 to 32.3 °C), and the temperature difference in the "E1 group" was 9.8 °C (from 1.1 to 10.9 °C), the temperature difference in the "E2 group" was 6.7 °C (from -8.7 to -2 °C). The Y-axis on the right corresponds to the histogram plot, representing the absorbance values at 450 nm (A$_{450}$) generated by the bead-based ELISA, the quantitative performance evaluation of the water cooling on-bead PCR on the cooling temperature of the nucleic acid solutions 30 was evaluated by the bead-based ELISA. The higher absorbance values at 450 nm generated by the bead-based ELISA indicate that this has better *in-situ* amplification performances during the water cooling on-bead PCR. The absorbance in the "O1 group" was 0.147, the absorbance in the "O2 group" was 0.168, the absorbance in the "Non-1 group" was 0.132, the absorbance in the "Non-2 group" was 0.120, the absorbance in the "Non (2x)-1 group" was 0.128, the absorbance in the "Non (2x)-2 group" was 0.138, the absorbance in the " E1 group " was 0.148, the absorbance in the " E2 group " was 0.195. Our results also revealed that if the temperature difference of the nucleic acid amplification solution 30 is smaller, the *in-situ* amplification performance of water cooling on-bead PCR will be better. The external auxiliary cooling unit 13 could provide better conditions to facilitate the nucleic acid amplification solution 30 maintain within the range of cooling temperature, and its cooling performance is even better than simply using a large volume (i.e., 100 to 200 μL) of the pre-cooled nucleic acid amplification solutions 30.

In the embodiment of the present invention, the water cooling on-bead PCR was performed under the photonic cycles with the NIR laser irradiations, in which the thermal cycling generated around the solid-phase carriers 40 is controlled by the photonic cycles, including power output and timing sequence of on and off of the NIR laser irradiations modulated by the integrated driver 11. Each photonic cycle triggered the thermal cycling generated around the solid-phase carriers 40 including the sequential processes of the nucleic acid denaturation, primer annealing, and polymerase extension. The interval of the photonic cycle for performing the nucleic acid denaturation ranges from 0.5 to 5 seconds, preferably 1 to 2.5 seconds, the power of the NIR laser ranges from 400 to 800 mW/0.16 cm$^2$, and the temperature generated around the solid-phase carriers 40 for performing the nucleic acid denaturation ranges from 85 to 95°C, preferably 95°C. The interval of the photonic cycle for performing the primer annealing and polymerase extension ranges from 2 to 15 seconds, preferably 5 to 15 seconds, with laser power ranges from 100 to 400 mW/0.16cm$^2$, and the temperature generated around the solid-phase carriers 40 for performing the primer annealing and polymerase extension ranges from 55 to 65°C, preferably 60°C. Furthermore, the total turnaround time of 50 photonic cycles for performing the water cooling on-bead PCR time ranges from 5 to 15 minutes.

The present invention is a nucleic acid detection method, wherein the aforementioned solid-phase carriers 40 exhibit magnetism, after the procedure of the water cooling on-bead PCR, the target amplicons 60 are generated and immobilized on the solid-phase carriers 40, and the detection of target amplicons 60 immobilized on the solid-phase carriers 40 is performed as the following steps:

Using an operation unit 15 to assist in purification, separation and concentration the amplicons 60 immobilized on the solid-phase carriers 40;

Using a detection module 17 to detect one or a combination of optical changes, thermal sensing changes, electrochemical changes, magnetic changes, or mass changes occurring on the amplicons 60 immobilized on the solid-phase carriers 40.

In the present invention, the methods for detecting the optical changes on the amplicons 60 immobilized on solid-phase carriers 40, comprised of detecting the change of light intensity generated directly by the primer 301 labelled with the nucleic acid tags or fluorophore 3011 incorporated into amplicons 60 with a spectrophotometer or a fluorometer, or detecting the optical changes or chemiluminescence changes generated by an enzyme-linked immunosorbent assay, or detecting spectral changes resulting from the repulsion on the amplicons 60 immobilized on the solid-phase carriers 40.

In the present invention, the method for detecting optical changes of the amplicons 60 immobilized on the solid-phase carriers 40, wherein the detection module comprises the nucleic acid lateral flow strips or the lateral flow immunoassay strips 171, further combined with thermal sensing, surface-enhanced Raman spectroscopy (SERS) or a combination thereof, thus enhancing the sensitivity of the nucleic acid lateral flow strip detection or lateral flow immunoassay strip 171.

In the present invention, the method for magnetic changes occurring on the amplicons 60 immobilized on the solid-phase carriers 40, including the detections of frequency-dependent AC susceptibility with the AC susceptometry, and the giant magnetoresistive measurement (GMR), or a combination of both.

In the present invention, the method for electrochemical changes occurring on the amplicons 60 immobilized on the solid-phase carriers 40, including one or a combination of both electrochemical detections coupled with enzyme-linked immunosorbent assay and electrochemical impedance spectroscopy (EIS).

In the present invention, a method for mass changes occurring on the amplicons 60 immobilized on the solid-phase carriers 40 is performed using a quartz crystal microbalance (QCM).

As illustrated above, the present invention has completed various experimental verifications for performing the water cooling on-bead PCR. Firstly, the linear calibration curve of the applied NIR laser powers and the micro-scale photothermal converted temperature of the solid-phased carriers 40 suspensions has been established to estimate the suitable temperature ranges for performing the *in-situ* amplification on the solid-phase carriers 40. In addition to the optimal conditions in photonic cycles of the external energy excitation (e.g., the output powers and irradiation frequency) and the cooling temperature of the nucleic acid amplification solution 30 on the *in-situ* amplification performance have also been discussed. By the coordination of the localized heating of the solid-phase carriers 40 induced by external energy and cooling temperature provided by the PCR reaction solutions 30, rapid thermal cycling generated around the solid-phase carriers 40 could be achieved. Furthermore, the subsequent modes for performing the detections of the amplicons 60 generated on the solid-phase carriers 40 and the operation examples for pretreatment of the analyte 20 have also been discussed. Taken together, our results demonstrated that the integrated platform of the present invention could achieve the target analytes enrichment, rapid nucleic acid amplification and detection in the sample-in and result-out manner. Therefore, the present invention can be applied to nucleic acid-based point-of-care testings (POCTs). In conclusion, the present invention confers the following advantages:

1. By using solid-phase carriers 40 with the high surface area to volume ratio, the combination of ligands on their surfaces can be flexibly functionalized according to the intended application (e.g., antibodies, nucleic acid aptamers, oligonucleotides, proteins, polysaccharides, or a combination thereof).

2. By controlling the coordination of the external energy excitation and the cooling temperature provided by the nucleic acid amplification solution 30 or the auxiliary cooling unit, the present invention can rapidly achieve the *in-situ* lysis of cells, the hybridization capture of target nucleic acids released from the analytes 20, and the localized temperature conditions required for performing *in-situ* nucleic acid amplification on the surface of the solid-phase carriers 40 (i.e., *in-situ* environment 50).

3. Since the above-mentioned *in-situ* environment 50 occurred on the surface of the solid-phase carriers 40, the *in-situ* nucleic acid amplification is constricted to the space adjacent to the surface of the solid-phase carriers 40. The pre-cooled nucleic acid amplification solutions 30 surrounding the solid-phase carriers 40 not only rapidly dissipate the heat from the solid-phase carriers 40 but also further constrict the spreading of heat front of the solid-phase carrier 40 upon the external excitation. This effect of localized heating contributes to the space adjacent to the surface of the solid-phase carriers 40 form the independent *in-situ* environment 50. However, in non *in-situ* environment 50, the cooling temperature of the pre-cooled nucleic acid amplification solution 30 can inhibit the polymerase activity, resulting in a reduction of the occurrence of the non-specific amplification. Furthermore, the technical feature of the water cooling on-bead PCR leads to create a thermal filed partitioning for performing the *in-situ* nucleic acid amplification on the solid-phased carriers 40, which is similar to the concept of droplet digital PCR (ddPCR) or emulsion PCR, the PCR reactions are further partitioned into tens of thousands of nano-liter sized droplets, where an independent PCR reaction takes place in physically isolated chambers. However, unlike the physical partitioning of PCR reactions that occurred in the ddPCR or emulsion PCR, the technical feature of the "virtual emulsion PCR or virtual droplet digital PCR" of the disclosed invention, which means that it does not need the additional the droplet generator and the assistance of the mineral oil and surfactants to partition test samples into the physically isolated droplets. The effect of virtual partitioning in the water cooling on-bead PCR can reduce the influences originating from the complexity of the analytes 20 on the nucleic acid amplification and subsequent detection reaction.

4. The low temperature of the nucleic acid amplification solution 30 significantly improves technical issues, such as evaporation and bubble generation that occur in conventional miniaturized nucleic acid amplification devices.

5. The present invention can achieve the ultra-fast thermal cycling required for performing *in-situ* nucleic acid amplification in the *in-situ* environment 50 on the solid-phase carriers 40, and subsequent purification, separation, and concentration of the amplicons 60 immobilized on the solid-phase carriers 40 under assistance with the magnetic operation unit 15. These advancements significantly reduce the overall turnaround time required for nucleic acid amplification and detection.

6. The present invention greatly simplifies thermal management in performing the nucleic acid amplification.

7. Simplified pretreatment of the analytes 20 and detection of the amplicons 60: The magnetic solid-phase carriers 40 can be applied for purification and concentration (enrichment) of the analytes 20, the biological substances 21, and the amplicons 60. Furthermore, the solid-phase carriers 40 can act as the signal substances on the test line 1711 of the lateral flow immunoassay strips 171, (e.g., to form the visually observable bandings on the test line and those of the thermal images detected through plasmonic thermal sensing). Altogether, by using the unique properties of the solid-phase carriers 40 (e.g., magnetism, the enrichment and amplification ligands functionalized, LSPR, and plasmonic heating). These characteristics facilitate the pretreatment of the analyte 20 and detection of the analyte 20 and the amplicons 60.

The above brief description of the present invention is intended to provide a basic explanation of several aspects and technical features of the present invention. A brief description of the invention is not a detailed representation of the present invention. Therefore, its purpose is not to specifically enumerate the critical or the essential elements of the invention, nor to define the scope of the invention. It is merely to present several concepts of the invention concisely.

**Claims**

1. A nucleic acid amplification method, **characterized in that** the nucleic acid amplification method comprises steps of:

   providing an analyte, a nucleic acid amplification solution, and a plurality of solid-phase carriers inside a reaction unit;
   using an external auxiliary cooling unit to continuously cool the reaction unit, such that the reaction unit is maintained at a cooling temperature; and
   using an external energy excitation unit to simultaneously excite the plurality of solid-phase carriers to generate heat, thereby creating a thermal in-situ environment around each of the plurality of solid-phase carriers upon external energy radiation by the external energy excitation unit; wherein the thermal in-situ environment dissipates by the cooling temperature maintained by the external auxiliary cooling unit upon pausing of the external energy radiation, and the cooling temperature maintained by the external auxiliary cooling unit constricts spreading of the thermal in-situ environment generated by the plurality of solid-phase carriers upon the external energy radiation,
   wherein, by modulating the external energy excitation unit, a thermal cycling within the thermal in-situ environment and a localized temperature condition required for performing in-situ nucleic acid amplification on a surface of each of the plurality of solid-phase carriers are generated; and
   wherein a portion of a plurality of amplicons generated through the in-situ nucleic acid amplification is retained on each of the plurality of solid-phase carriers, while another portion of the plurality of amplicons is released into the nucleic acid amplification solution.

2. The nucleic acid amplification method as described in claim 1, **characterized in that** a ratio of a total volume of the plurality of solid-phase carriers to a volume of the nucleic acid amplification solution ranges from 1:200 to $1:1 \times 10^9$.

3. The nucleic acid amplification method as described in claim 1, **characterized in that** a size of each of the plurality of solid-phase carriers ranges from 8 to 2,000,000 nm.

4. The nucleic acid amplification method as described in claim 1, **characterized in that** the cooling temperature ranges from -10 to 50°C.

5. The nucleic acid amplification method of claim 1, **characterized in that** the reaction unit or the nucleic acid amplification solution is pre-cooled to the cooling temperature or placed in the external auxiliary cooling unit.

6. The nucleic acid amplification method as described in claim 1, **characterized in that** the analyte is a cell, an organelle, a bacterium, a virus, a protozoan, or a combination thereof.

7. The nucleic acid amplification method of claim 1, **characterized in that** each of the plurality of solid-phase carrier comprises:

   a multifunctional body;
   at least one enrichment ligand, wherein the at least one enrichment ligand is bound to a surface of the multifunctional body and is configured to capture the analyte; and
   at least one amplification ligand, wherein the at least one amplification ligand is bound to the surface of the multifunctional body and is configured to bind a biological substance.

8. The nucleic acid amplification method as described in claim 7, **characterized in that** the biological substance is deoxyribonucleic acid or ribonucleic acid released from the analyte.

9. The nucleic acid amplification method as described in claim 6, **characterized in that** one portion of each of the plurality of solid-phase carriers comprises:

   an enrichment body;
   at least one enrichment ligand, wherein the at least one enrichment ligand is bound to a surface of the enrichment body and is configured to capture the analyte;

   wherein one portion of each of the plurality of solid-phase carriers comprises:

   an amplification body;
   at least one amplification ligand, wherein the at least one amplification ligand is bound to a surface of the amplification body and is configured to capture a biological substance released by the analyte.

10. The nucleic acid amplification method as described in claim 9, **characterized in that** the biological substance is deoxyribonucleic acid or ribonucleic acid released from the analyte.

11. The nucleic acid amplification method as described in claim 1, **characterized in that** the analyte is a free deoxyribonucleic acid or a free ribonucleic acid.

**12.** The nucleic acid amplification method as described in claim 11, **characterized in that** each of the plurality of solid-phase carriers comprises:

> an amplification body; and
> at least one amplification ligand, wherein the at least one amplification ligand is bound to a surface of the amplification body and is configured to capture the analyte.

**13.** A rapid nucleic acid amplification platform for performing the nucleic acid amplification method as described in claim 1, **characterized in that** the rapid nucleic acid amplification platform comprises:

> a reaction unit configured to accommodate a reaction solution, an analyte, a nucleic acid amplification solution, and a plurality of solid-phase carriers evenly distributed in the nucleic acid amplification solution;
> an external auxiliary cooling unit connected to the reaction unit and disposed around the reaction unit, configured to continuously cool down the reaction unit and maintains the nucleic acid amplification solution at a cooling temperature;
> an external energy excitation unit connected to the reaction unit and configured to excite the plurality of solid-phase carriers, thereby generating heat by the plurality of solid-phase carriers;
> an integrated driver connected to the external energy excitation unit and configured to modulate energy output and timing sequence of on and off of the external energy excitation unit.

**14.** A nucleic acid detection method for performing nucleic detection after completing the nucleic acid detection method as claimed in any one of claims 1 to 12, **characterized in that** the nucleic acid detection method comprises steps of:

> using an operation unit to assist in purification, separation and concentration the portion of the plurality of amplicons immobilized on the plurality of solid-phase carriers;
> using a detection module to detect optical changes, thermal sensing changes, electrochemical changes, magnetic changes, mass changes or a combination thereof occurring on the portion of the plurality of amplicons immobilized on the plurality of solid-phase carriers.

**15.** The nucleic acid detection method as described in claim 14, **characterized in that** using the detection module to detect optical change occurring on the portion of the plurality of amplicons immobilized on the plurality of solid-phase carriers comprises mixing the portion of the plurality of amplicons with a nucleic acid tag labeled with a fluorophore and detecting the light intensity using a spectrophotometer, or detecting the optical changes or chemiluminescence changes generated by the portion of the plurality of amplicons using an enzyme-linked immunosorbent assay, or detecting spectral changes resulting from binding of the portion of the plurality of amplicons to the plurality of solid-phase carriers.

**16.** The nucleic acid detection method as described in claim 15, **characterized in that** wherein using the detection module to detect the optical changes on the portion of the plurality of amplicons immobilized on the plurality of solid-phase carriers comprises a nucleic acid lateral flow strip or a lateral flow immunoassay strip, which are configured to detect the optical changes.

**17.** The nucleic acid detection method as described in claim 16, **characterized in that** analytical sensitivity of the nucleic acid lateral flow strip or the lateral flow immunoassay strip is enhanced by combining the nucleic acid lateral flow strip or the lateral flow immunoassay strip with a thermal sensing detection, surface plasmon resonance spectroscopy, or a combination thereof.

**18.** The nucleic acid detection method as described in claim 14, **characterized in that** using the detection module to detect the electrochemical changes occurring on the portion of the plurality of amplicons immobilized on the plurality of solid-phase carriers comprises one or a combination of both electrochemical detection coupled with enzyme-linked immunosorbent assay and electrochemical impedance spectroscopy.

**19.** The nucleic acid detection method as described in claim 14, **characterized in that** using the detection module to detect the magnetic changes occurring on the portion of the plurality of amplicons on the plurality of solid-phase carriers includes detections of frequency-dependent alternating current susceptibility with alternating current susceptometry, giant magnetoresistive measurement, or a combination thereof.

**20.** The nucleic acid detection method as described in claim 14, **characterized in that** using the detection module to detect the mass changes occurring on the portion of the plurality of amplicons immobilized on the plurality of solid-phase carriers is performed using a quartz crystal microbalance.

**Patentansprüche**

**1.** Ein Verfahren zur Amplifikation von Nukleinsäuren, **dadurch gekennzeichnet, dass** das Verfahren zur Amplifikation von Nukleinsäuren die folgenden Schritte umfasst:

Bereitstellen eines Analyten, einer Nukleinsäure-Amplifikationslösung und einer Vielzahl von Festphasenträgern innerhalb einer Reaktionseinheit;

Verwenden einer externen Hilfskühleinheit zum kontinuierlichen Kühlen der Reaktionseinheit, so dass die Reaktionseinheit auf einer Kühltemperatur gehalten wird; und

Verwenden einer externen Energieanregungseinheit zum gleichzeitigen Anregen der Vielzahl von Festphasenträgern, um Wärme zu erzeugen, wodurch bei externer Energiestrahlung durch die externe Energieanregungseinheit eine thermische In-situ-Umgebung um jeden der Vielzahl von Festphasenträgern erzeugt wird; wobei sich die thermische In-situ-Umgebung durch die von der externen Hilfskühleinheit aufrechterhaltene Kühltemperatur beim Pausieren der externen Energiestrahlung auflöst und die von der externen Hilfskühleinheit aufrechterhaltene Kühltemperatur die Ausbreitung der durch die Vielzahl von Festphasenträgern bei der externen Energiestrahlung erzeugten thermischen In-situ-Umgebung einschränkt, wobei durch Modulieren der externen Energieanregungseinheit ein thermisches Zyklieren innerhalb der thermischen In-situ-Umgebung und eine lokalisierte Temperaturbedingung erzeugt werden, die zum Durchführen einer In-situ-Nukleinsäureamplifikation auf einer Oberfläche jedes der Vielzahl von Festphasenträgern erforderlich sind; und

wobei ein Teil einer Vielzahl von Amplikons, die durch die In-situ-Nukleinsäureamplifikation erzeugt werden, auf jedem der Vielzahl von Festphasenträgern zurückgehalten wird, während ein anderer Teil der Vielzahl von Amplikons in die Nukleinsäure-Amplifikationslösung freigesetzt wird.

2. Das Verfahren zur Amplifikation von Nukleinsäuren wie in Anspruch 1 beschrieben, **dadurch gekennzeichnet, dass** ein Verhältnis eines Gesamtvolumens der Vielzahl von Festphasenträgern zu einem Volumen der Nukleinsäure-Amplifikationslösung im Bereich von 1:200 bis 1:1x10$^9$ liegt.

3. Das Verfahren zur Amplifikation von Nukleinsäuren wie in Anspruch 1 beschrieben, **dadurch gekennzeichnet, dass** eine Größe jedes der Vielzahl von Festphasenträgern im Bereich von 8 bis 2,000,000 nm liegt.

4. Das Verfahren zur Amplifikation von Nukleinsäuren wie in Anspruch 1 beschrieben, **dadurch gekenn-** zeichnet, dass die Kühltemperatur im Bereich von -10 bis 50 °C liegt.

5. Das Verfahren zur Amplifikation von Nukleinsäuren nach Anspruch 1, **dadurch gekennzeichnet, dass** die Reaktionseinheit oder die Nukleinsäure-Amplifikationslösung auf die Kühltemperatur vorgekühlt oder in der externen Hilfskühleinheit platziert wird.

6. Das Verfahren zur Amplifikation von Nukleinsäuren wie in Anspruch 1 beschrieben, **dadurch gekennzeichnet, dass** der Analyt eine Zelle, eine Organelle, ein Bakterium, ein Virus, ein Protozoon oder eine Kombination davon ist.

7. Das Verfahren zur Amplifikation von Nukleinsäuren nach Anspruch 1, **dadurch gekennzeichnet, dass** jeder der Vielzahl von Festphasenträgern umfasst:

einen multifunktionalen Körper;
mindestens einen Anreicherungsliganden, wobei der mindestens eine Anreicherungsligand an eine Oberfläche des multifunktionalen Körpers gebunden ist und so konfiguriert ist, dass er den Analyten einfängt; und
mindestens einen Amplifikationsliganden, wobei der mindestens eine Amplifikationsligand an die Oberfläche des multifunktionalen Körpers gebunden ist und so konfiguriert ist, dass er eine biologische Substanz bindet.

8. Das Verfahren zur Amplifikation von Nukleinsäuren wie in Anspruch 7 beschrieben, **dadurch gekennzeichnet, dass** die biologische Substanz Desoxyribonukleinsäure oder Ribonukleinsäure ist, die aus dem Analyten freigesetzt wird.

9. Das Verfahren zur Amplifikation von Nukleinsäuren wie in Anspruch 6 beschrieben, **dadurch gekennzeichnet, dass** ein Teil jedes der Vielzahl von Festphasenträgern umfasst:

einen Anreicherungskörper;
mindestens einen Anreicherungsliganden, wobei der mindestens eine Anreicherungsligand an eine Oberfläche des Anreicherungskörpers gebunden ist und so konfiguriert ist, dass er den Analyten einfängt;

wobei ein Teil jedes der Vielzahl von Festphasenträgern umfasst:

einen Amplifikationskörper;
mindestens einen Amplifikationsliganden, wobei der mindestens eine Amplifikationsligand an eine Oberfläche des Amplifikationskörpers gebunden ist und so konfiguriert ist, dass er eine durch den Analyten freigesetzte biologische Substanz einfängt.

10. Das Verfahren zur Amplifikation von Nukleinsäuren wie in Anspruch 9 beschrieben, **dadurch gekennzeichnet, dass** die biologische Substanz Desoxyribonukleinsäure oder Ribonukleinsäure ist, die aus dem Analyten freigesetzt wird.

11. Das Verfahren zur Amplifikation von Nukleinsäuren wie in Anspruch 1 beschrieben, **dadurch gekennzeichnet, dass** der Analyt eine freie Desoxyribonukleinsäure oder eine freie Ribonukleinsäure ist.

12. Das Verfahren zur Amplifikation von Nukleinsäuren wie in Anspruch 11 beschrieben, **dadurch gekennzeichnet, dass** jeder der Vielzahl von Festphasenträgern umfasst:

einen Amplifikationskörper; und
mindestens einen Amplifikationsliganden, wobei der mindestens eine Amplifikationsligand an eine Oberfläche des Amplifikationskörpers gebunden ist und so konfiguriert ist, dass er den Analyten einfängt.

13. Eine schnelle Nukleinsäure-Amplifikationsplattform zur Durchführung des Verfahrens zur Amplifikation von Nukleinsäuren wie in Anspruch 1 beschrieben, **dadurch gekennzeichnet, dass** die schnelle Nukleinsäure-Amplifikationsplattform umfasst:

eine Reaktionseinheit, die so konfiguriert ist, dass sie eine Reaktionslösung, einen Analyten, eine Nukleinsäure-Amplifikationslösung und eine Vielzahl von Festphasenträgern aufnimmt, die gleichmäßig in der Nukleinsäure-Amplifikationslösung verteilt sind;
eine externe Hilfskühleinheit, die mit der Reaktionseinheit verbunden und um die Reaktionseinheit herum angeordnet ist, die so konfiguriert ist, dass sie die Reaktionseinheit kontinuierlich abkühlt und die Nukleinsäure-Amplifikationslösung auf einer Kühltemperatur hält;
eine externe Energieanregungseinheit, die mit der Reaktionseinheit verbunden und so konfiguriert ist, dass sie die Vielzahl von Festphasenträgern anregt, wodurch durch die Vielzahl von Festphasenträgern Wärme erzeugt wird;
einen integrierten Treiber, der mit der externen Energieanregungseinheit verbunden und so konfiguriert ist, dass er die Energieabgabe und die zeitliche Abfolge des Ein- und Ausschaltens der externen Energieanregungseinheit moduliert.

14. Ein Verfahren zum Nachweis von Nukleinsäuren zur Durchführung eines Nukleinsäurenachweises nach Abschluss des in einem der Ansprüche 1 bis 12 beanspruchten Verfahrens zum Nachweis von Nukleinsäuren, **dadurch gekennzeichnet, dass** das Verfahren zum Nachweis von Nukleinsäuren die folgenden Schritte umfasst:

Verwenden einer Operationseinheit zur Unterstützung bei der Reinigung, Trennung und Konzentration des Teils der Vielzahl von Amplikons, die auf der Vielzahl von Festphasenträgern immobilisiert sind;
Verwenden eines Nachweismoduls zum Nachweis von optischen Veränderungen, thermischen Sensorveränderungen, elektrochemischen Veränderungen, magnetischen Veränderungen, Massenveränderungen oder einer Kombination davon, die an dem Teil der Vielzahl von Amplikons auftreten, die auf der Vielzahl von Festphasenträgern immobilisiert sind.

15. Das Verfahren zum Nachweis von Nukleinsäuren wie in Anspruch 14 beschrieben, **dadurch gekennzeichnet, dass** die Verwendung des Nachweismoduls zum Nachweis einer optischen Veränderung, die an dem Teil der Vielzahl von Amplikons auftritt, die auf der Vielzahl von Festphasenträgern immobilisiert sind, das Mischen des Teils der Vielzahl von Amplikons mit einem mit einem Fluorophor markierten Nukleinsäure-Tag und das Nachweisen der Lichtintensität unter Verwendung eines Spektrophotometers umfasst, oder das Nachweisen der optischen Veränderungen oder Chemilumineszenzveränderungen, die durch den Teil der Vielzahl von Amplikons erzeugt werden, unter Verwendung eines enzymgekoppelten Immunadsorptionstests (ELISA), oder das Nachweisen spektraler Veränderungen, die aus der Bindung des Teils der Vielzahl von Amplikons an die Vielzahl von Festphasenträgern resultieren.

16. Das Verfahren zum Nachweis von Nukleinsäuren wie in Anspruch 15 beschrieben, **dadurch gekennzeichnet, dass** die Verwendung des Nachweismoduls zum Nachweis der optischen Veränderungen an dem Teil der Vielzahl von Amplikons, die auf der Vielzahl von Festphasenträgern immobilisiert sind, einen Nukleinsäure-Lateral-Flow-Teststreifen oder einen Lateral-Flow-Immunoassay-Teststreifen umfasst, die so konfiguriert sind, dass sie die optischen Veränderungen nachweisen.

17. Das Verfahren zum Nachweis von Nukleinsäuren wie in Anspruch 16 beschrieben, **dadurch gekennzeichnet, dass** die analytische Sensitivität des Nukleinsäure-Lateral-Flow-Teststreifens oder des Lateral-Flow-Immunoassay-Teststreifens durch die Kombination des Nukleinsäure-Lateral-Flow-Teststreifens oder des Lateral-Flow-Immunoassay-Teststreifens mit einem thermischen Sensornachweis, Oberflächenplasmonenresonanzspektroskopie oder einer Kombination davon verbessert wird.

**18.** Das Verfahren zum Nachweis von Nukleinsäuren wie in Anspruch 14 beschrieben, **dadurch gekennzeichnet, dass** die Verwendung des Nachweismoduls zum Nachweis der elektrochemischen Veränderungen, die an dem Teil der Vielzahl von Amplikons auftreten, die auf der Vielzahl von Festphasenträgern immobilisiert sind, eine oder eine Kombination aus beidem, elektrochemischer Detektion gekoppelt mit einem enzymgekoppelten Immunadsorptionstest und elektrochemischer Impedanzspektroskopie, umfasst.

**19.** Das Verfahren zum Nachweis von Nukleinsäuren wie in Anspruch 14 beschrieben, **dadurch gekennzeichnet, dass** die Verwendung des Nachweismoduls zum Nachweis der magnetischen Veränderungen, die an dem Teil der Vielzahl von Amplikons auf der Vielzahl von Festphasenträgern auftreten, den Nachweis der frequenzabhängigen Wechselstromsuszeptibilität mit Wechselstromsuszeptometrie, Riesenmagnetowiderstandsmessung oder einer Kombination davon einschließt.

**20.** Das Verfahren zum Nachweis von Nukleinsäuren wie in Anspruch 14 beschrieben, **dadurch gekennzeichnet, dass** die Verwendung des Nachweismoduls zum Nachweis der Massenveränderungen, die an dem Teil der Vielzahl von Amplikons auftreten, die auf der Vielzahl von Festphasenträgern immobilisiert sind, unter Verwendung einer Schwingquarz-Mikrowaage durchgeführt wird.

**Revendications**

**1.** Un procédé d'amplification d'acide nucléique, **caractérisé en ce que** le procédé d'amplification d'acide nucléique comprend les étapes consistant à:

fournir un analyte, une solution d'amplification d'acide nucléique et une pluralité de supports en phase solide à l'intérieur d'une unité de réaction; utiliser une unité de refroidissement auxiliaire externe pour refroidir en continu l'unité de réaction, de telle sorte que l'unité de réaction soit maintenue à une température de refroidissement; et utiliser une unité d'excitation d'énergie externe pour exciter simultanément la pluralité de supports en phase solide afin de générer de la chaleur, créant ainsi un environnement thermique in situ autour de chacun de la pluralité de supports en phase solide lors du rayonnement d'énergie externe par l'unité d'excitation d'énergie externe; dans lequel l'environnement thermique in situ se dissipe par la température de refroidissement maintenue par l'unité de refroidissement auxiliaire externe lors de l'interruption du rayonnement d'énergie externe, et la température de refroidissement maintenue par l'unité de refroidissement auxiliaire externe restreint la propagation de l'environnement thermique in situ généré par la pluralité de supports en phase solide lors du rayonnement d'énergie externe,

dans lequel, en modulant l'unité d'excitation d'énergie externe, un cyclage thermique au sein de l'environnement thermique in situ et une condition de température localisée requise pour effectuer une amplification d'acide nucléique in situ sur une surface de chacun de la pluralité de supports en phase solide sont générés; et

dans lequel une partie d'une pluralité d'amplicons générés par l'amplification d'acide nucléique in situ est retenue sur chacun de la pluralité de supports en phase solide, tandis qu'une autre partie de la pluralité d'amplicons est libérée dans la solution d'amplification d'acide nucléique.

**2.** Le procédé d'amplification d'acide nucléique tel que décrit dans la revendication 1, **caractérisé en ce qu'**un rapport d'un volume total de la pluralité de supports en phase solide sur un volume de la solution d'amplification d'acide nucléique est compris entre 1:200 et $1:1\times10^9$.

**3.** Le procédé d'amplification d'acide nucléique tel que décrit dans la revendication 1, **caractérisé en ce qu'**une taille de chacun de la pluralité de supports en phase solide est comprise entre 8 et 2,000,000 nm.

**4.** Le procédé d'amplification d'acide nucléique tel que décrit dans la revendication 1, **caractérisé en ce que** la température de refroidissement est comprise entre -10 et 50 °C.

**5.** Le procédé d'amplification d'acide nucléique de la revendication 1, **caractérisé en ce que** l'unité de réaction ou la solution d'amplification d'acide nucléique est prérefroidie à la température de refroidissement ou placée dans l'unité de refroidissement auxiliaire externe.

**6.** Le procédé d'amplification d'acide nucléique tel que décrit dans la revendication 1, **caractérisé en ce que** l'analyte est une cellule, un organite, une bactérie, un virus, un protozoaire, ou une combinaison de ceux-ci.

**7.** Le procédé d'amplification d'acide nucléique de la revendication 1, **caractérisé en ce que** chacun de la pluralité de supports en phase solide comprend:

un corps multifonctionnel;

au moins un ligand d'enrichissement, dans lequel ledit au moins un ligand d'enrichissement est lié à une surface du corps multifonctionnel et est configuré pour capturer l'analyte; et

au moins un ligand d'amplification, dans lequel ledit au moins un ligand d'amplification est lié à la surface du corps multifonctionnel et est configuré pour lier une substance biologique.

8. Le procédé d'amplification d'acide nucléique tel que décrit dans la revendication 7, **caractérisé en ce que** la substance biologique est un acide désoxyribonucléique ou un acide ribonucléique libéré par l'analyte.

9. Le procédé d'amplification d'acide nucléique tel que décrit dans la revendication 6, **caractérisé en ce qu'**une partie de chacun de la pluralité de supports en phase solide comprend:

un corps d'enrichissement;

au moins un ligand d'enrichissement, dans lequel ledit au moins un ligand d'enrichissement est lié à une surface du corps d'enrichissement et est configuré pour capturer l'analyte;

dans lequel une partie de chacun de la pluralité de supports en phase solide comprend:

un corps d'amplification;

au moins un ligand d'amplification, dans lequel ledit au moins un ligand d'amplification est lié à une surface du corps d'amplification et est configuré pour capturer une substance biologique libérée par l'analyte.

10. Le procédé d'amplification d'acide nucléique tel que décrit dans la revendication 9, **caractérisé en ce que** la substance biologique est un acide désoxyribonucléique ou un acide ribonucléique libéré par l'analyte.

11. Le procédé d'amplification d'acide nucléique tel que décrit dans la revendication 1, **caractérisé en ce que** l'analyte est un acide désoxyribonucléique libre ou un acide ribonucléique libre.

12. Le procédé d'amplification d'acide nucléique tel que décrit dans la revendication 11, **caractérisé en ce que** chacun de la pluralité de supports en phase solide comprend:

un corps d'amplification; et

au moins un ligand d'amplification, dans lequel ledit au moins un ligand d'amplification est lié à une surface du corps d'amplification et est configuré pour capturer l'analyte.

13. Une plateforme d'amplification rapide d'acide nucléique pour mettre en œuvre le procédé d'amplification d'acide nucléique tel que décrit dans la revendication 1, **caractérisée en ce que** la plateforme d'amplification rapide d'acide nucléique comprend:

une unité de réaction configurée pour recevoir une solution de réaction, un analyte, une solution d'amplification d'acide nucléique et une pluralité de supports en phase solide répartis uniformément dans la solution d'amplification d'acide nucléique;

une unité de refroidissement auxiliaire externe reliée à l'unité de réaction et disposée autour de l'unité de réaction, configurée pour refroidir en continu l'unité de réaction et maintient la solution d'amplification d'acide nucléique à une température de refroidissement;

une unité d'excitation d'énergie externe reliée à l'unité de réaction et configurée pour exciter la pluralité de supports en phase solide, générant ainsi de la chaleur par la pluralité de supports en phase solide;

un pilote intégré relié à l'unité d'excitation d'énergie externe et configuré pour moduler la sortie d'énergie et la séquence temporelle de marche et d'arrêt de l'unité d'excitation d'énergie externe.

14. Un procédé de détection d'acide nucléique pour effectuer une détection nucléique après l'achèvement du procédé de détection d'acide nucléique selon l'une quelconque des revendications 1 à 12, **caractérisé en ce que** le procédé de détection d'acide nucléique comprend les étapes consistant à:

utiliser une unité d'opération pour aider à la purification, la séparation et la concentration de la partie de la pluralité d'amplicons immobilisés sur la pluralité de supports en phase solide;

utiliser un module de détection pour détecter des changements optiques, des changements de détection thermique, des changements électrochimiques, des changements magnétiques, des changements de masse ou une combinaison de ceux-ci se produisant sur la partie de la pluralité d'amplicons immobilisés sur la pluralité de supports en phase solide.

15. Le procédé de détection d'acide nucléique tel que décrit dans la revendication 14, **caractérisé en ce que** l'utilisation du module de détection pour détecter un changement optique se produisant sur la partie de la pluralité d'amplicons immobilisés sur la pluralité de supports en phase solide comprend le mélange de la partie de la pluralité d'amplicons avec un marqueur d'acide nucléique marqué avec un fluorophore et la détection de l'intensité lumineuse à l'aide d'un spectrophotomètre, ou la détection des changements optiques ou des changements de chimiluminescence générés par la partie de la pluralité d'amplicons en utilisant un test immuno-enzymatique, ou la détection des changements spectraux résultant de la liaison de la partie de la pluralité d'amplicons à la pluralité de supports en phase solide.

**16.** Le procédé de détection d'acide nucléique tel que décrit dans la revendication 15, **caractérisé en ce que** l'utilisation du module de détection pour détecter les changements optiques sur la partie de la pluralité d'amplicons immobilisés sur la pluralité de supports en phase solide comprend une bandelette à flux latéral d'acide nucléique ou une bandelette de test immunologique à flux latéral, qui sont configurées pour détecter les changements optiques.

**17.** Le procédé de détection d'acide nucléique tel que décrit dans la revendication 16, **caractérisé en ce que** la sensibilité analytique de la bandelette à flux latéral d'acide nucléique ou de la bandelette de test immunologique à flux latéral est améliorée en combinant la bandelette à flux latéral d'acide nucléique ou la bandelette de test immunologique à flux latéral avec une détection par capteur thermique, une spectroscopie de résonance plasmonique de surface, ou une combinaison de celles-ci.

**18.** Le procédé de détection d'acide nucléique tel que décrit dans la revendication 14, **caractérisé en ce que** l'utilisation du module de détection pour détecter les changements électrochimiques se produisant sur la partie de la pluralité d'amplicons immobilisés sur la pluralité de supports en phase solide comprend l'un ou une combinaison à la fois d'une détection électrochimique couplée à un test immuno-enzymatique et d'une spectroscopie d'impédance électrochimique.

**19.** Le procédé de détection d'acide nucléique tel que décrit dans la revendication 14, **caractérisé en ce que** l'utilisation du module de détection pour détecter les changements magnétiques se produisant sur la partie de la pluralité d'amplicons sur la pluralité de supports en phase solide inclut des détections de susceptibilité en courant alternatif dépendant de la fréquence avec une susceptométrie en courant alternatif, une mesure magnétorésistive géante, ou une combinaison de celles-ci.

**20.** Le procédé de détection d'acide nucléique tel que décrit dans la revendication 14, **caractérisé en ce que** l'utilisation du module de détection pour détecter les changements de masse se produisant sur la partie de la pluralité d'amplicons immobilisés sur la pluralité de supports en phase solide est effectuée en utilisant une microbalance à cristal de quartz.

FIG. 1(a)

FIG. 1(b)

FIG. 2

FIG. 3

FIG. 4(a)                    FIG. 4(b)

FIG. 4(c)

FIG. 5

FIG. 6

FIG. 7(a)

FIG. 7(b)

FIG. 7(c)

FIG. 7(d)

FIG. 7(e)

FIG. 7(f)

FIG. 7(g)

FIG. 7(h)

FIG. 7(i)

FIG. 7(j)

FIG. 7(k)

FIG. 7(1)

Mixing a sample with a plurality of solid-phase carriers, the sample includes at least one analyte, and the plurality of solid-phase carriers binds to at least one target analyte, then proceed to step S20 — S10

In the reaction unit, use the operation unit to wash away impurities, and concentrate (enrich) the target analyte.
If the at least one analyte is a free deoxyribonucleic acid or ribonucleic acid, proceed directly to step S51; otherwise, proceed to step S30 — S20

At least one energy excitation unit provides external energy to the plurality of solid-phase carriers, raising the thermal fields of the plurality of solid-phase carriers to the denaturation temperature (e.g., 80 to 95°C) of the analyte, then proceed to step S40. — S30

The at least one analyte 20 undergoes lysis, releasing a biological substance 21 (e.g., deoxyribonucleic acid or ribonucleic acid), then proceed to step S50. — S40    S51

Controlling the energy excitation unit to provide external energy to the plurality of solid-phase carriers until reaching a nucleic acid hybridization temperature, causing the plurality of solid-phase carriers to bind with at least one biological substance, and using the operation unit to purify, separate, and enrich the multiple solid-phase carriers, then proceed to step S60.

Controlling the energy excitation unit to provide an external energy to the plurality of solid-phase carriers until reaching a nucleic acid hybridization temperature, causing the plurality of solid-phase carriers to bind with at least one analyte, and using the operation unit to purify, separate, and enrich the multiple solid-phase carriers, then proceed to step S60.

S50

Adding pre-cooled nucleic acid amplification solution (i.e., with a temperature of the nucleic acid amplification solution ranging from -10 to 4°C) to the reaction unit and combining it with the external auxiliary cooling unit. If performing water cooling on-bead polymerase chain reaction, then proceed directly to step S70. If performing water cooling water cooling on-bead loop-mediated isothermal amplification, then proceed directly to step S71. — S60    S71

Controlling the energy excitation unit to provide external energy to the plurality of solid-phase carriers, allowing the in-situ environment of the solid-phase carriers to reach the temperature required for nucleic acid denaturation (i.e., 90 to 95°C) and primer annealing/polymerase extension (i.e., 60 to 65°C) in the polymerase chain reaction, and at least one enzyme will use for performing in-situ nucleic acid amplification on the plurality of solid-phase carriers. If performing the detection procedure, then proceed to step S80.

Controlling the energy excitation unit to provide an external energy to the plurality of solid-phase carriers, allowing the in-situ environment surrounding their surfaces to reach the temperature required for performing loop-mediated isothermal amplification (i.e., 60 to 65°C), and at least one enzyme will use for performing in-situ nucleic acid amplification on the plurality of solid-phase carriers. If performing the detection procedure, then proceed to step S80.

S70

Using the operation unit to separate the amplification bodies from the supernatant. — S80

Use a detection module to detect the changes of signal readouts on the amplicons immobilized on the amplification bodies, by detecting colorimetric changes, luminescence changes, or a combination thereof generated by recognizing the nucleic acid tags on the amplicons with antibodies conjugated with enzymes (i.e., horseradish peroxidase, alkaline phosphatase). — S90

FIG. 8

FIG. 9(a)

FIG. 9(b)

FIG. 9(c)

FIG. 9(d)

FIG. 9(e)

FIG. 10(a)

FIG. 10(b)

$$y=0.0778x+44.126$$
$$R^2=0.9104$$

FIG. 11(a)

FIG. 11(b)

FIG. 12(a)

FIG. 12(b)

FIG. 13(a)

FIG. 13(b)

FIG. 14(a)

FIG. 14(b)

EP 4 458 982 B1

FIG. 15(a)

FIG. 15(b)

FIG. 16(a)

$$0 \quad 1 \quad 10 \quad 10^2 \quad 10^3 \quad 10^4 \quad \text{malB gene fragment (copy number)}$$

control line

test line

FIG. 16(b)

FIG. 17(a)

FIG. 17(b)

| laser power ($mW/0.16\ cm^2$) | 0 | 300 | 400 | 500 |
|---|---|---|---|---|
| Escherichia coli (colonies) | 28 | 17 | 2 | 0 |

FIG. 18(a)

FIG. 18(b)

S100 — capture and concentration of the target analyte

S200 — photothermal lysis of the analyte and capture of released biological substance

S300 — water cooling on-bead polymerase chain reaction using laser excitation

S400 — detection using lateral flow immunoassay strip

FIG. 19(a)

FIG. 19(b)

FIG. 20(a)

room temperature

Escherichia coli (colony-forming units)

0          8          80          8x10²          8x10³

24 mW

140 mW

FIG. 20(b)

FIG. 20(c)

FIG. 21(a)

FIG. 21(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20190048397 A1
- US 20180080064 A1
- US 20180297120 A1
- US 20190143334 A1
- WO 2013113910 A1
- US 20140377764 A1
- DE 102012201475 B4
- CN 107604052 A
- EP 2809806 B1
- EP 3733292 A1
- US 20190249168 A1
- EP 2481817 A1

### Non-patent literature cited in the description

- Loop-mediated isothermal amplification (LAMP)-review and classification of methods for sequence-specific detection. **LISA BECHERER** ; **NADINE BORST** ; **MOHAMMED BAKHEIT** ; **SIEGHARD FRISCHMANN** ; **ROLAND ZENGERLE** ; **FELIX VON STETTEN**. Analytical Methods. 14 February 2020
- **PATRICK HARDINGE** ; **JAMES A. H. MURRAY**. Reduced False Positives and Improved Reporting of Loop-Mediated Isothermal Amplification using Quenched Fluorescent Primers. *Scientific Reports*, 14 May 2019
- **SANG HUN LEE** ; **SEUNG-MIN PARK** ; **BRIAN N. KIM** ; **OH SEOK KWON** ; **WON-YEP RHO** ; **BONG-HYUN JUN**. Emerging ultrafast nucleic acid amplification technologies for next-generation molecular diagnostics. *Biosensors and Bioelectronics*, 18 June 2019
- **YOU et al.** Ultrafast Photonic PCR Based on Photothermal Nanomaterials. *Trends in Biotechnology*, 2020
- **LARS ULLERICH** ; **STEPHANIE CAMPBELL** ; **FRANK KRIEG-SCHNEIDER** ; **FEDERICO BÜRSGENS** ; **JOACHIM STEHR**. Pulse Controlled Amplification (PCA)" to achieve ultra-fast PCR. According to the literature titled "Ultra-fast PCR technologies for point-of-care testing. *Journal of Laboratory Medicine*, 12 October 2017
- Pulse-Controlled Amplification-A new powerful tool for on-site diagnostics under resource limited conditions. *PLOS NEGLECTED TROPICAL DISEASES*, 29 January 2021